# EUROPÄISCHE PATENTSCHRIFT

(11) **EP 1 554 282 B1**
(45) Veröffentlichungstag und Bekanntmachung des Hinweises auf die Patenterteilung: **28.05.2008**
(21) Anmeldenummer: 03762539.9
(22) Anmeldetag: 27.06.2003
(51) Int. Cl.: C07D 489/08, C07D 489/02, C07D 489/04, A61K 31/485, A61P 25/04, A61P 25/20

(54) **MORPHINANDERIVATE SUBSTITUIERT IN POSITION 14 UND DEREN QUARTÄRE AMMONIUMSALZE, HERSTELLUNGSVERFAHREN UND VERWENDUNG**
MORPHINAN DERIVATIVES SUBSTITUTED IN POSITION 14 AND QUATERNARY AMMONIUM SALTS THEREOF, METHOD FOR PRODUCTION AND USE THEREOF
DERIVES DE MORPHINANE SUBSTITUES EN POSITION 14 ET LEURS SELS D'AMMONIUM QUATERNAIRE, PROCEDE DE PRODUCTION DE CES COMPOSES ET LEUR UTILISATION

(30) Priorität: 03.07.2002 DE 10229842
(43) Veröffentlichungstag der Anmeldung: 20.07.2005
(73) Patentinhaber: ALCASYNN PHARMACEUTICALS GMBH, 6020 Innsbruck (AT)
(72) Erfinder: SCHMIDHAMMER, Helmut, A-6020 Innsbruck (AT); SPETEA, Mariana, A-6020 Innsbruck (AT); SCHÜTZ, Johannes, A-6020 Innsbruck (AT); GREINER, Elisabeth, A-6082 Patsch (AT); SCHÜLLNER, Falko, A-6068 Mils (AT); SAILER, Bettina, A-6020 Innsbruck (AT); STÜBEGGER, Kurt, A-6020 Innsbruck (AT)
(74) Vertreter: Grünecker, Kinkeldey, Stockmair & Schwanhäusser Anwaltssozietät
(86) Internationale Anmeldenummer: PCT/EP2003/006866
(87) Internationale Veröffentlichungsnummer: WO 2004/005294

(56) Entgegenhaltungen:
- EP-A- 0 030 685
- EP-A- 0 250 796
- DE-A- 3 412 727
- GB-A- 1 300 419
- US-A- 4 272 540
- US-A- 4 390 699
- US-A- 4 912 114
- CHENG, C.Y. ET AL.: "N-Cubylmethyl Substituted Morphinoids as Novel Narcotic Antagonists" BIOORGANIC & MEDICINAL CHEMISTRY, Bd. 4, Nr. 1, 1996, Seiten 73-80, XP002254801
- COOP, A. ET AL.: "Delta Opioid Binding Selectivity of 3-Ether Analogs of Naltrindole" BIOORGANIC & MEDICINAL CHEMISTRY LETTERS, Bd. 9, 1999, Seiten 3435-3438, XP002254802
- SCHÜTZ, J. ET AL.: "Synthesis and Biological Evaluation of 14-Alkoxymorphinans. 17. Highly delta Opioid Receptor Selective 14-Alkoxy-Substituted Indolo- and Benzofuromorphinans" J. MED. CHEM., Bd. 45, 2002, Seiten 5378-5383, XP002254803
- SCHMIDHAMMER H ET AL: "SYNTHESIS AND BIOLOGICAL EVALUATION OF 14-ALKOXYMORPHINANS. 1. HIGHLY POTENT OPIOID AGONISTS IN THE SERIES OF (-)-14-METHOXY-N-METHYLMORPHI NAN-6-ONES" JOURNAL OF MEDICINAL CHEMISTRY, AMERICAN CHEMICAL SOCIETY. WASHINGTON, US, Bd. 27, Nr. 12, 1984, Seiten 1575-1579, XP001117678 ISSN: 0022-2623 in der Anmeldung erwähnt
- KLEIN P ET AL: "O3-(2-Carbomethoxyallyl) ethers of opioid ligands derived from oxymorphone, naltrexone, etorphine, diprenorphine, norbinaltorphimine, an naltrindole. Unexpected O3-dealkylation in the opioid radioligand displacement assay" JOURNAL OF MEDICINAL AND PHARMACEUTICAL CHEMISTRY, AMERICAN CHEMICAL SOCIETY. EASTON, US, Bd. 35, Nr. 24, 1992, Seiten 4589-4594, XP002265647
- PORTOGHESE P S ET AL: "Synthesis of naltrexone-derived delta-opioid antagonists. Role of conformation of the delta address moiety" JOURNAL OF MEDICINAL CHEMISTRY, AMERICAN CHEMICAL SOCIETY. WASHINGTON, US, Bd. 37, Nr. 5, 1994, Seiten 579-585, XP002952991 ISSN: 0022-2623

## Beschreibung

Die vorliegende Erfindung betrifft eine Klasse von Morphinanverbindungen und deren quartäre Ammoniumsalze substituiert in Position 14, die als hochaktive Analgetika aber auch als Opioidantagonisten verwendet werden können. Die vorliegende Erfindung bezieht sich auch auf deren pharmazeutisch akzeptierbaren Salze und leicht zugänglichen Derivate, auf einen Prozess zu deren Herstellung und deren Verwendung in der Herstellung pharmazeutischer Spezialitäten.

Die Existenz von Opioid-Rezeptoren als Rezeptoren des Zentralnervensystems (ZNS), welche analgetische Wirkung vermitteln, ist eindeutig erwiesen. Diese Rezeptoren werden in drei Subtypen, µ, κ und δ eingeteilt. Aktivierung dieser Rezeptoren durch Opioidagonisten hat einen analgetischen Effekt zur Folge. Die Aktivierung der µ-Rezeptoren bewirkt den höchsten analgetischen Effekt, wobei besonders N-methylsubstituierte Morphinane mit einer Sauerstofffunktion in Position 6 (Morphin, Oxymorphon, Hydromorphon etc.) besonders aktive Opioidagonisten sind und als effektive Analgetika eingesetzt werden. In der Vergangenheit wurde viel Arbeit in Struktur-Aktivitäts-Beziehungsstudien dieser Substanzklasse investiert.

Im Journal of Medicinal Chemistry 1984, 27, S. 1575-1579 sind verschiedene 14-Methoxy-N-methylmorphinan-6-one mit verschiedenen Substituenten in Position 3 beschrieben. Diese Derivate zeigen höhere analgetische Aktivität als deren 14-Hydroxyanaloga.

Eine detaillierte Studie von 5-Methyloxymorphon (= 14-Hydroxy-5-methyldihydromorphinon) ist in Helvetica Chimica Acta (1988, 71, S. 1801-1804) beschrieben, die zu dem Ergebnis gelangt, daß die Einführung einer 5-Methylgruppe die opioidagonistischen Eigenschaften von Oxymorphon verringert.

Eine weitere Studie an 14-Alkoxy-N-methylmorphinan-6-onen ist in Helvetica Chimica Acta 1989, 72, S. 1233-1239 beschrieben, in welcher der Einfluß verschiedener Substituenten in Position 3 und des Aminstickstoffs evaluiert wurden.

Die Deutsche Offenlegungsschrift DE 3412 727 beschreibt 14-Alkoxy-N-methylmorphinan-6-one (14-O-Alkyloxymorphone) mit höherer Aktivität als deren 14-Hydroxy-Analoga.

Wenn anstelle der N-Methylgruppe z. B. eine N-Cyclopropylmethylgruppe, eine N-Allylgruppe oder eine N-Tetrahydrofurfurylgruppe eingeführt wird, so resultieren normalerweise Verbindungen, die antagonistische Wirkungen an Opioidrezeptoren hervorrufen (s. Prog. Med. Chem. 1998, 35, 83-132). Solche Opioidantagonisten heben sowohl die analgetische Wirkung als auch die Nebenwirkungen von Opioidagonisten (z.B. Atemdepression oder Obstipation) auf.

Morphinanderivate und ähnliche heterocyclische Verbindungen sind auch in den folgenden Schriften offenbart:
Cheng, C. Y., et al., Bioorganic & Medicinal Chemistry, Bd. 4, Nr. 1, 1996, Seiten 73 bis 80;
GB-A-1 300 419;
EP-A-0 250 796;
Coop, A., et al., Bioorganic & Medicinal Chemistry Letters, Bd. 9, 1999, Seiten 3435 bis 3438;
Schütz, J., et al., J. Med. Chem. Bd. 45, 2002, Seiten 5378 bis 5383;
US-A-4 272 540;
Schmidhammer, H., et al., Journal of Medicinal Chemistry, American Chemical Society, Washington, US, Bd. 27, Nr. 12, 1984, Seiten 1575 bis 1579;
DE 34 12 727 A;
Klein, P., et al., Journal of Medicinal and Pharmaceutical Chemistry, American Chemical Society, Easton, US, Bd. 35, Nr. 24, 1992, Seiten 4589 bis 4594;
Portoghese, P. S., et al., Jounal of Medicinal Chemistry, American Chemical Society, Washington, US, Bd. 37, Nr. 5,1994, Seiten 579 bis 585;
EP-A-0 030 685;
US-A-4 390 699;
US-A-4 912 114.

Überraschenderweise wurde nun gefunden, dass z.B. N-cyGopropyhmethyl-, N-allyl- oder N-tetrahydrofurfurylsubstituierte Morphinan-6-one mit Arylalkylsubstituenten oder längerkettigen Alkylsubstituenten in Position 14 hochaktive Opioidagonisten mit ausgezeichneter analgetischer Aktivität sind. Die sehr hohe analgetische Aktivität ermöglicht niedrigere Dosierung, was eine niedrigere Nebenwirkungsrate zur Folge hat.

Weiters wurde überraschenderweise gefunden, dass eine Quartemisierung von Morphinan-6-onen, welche Arylalkylsubstituenten oder Alkylsubstituenten in Position 14 aufweisen, einerseits zu Substanzen mit hoher analgetischer Aktivität führt, andererseits aber auch zu Substanzen mit ausgezeichnetem Opioidantagonismus. Die bisher beschriebenen Quartemisierungen von 14-Hydroxymorphinan-6-onen (z.B. Oxymorphon oder Naloxon) führte zu einer signifikanten Abschwächung der Wirksamkeit (s. M. A. lorio et al., Eur. J. Med. Chem. - Chim. Ther. 1984, 19, 301-303). Weiters wurde gefunden, dass diese neuen Substanzen ihre Wirkung in erster Linie in der Peripherie entfalten, da sie die Blut-Hirn-Schranke nicht oder nur in geringem Ausmaß überwinden können.

Die vorliegende Erfindung liefert hochaktive Verbindungen der Formeln (I), (la), (IA) und (IAa) wie in den Ansprüchen 1 und 2 definiert. Bevorzugte Ausführungsformen sind in den Unteransprüchen angegeben, zusammen wit weiteren Aspekten der vorliegenden Erfindung, wie Zusammensetzungen und Verwendungen. Die insbesondere im Zusammenhang mit den Verbindungen gemachten Ausführungen über bevorzugte Ausführungsformen gelten analog auch für die weiteren Aspekte der Erfindung, wie die Zusammensetzungen und Verwendungen.

Zusätzliche bevorzugte Ausführungsformen sind im folgenden definiert.

Verbindungen der Formeln (I) und (la), in denen die Substituenten die folgende Bedeutung haben:
R₁: C₁-C₆-Alkyl; C₂-C₆-Alkenyl; C₂-C₆-Alkinyl; C₄-C₁₆-Cycloalkylalkyl, worin Cycloalkyl C₃-C₁₀ und Alkyl C₁-C₆ ist; C₅-C₁₆-Cycloalkylalkenyl, worin Cycloalkyl C₃-C₁₀ ist und Alkenyl C₂-C₆ ist; C₅-C₁₆-Cycloalkylalkinyl, worin Cycloalkyl C₃-C₁₀ ist und Alkinyl C₂-C₆ ist; C₇-C₁₆-Arylalkyl, worin Aryl C₆-C₁₀-Aryl ist und Alkyl C₁-C₆-Alkyl ist; C₈-C₁₆-Arylalkenyl, worin Aryl C₆-C₁₀-Aryl und Alkenyl C₂-C₆-Alkenyl ist; C₈-C₁₆-Arylalkinyl, worin Aryl C₆-C₁₀-Aryl ist und Alkinyl C₂-C₆-Alkinyl;
R₂: C₄-C₆-Alkyl; C₂-C₆-Alkenyl; C₂-C₆-Alkinyl; C₄-C₁₆-Cycloalkylalkyl, worin Cycloalkyl C₃-C₁₀ ist und Alkyl C₁-C₆ ist; C₅-C₁₆-Cycloalkylalkenyl, worin Cycloalkyl C₃-C₁₀ ist und Alkenyl C₂-C₆ ist; C₅-C₁₆-Cycloalkylalkinyl, worin Cycloalkyl C₃-C₁₀ ist und Alkinyl C₂-C₆ ist; C₇-C₁₆-Arylalkyl, worin Aryl C₆-C₁₀-Aryl ist und Alkyl C₁-C₆-Alkyl ist; C₈-C₁₆-Arylalkenyl, worin Aryl C₆-C₁₀-Aryl und Alkenyl C₂-C₆-Alkenyl ist; C₈-C₁₆-Arylalkinyl, worin Aryl C₆-C₁₀-Aryl ist und Alkinyl C₂-C₆-Alkinyl; C₃-C₆-Alkenoyl; C₃-C₆-Alkinoyl; C₉-C₁₆-Arylalkenoyl, worin Aryl C₆-C₁₀-Aryl ist und Alkenoyl C₃-C₆-Alkenoyl ist; C₉-C₁₆-Arylalkinoyl, worin Aryl C₆-C₁₀-Aryl ist und Alkinoyl C₃-C₆-Alkinoyl ist;
R₃: Wasserstoff; C₁-C₆-Alkyl; C₂-C₆-Alkenyl; C₇-C₁₆-Arylalkyl, worin Aryl C₆-C₁₀-Aryl ist und Alkyl C₁-C₆-Alkyl ist; C₈-C₁₆-Arylalkenyl, worin Aryl C₆-C₁₀-Aryl und Alkenyl C₂-C₆-Alkenyl ist; Alkoxyalkyl, worin Alkoxy C₁-C₆-Alkoxy und Alkyl C₁-C₆-Alkyl ist; CO₂(C₁-C₆-Alkyl); CO₂H; CH₂OH.
R_{4:} Wasserstoff; Hydroxy; C₁-C₆-Alkyloxy; C₂-C₁₀-Alkyloxyalkoxy, worin Alkyloxy C₁-C₄ ist und Alkoxy C₁-C₆-Alkyloxy C₁-C₆-Alkyloxy ist; C₂-C₆-Alkenyloxy; C₂-C₆-Alkinyloxy; C₄-C₁₆-Cycloalkylalkyloxy, worin Cycloalkyl C₃-C₁₀ ist und Alkyl C₁-C₆ ist; C₅-C₁₆-Cycloalkylalkenyloxy, worin Cycloalkyl C₃-C₁₀ ist und Alkenyl C₂-C₆ ist C₅-C₁₆-Cycloalkylalkinyloxy, worin Cycloalkyl C₃-C₁₀ ist und Alkinyl C₂-C₆ ist; C₇-C₁₆-Arylalkyloxy, worin Aryl C₆-C₁₀-Aryl ist und Alkyl C₁-C₆-Alkyl ist; C₈-C₁₆-Arylalkenyloxy, worin Aryl C₆-C₁₀-Aryl und Alkenyl C₂-C₆-Alkenyl ist; C₈-C₁₆-Arylalkinyloxy, worin Aryl C₆-C₁₀-Aryl ist und Alkinyl C₂-C₆-Alkinyl, C₁-C₆-Alkanoyloxy; C₁-C₆-Alkenoyloxy; C₁-C₆-Alkinoyloxy; C₇-C₁₀-Arylalkanoyloxy, worin Aryl C₆-C₁₀-Aryl ist und Alkanoyloxy C₁-C₆-Alkylanoyloxy ist; C₉-C₁₆-Arylalkenoyloxy, worin Aryl C₆-C₁₀-Aryl ist und Alkenoyloxy C₃-C₆-Alkenoyloxy ist; C₇-C₁₆-Arylalkinoyloxy, worin Aryl C₆-C₁₀-Aryl ist und Alkinoyloxy C₃-C₆-Alkinoyloxy ist;
R_{5:} Wasserstoff: Hydroxy; C₁-C₆-Alkyloxy; C₂-C₁₀-Alkyloxyalkoxy, worin Alkyloxy C₁-C₄ ist und Alkoxy C₁-C₆-Alkyloxy C₁-C₆-Alkyloxy ist; C₂-C₆-Alkenyloxy; C₂-C₆-Alkinyloxy; C₄-C₁₆-Cycloalkylalkyloxy, worin Cycloalkyl C₃-C₁₀ ist und Alkyl C₁-C₆ ist; C₅-C₁₆-Cycloalkylalkenyloxy, worin Cycloalkyl C₃-C₁₀ ist und Alkenyl C₂-C₆ ist, C₅-C₁₆-Cycloalkylalkinyloxy, worin Cycloalkyl C₃-C₁₀ ist und Alkinyl C₂-C₆ ist; C₇-C₁₆-Arylalkyloxy, worin Aryl C₆-C₁₀-Aryl ist und Alkyl C₁-C₆-Alkyl ist; C₈-C₁₆-Arylalkenyloxy, worin Aryl C₆-C₁₀-Aryl und Alkenyl C₂-C₆-Alkenyl ist; C₈-C₁₆-Arylalkinyloxy, worin Aryl C₆-C₁₀-Aryl ist und Alkinyl C₂-C₆-Alkinyl; C₁-C₆-Alkanoyloxy; C₇-C₁₆-Arylalkanoyloxy, worin Aryl C₆-C₁₀-Aryl ist und Alkanoyloxy C₁-C₆-Alkylanoyloxy ist;
X ist Sauerstoff,

In der vorliegenden Erfindung bestehen Alkyl, Alkenyl und Alkinyl aus verzweigtem oder unverzweigtem Alkyl, Alkenyl und Alkinyl.
Aryl kann unsubstituiert oder mono-, di- oder trisubstituiert sein unabhängig mit Hydroxy, Halogen, Nitro, Cyano, Thiocyanato, Trifluormethyl, C₁-C₃-Alkyl, C₁-C₃-Alkoxy, CO₂H, CONH₂, CO₂(C₁-C₃-Alkyl), CONH(C₁-C₃-Alkyl), CON(C₁-C₃-Alkyl)₂, CO(C₁-C₃-Alkyl); amino; (C₁-C₃-Monoalkyl)amino, (C₁-C₃-Dialkyl)amino, C₅-C₆-Cycloalkylamino; (C₁-C₃-Alkanoyl)amido, SH, SO₃H, SO₃(C₁-C₃-Alkyl), SO₂(C₁-C₃-Alkyl), SO(C₁-C₃-Alkyl), C₁-C₃-Alkylthio or C₁-C₃-Alkanoylthio.
Die oben angeführten Definitionen für Alkyl, Alkenyl, Alkinyl und Aryl sind für alle Substituenten der vorliegenden Anmeldung gültig.

Enthält die (cyclische gesättigte Gruppe) Heteroatome, so liegen bevorzugt 1 bis 3 Heteroatome vor, insbesondere bevorzugt 1 oder 2 und am meisten bevorzugt ein Heteroatom, ausgewählt bevorzugt unter O, S, N, P und B und stärker bevorzugt unter O, S und N.

In Formeln (IA) und (IAa) haben die Substituenten bevorzugt die folgende Bedeutung:
R₁: C₁-C₆-Alkyl; C₂-C₆-Alkenyl; C₂-C₆-Alkinyl; C₄-C₁₆-Cycloalkylalkyl, worin Cycloalkyl C₃-C₁₀ und Alkyl C₁-C₆ ist; C₅-C₁₆-Cycloalkylalkenyl, worin Cycloalkyl C₃-C₁₀ ist und Alkenyl C₂-C₆ ist; C₅-C₁₆-Cycloalkylalkinyl, worin Cycloalkyl C₃-C₁₀ ist und Alkinyl C₂-C₆ ist; C₇-C₁₆-Arylalkyl, worin Aryl C₆-C₁₀-Aryl ist und Alkyl C₁-C₆-Alkyl ist; C₈-C₁₆-Arylalkenyl, worin Aryl C₆-C₁₀-Aryl und Alkenyl C₂-C₆-Alkenyl ist; C₈-C₁₆-Arylalkinyl, worin Aryl C₆-C₁₀-Aryl ist und Alkinyl C₂-C₆-Alkinyl;
Die beiden Substituenten R₁ können gleich oder verschieden sein;
R₂: C₁-C₆-Alkyl; C₂-C₆-Alkenyl; C₂-C₆-Alkinyl; C₄-C₁₆-Cycloalkylalkyl, worin Cycloalkyl C₃-C₁₀ ist und Alkyl C₁-C₆ ist; C₅-C₁₆-Cycloalkylalkenyl, worin Cycloalkyl C₃-C₁₀ ist und Alkenyl C₂-C₆ ist; C₅-C₁₆-Cycloalkylalkinyl, worin Cycloalkyl C₃-C₁₀ ist und Alkinyl C₂-C₆ ist; C₇-C₁₆-Arylalkyl, worin Aryl C₆-C₁₀-Aryl ist und Alkyl C₁-C₆-Alkyl ist; C₈-C₁₆-Arylalkenyl, worin Aryl C₆-C₁₀-Aryl und Alkenyl C₂-C₆-Alkenyl ist; C₈-C₁₆-Arylalkinyl, worin Aryl C₆-C₁₀-Aryl ist und Alkinyl C₂-C₆-Alkinyl; C₁-C₆-Alkanoyl; C₃-C₆-Alkenoyl; C₃-C₆-Alkinoyl; C₇-C₁₆-Arylalkanoyl, worin Aryl C₆-C₁₀-Aryl und Alkanoyl C₁-C₆-Alkyl, C₉-C₁₆-Arylalkenoyl, worin Aryl C₆-C₁₀-Aryl ist und Alkenoyl C₃-C₆-Alkenoyl ist; C₉-C₁₆-Arylalkinoyl, worin Aryl C₆-C₁₀-Aryl ist und Alkinoyl C₃-C₆-Alkinoyl ist;
R₃: Wasserstoff; C₁-C₆-Alkyl; C₂-C₆-Alkenyl; C₇-C₁₆-Arylalkyl, worin Aryl C₆-C₁₀-Aryl ist und Alkyl C₁-C₆-Alkyl ist; C₈-C₁₆-Arylalkenyl, worin Aryl C₆-C₁₀-Aryl und Alkenyl C₂-C₆-Alkenyl ist; Alkoxyalkyl, worin Alkoxy C₁-C₆-Alkoxy und Alkyl C₁-C₆-Alkyl ist; CO₂(C₁-C₆-Alkyl); CO₂H; CH₂OH.
R_{4:} Wasserstoff; Hydroxy; C₁-C₆-Alkyloxy; C₂-C₁₀-Alkyloxyalkoxy, worin Alkyloxy C₁-C₄ ist und Alkoxy C₁-C₆-Alkyloxy C₁-C₆-Alkyloxy ist; C₂-C₆-Alkenyloxy; C₂-C₆-Alkinyloxy; C₄-C₁₆-Cycloalkylalkyloxy, worin Cycloalkyl C₃-C₁₀ ist und Alkyl C₁-C₆ ist; C₅-C₁₆-Cycloalkylalkenyloxy, worin Cycloalkyl C₃-C₁₀ ist und Alkenyl C₂-C₆ ist; C₅-C₁₈-Cycloalkylalkinyloxy, worin Cycloalkyl C₃-C₁₀ ist und Alkinyl C₂-C₆ ist; C₇-C₁₆-Arylalkyloxy, worin Aryl C₆-C₁₀-Aryl ist und Alkyl C₁-C₆-Alkyl ist; C₈-C₁₆-Arylalkenyloxy, worin Aryl C₆-C₁₀-Aryl und Alkenyl C₂-C₆-Alkenyl ist; C₈-C₁₆-Arylalkinyloxy, worin Aryl C₆-C₁₀-Aryl ist und Alkinyl C₂-C₆-Alkinyl; C₁-C₆-Alkanoyloxy; C₁-C₆-Alkenoyloxy; C₁-C₆-Alkinoyloxy; C₇-C₁₆-Arylalkanoyloxy, worin Aryl C₆-C₁₀-Aryl ist und Alkanoyloxy C₁-C₆-Alkylanoyloxy ist; C₉-C₁₆-Arylalkenoyloxy, worin Aryl C₆-C₁₀-Aryl ist und Alkenoyloxy C₃-C₆-Alkenoyloxy ist; C₇-C₁₆-Arylalkinoyloxy, worin Aryl C₆-C₁₀-Aryl ist und Alkinoyloxy C₃-C₆-Alkinoyloxy ist;
R_{5:} Wasserstoff: Hydroxy; C₁-C₆-Alkyloxy; C₂-C₁₀-Alkyloxyalkoxy, worin Alkyloxy C₁-C₄ ist und Alkoxy C₁-C₆-Alkyloxy C₁-C₆-Alkyloxy ist; C₂-C₆-Alkenyloxy; C₂-C₆-Alkinyloxy; C₄-C₁₆-Cycloalkylalkyloxy, worin Cycloalkyl C₃-C₁₀ ist und Alkyl C₁-C₆ ist; C₅-C₁₆-Cycloalkylalkenyloxy, worin Cycloalkyl C₃-C₁₀ ist und Alkenyl C₂-C₆ ist; C₅-C₁₆-Cycloalkylalkinyloxy, worin Cycloalkyl C₃-C₁₀ ist und Alkinyl C₂-C₆ ist; C₇-C₁₆-Arylalkyloxy, worin Aryl C₆-C₁₀-Aryl ist und Alkyl C₁-C₁₆-Alkyl ist; C₈-C₁₆-Arylalkenyloxy, worin Aryl C₆-C₁₀-Aryl und Alkenyl C₂-C₆-Alkenyl ist; C₈-C₁₆-Arylalkinyloxy, worin Aryl C₆-C₁₀-Aryl ist und Alkinyl C₂-C₆-Alkinyl; C₁-C₆-Alkanoyloxy; C₇-C₁₆-Arylalkanoyloxy, worin Aryl C₆-C₁₀-Aryl ist und Alkanoyloxy C₁-C₆-Alkylanoyloxy ist;
X ist Sauerstoff.

In der vorliegenden Erfindung bestehen Alkyl, Alkenyl und Alkinyl aus verzweigtem oder unverzweigtem Alkyl, Alkenyl und Alkinyl.
Aryl kann unsubstituiert oder mono-, di- oder trisubstituiert sein unabhängig mit Hydroxy, Halogen, Nitro, Cyano, Thiocyanato, Trifluormethyl, C₁-C₃-Alkyl, C₁-C₃-Alkoxy, CO₂H, CONH₂, CO₂(C₁-C₃-Alkyl), CONH(C₁-C₃-Alkyl), CON(C₁-C₃-Alkyl)₂, CO(C₁-C₃-Alkyl); amino, (C₁-C₃-Monoalkyl)amino, (C₁-C₃-Dialkyl)amino, C₅-C₆-Cycloalkylamino; (C₁-C₃-Alkanoyl)amido, SH, SO₃H, SO₃(C₁-C₃-Alkyl), SO₂(C₁-C₃-Alkyl), SO(C₁-C₃-Alkyl), C₁-C₃-Alkylthio or C₁-C₃-Alkanoylthio.
Die oben angeführten Definitionen für Alkyl, Alkenyl, Alkinyl und Aryl sind für alle Substituenten der vorliegenden Anmeldung gültig.

Enthält die (cyclische gesättigte Gruppe) Heteroatome, so liegen bevorzugt 1 bis 3 Heteroatome vor, insbesondere bevorzugt 1 oder 2 und am meisten bevorzugt ein Heteroatom, ausgewählt bevorzugt unter O, S, N, P und B und stärker bevorzugt unter O, S und N.

Die Verbindungen der vorliegenden Erfindung umfassen auch pharmazeutisch und pharmakologisch akzeptierbare Salze der Verbindungen der Formel (I). Entsprechend der vorliegenden Erfindung sind sowohl anorganische als auch organische Salze geeignet. Beispiele für geeignete anorganische Salze für die vorliegende Erfindung sind Hydrochloride, Hydrobromide, Sulfate und Phosphate. Mögliche organische Salze sind zum Beispiel Methansulfonate, Salicylate, Fumarate, Maleinate, Succinate, Aspartate, Citrate, Oxalate und Orotate.

In einer bevorzugten Darstellung der Formel (I) oder (la), wenn X Sauerstoff ist, sind R₁ C₁-C₆-Alkyl, C₂-C₆-Alkenyl, C₄-C₁₆-Cycloalkylalkyl, worin Cycloalkyl C₃-C₁₀ ist und Alkyl C₁-C₆ ist, C₇-C₁₆-Arylalkyl, worin Aryl C₆-Aryl ist und Alkyl C₁-C₃-Alkyl ist; R₂ C₇-C₁₆-Arylalkyl, worin Aryl C₆-C₁₀-Aryl ist und Alkyl C₁-C₆-Alkyl ist; C₈-C₁₆-Arylalkenyl, worin Aryl C₆-C₁₀-Aryl und Alkenyl C₂-C₆-Alkenyl ist; R₃ Wasserstoff oder Methyl; R₄ Hydroxy, Methoxy oder Acetoxy.

Stärker bevorzugte Verbondungen, in denen X Sauerstoff ist, sind solche, bei denen R₁ bis R₄ jeweils wie folgt sind (wobei diese Verbindungen als freie Base/Säure oder als akzeptables Salz vorliegen können):
R₁: Methyl, Ethyl, Propyl, Butyl, Pentyl, Hexyl, insbesondere Methyl, Ethyl, Propyl und am stärksten bevorzugt Methyl
R₂: Phenylmethylen (in den Beispielen wird die Benennung Phenylmethyl verwendet), Phenylethylen, Phenylpropylen, Phenylbutylen, Phenylpentylen, Phenylhexylen, wobei der Phenylkern optional substituiert sein kann, insbesondere Phenylpropylen (in den Beispielen wird dieser Rest auch Phenylpropyl genannt)
R₃: Methyl, Ethyl, Propyl, Butyl, Pentyl, Hexyl, insbesondere Methyl, Ethyl, Propyl und am stärksten bevorzugt Methyl
R₄: OH, Methoxy, Acetoxy, insbesondere OH

Ein Beispiel einer insbesondere bevorzugten Verbindung ist 4,5α-Epoxy-3-hydroxy-5β,17-dimethyl-14β-[(3-phenylpropyl)oxy]morphinan-6-on, insbesondere das Hydrochlorid und das Hydrobromid.

In einer besonders bevorzugten Darstellung der vorliegenden Erfindung wird die Verbindung ausgewählt aus:
17-Allyl-4,5α-epoxy-3-methoxy-14β-(3-phenylpropyloxy)morphinan-6-on
17-Allyl-4,5α-epoxy-3-hydroxy-14β-(3-phenylpropyloxy)morphinan-6-on
17-Allyl-4,5α-epoxy-3-methoxy-5β-methyl-14β-(3-phenylpropyloxy)morphinan-6-on
17-Allyl-4,5α-epoxy-3-hydroxy-5β-methyl-14β-(3-phenylpropyloxy)morphinan-6-on
17-Cyclobutylmethyl-4,5a-epoxy-3-methoxy-14β-(3-phenylpropyloxy)morphinan-6-on
17-Cydobutylmethyl-4,5a-epoxy-3-hydroxy-14β-(3-phenytpropyloxy)morphinan-6-on
17-Cyclobutylmethyl-4,5a-epoxy-3-methoxy-5β-methyl-14β-(3-phenylpropyloxy)morphinan-6-on
17-Cyclobutylmethyl-4,5a-epoxy 3fiydroxy-5β-methyl-14β-(3-phenylpropyloxy)morphinan-6-on
17-Cyclopropylmethyl-4,5α-epoxy-3-methoxy-14β-(3-phenylpropyloxy)morphinan-6-on
17-Cyclopropylmethyl-4,5α-epoxy-3-hydroxy-14β-(3-phenylpropyloxy)morphinan-6-on
17-Cydopropylmethyl-4,5α-epoxy-3-methoxy-5β-methyl-14β-(3-phenylpropyloxy)morphinan-6-on
17-Cyclopropylmethyl-4,5α-epoxy-3-hydroxy-5β-methyl-14β-(3-phenylpropyloxy)morphinan-6-on
4,5α-Epoxy-3-methoxy-5β,17-dimethyl-14β-[(3-phenylpmpyl)oxy]morphinan-6-on
4,5α-Epoxy-3-hydroxy-5β,17-dimethyl-14β-[(3-phenylpropyl)oxy]morphinan-6-on
17-Propyl-4,5α-epoxy-3-methoxy-14β-(3-phenylpropyloxy)morphinan-6-on
17-Propyl-4,5α-epoxy-3-hydroxy-14β-(3-phenylpropyloxy)morphinan-6-on
17-Propyl -4,5α-epoxy-3-methoxy-5β-methyl-14β-(3-phenylpropyloxy)morphinan-6-on
17-Propyl-4,5α-epoxy-3-hydroxy-5β-methyl-14β-(3-phenylpropyloxy)morphinan-6-on
17-Tetrahydrofurfuryl-4,5α-epoxy-3-methoxy-14β-(3-phenylpropyloxy)morphinan-6-on
17-Tetrahydrofurfuryl-4,5α-epoxy-3-hydroxy-14β-(3-phenylpropyloxy)morphinan-6-on
17-Tetrahydrofurfuryl-4,5α-epoxy-3-methoxy-5β-methyl-14β-(3-phenylpropyloxy)morphinan-6-on
17-Tetrahydrofurturyl-4,5α-epoxy-3-hydroxy-5β-methyl-14β-(3-phenylpropyloxy)morphinan-6-on
17-(2-Phenylethyl)-4,5α-epoxy-3-methoxy-14β-(3-phenylpropyloxy)morphinan-6-on
17-(2-Phenylethyl)-4,5α-epoxy-3-hydroxy-14β-(3-phenylpropyloxy)morphinan-6-on
17-(2-Phenylethyl)-4,5α-epoxy-3-methoxy-5β-methyl-14β-(3-phenylpropyloxy)morphinan-6-on
17-(2-Phenylethyl)-4,5α-epoxy-3-hydroxy-5β-methyl-14β-(3-phenylpropyloxy)morphinan-6-on
17-Ethyl-4,5α-epoxy-3-methoxy-14β-(3-phenylpropyloxy)morphinan-6-on
17-Ethyl-4,5α-epoxy-3-hydroxy-14β-(3-phenylpropyloxy)morphinan-6-on
17-Ethyl-4,5α-epoxy-3-methoxy-5β-methyl-14β-(3-phenylpropyloxy)morphinan-6-on
17-Ethyl-4,5α-epoxy-3-hydroxy-5β-methyl-14β-(3-phenylpropyloxy)morphinan-6-on
17-CyGopropylmethyl-4,5α-epoxy-3-hydroxy-14β-[(2-methylbenzyl)oxy]-morphinan-6-on
14β-[(2-Chlorbenzyl)oxy]-17-(cyclopropytmethyl)-4,5α-epoxy-3-hydroxymorphinan-6-on
14β-Benzyloxy-17-cyclopropylmethyl-4,5α-epoxy-3-hydroxymorphinan-6-on
14β-Butoxy-17-cyclopropylmethyl-4,5α-epoxy-3-hydroxymorphinan-6-on
17-Cyclopropylmethyl-4,5α-epoxy-3-hydroxy-14β-[(3-methylbutyl)oxy]morphinan-6-on
4,5α-Epoxy-5β,17-dimethyl-14β-[(3-phenylpropyl)oxy]-3-[(prop-2-inyl)oxy]morphinan-6-on
14β-[(3-Chlorbenzyl)oxy]-4,5α-epoxy-17-methyl-3-[(prop-2-inyl)oxy]morphinan-6-on
4,5α-Epoxy-17-ethyl-3-methoxy-14β-[(3-phenylpropyl)oxy]morphinan-6-on
4,5α-Epoxy-17-ethyl-3-hydroxy-14β-[(3-phenylpropyl)oxy]morphinan-6-on
4,5α-Epoxy-3-hydroxy-14β-[(3-methylbutyl)oxy]-17-propylmorphinan-6-on
5β-Benzyl-14-methoxycodeinon (= 5-Benzyl-7,8-didehydro-4,5α-epoxy-3,14β-dimethoxy-17-methyl-morphinan-6-on)
5β-Benzyl-4,5α-epoxy-3,14β-dimethoxy-17-methylmorphinan-6-on
5β-Benzyl-4,5α-epoxy-3-hydroxy-14β-methoxy-17-methylmorphinan-6-on
4-Hydroxy-3-methoxy-17-methyl-14-[(3-phenylpropyl)oxy]-morphinan-6-on
3,4-Dimethoxy-17-methyl-14-[(3-phenylpropyl)oxy]morphinan-6-on
14β-Benzyloxy-4-hydroxy-3-methoxy-17-methylmorphinan-6-on
14β-Benzyloxy-3,4-dimethoxy-17-methylmorphinan-6-on
4-Hydroxy-3-methoxy-17-methyl-14β-[(2-naphthylmethyl)oxy]morphinan-6-on
3,4-Dimethoxy-17-methyl-14β-[(2-naphtylmethyl)oxy]morphinan-6-on
4-Hydroxy-3-methoxy-5β,17-dimethyl-14β-[(3-pheny)propyl)oxy]morphinan-6-on
3,4-Dimethoxy-5β,17-dimethyl-14β-[(3-phenylpropyl)oxy]morphinan-6-on
14β-Ethoxy-4-hydroxy-3-methoxy-5β,17-dimethylmorphinan-6-on
14β-Ethoxy-3,4-dimethoxy-5β,17-dimethylmorphinan-6-on
14β-Benryloxy-3,4-dimethoxy-5β,17-dimethylmorphinan-6-on

In einer bevorzugten Darstellung der Formel (IA) oder (IAa), wenn X Sauerstoff ist, sind R₁ C₁-C₆-Alkyl, C₂-C₆-Alkenyl, C₄-C₁₆-Cycloalkylalkyl, worin Cycloalkyl C₃-C₁₀ ist und Alkyl C₁-C₆ ist, C₇-C₁₆-Arylalkyl, worin Aryl C₆-Aryl ist und Alkyl C₁-C₃-Alkyl ist; R₂ C₁-C₆-Alkyl, C₂-C₆-Alkenyl, C₇-C₁₆-Arylalkyl, worin Aryl C₆-C₁₀-Aryl ist und Alkyl C₁-C₆-Alkyl ist; C₈-C₁₆-Arylalkenyl, worin Aryl C₆-C₁₀-Aryl und Alkenyl C₂-C₆-Alkenyl ist; R₃ Wasserstoff oder Methyl; R₄ Hydroxy, Methoxy oder Acetoxy.

In einer besonders bevorzugten Darstellung der vorliegenden Erfindung wird die Verbindung ausgewählt aus:
4,5α-Epoxy-3-hydroxy-17,17-dimethyl-6-oxo-14β-[(3-phenylpropyl]oxy]morphinaniumiodid
(17S)-4,5α-Epoxy-17-ethyl-3-hydroxy-17-methyl-6-oxo-14β-[(3-phenylpropyl]oxy]morphinaniumiodid
(17R)-4,5α-Epoxy-3-hydroxy-17-methyl-6-oxo-14β-[(3-phenylpropyl)oxy]-17-[(2-R,S-tetrahydrofurfuran-2-yl)methyl]morphinaniumiodid
(17R)-17-Allyl-4,5α-epoxy-14β-ethoxy-3-hydroxy-17-methyl-6-oxomorphinaniumiodid
(17R)-17-Allyl-4,5α-epoxy 3-hydroxy-14β-methoxy-17-methyl-6-oxomorphinaniumiodid
(17S)-17-Allyl-4,5α-epoxy-3-hydroxy-14β-methoxy-17-methyl-6-oxomorphinaniumiodid
4,5α-Epoxy-3-hydroxy-14β-methoxy-17,17-dimethyl-6-oxo-morphinaniumiodid
5β-Benzyl-14β-(butyloxy)-4,5α-epoxy-3-hydroxy-17,17-dimethyl-6-oxomorphinaniumiodid
(17S)-17-Allyl-5β-benryl-14β-butoxy-4,5α-epoxy-3-hydroxy-17-methyl-6-oxomorphinaniumiodid
14β-Butoxy-4,5α-epoxy-3-hydroxy-17,17-imethyl-6-oxomorphinaniumiodid
(17R)-17-Cyclopropylmethyl-4,5α-epoxy-3-hydroxy-17-methyl-6-oxo-14β-[(3-phenylpropyl)oxy]morphinaniumiodid
(17R)-17-Cyclopropylmethyl-4,5α-epoxy-3-methoxy-17-methyl-6-oxo-14β-[(3-phenylpropyl)oxy]morphinaniumiodid
(17R)-17-Cyclopropylmethyl-4,5α-epoxy-3-hydroxy-17-methyl-6-oxo-14β-[(2-phenylbenzyl)oxy]morphinaniumiodid
(17R)-14β-[(4-Chlorbenzyl)oxy]-17-cyclopropylmethyl-4,5α-epoxy-3-hydroxy-17-methyl-6-oxomorphinaniumiodid
17(R)-4,5α-Epoxy-3-hydroxy-14β-methoxy-17-methyl-6-oxo-17-(2-phenylethyl)morphinaniumiodid

Es wurde nun gefunden, dass die Verbindungen der gegenständlichen Erfindung (Morphinanverbindungen und deren Quartärsalze substituiert in Position 14) wirksame Opioid-Rezeptortiganden repräsentieren und ein hohes therapeutisches Einsatzpotential aufweisen, als Analgetika, Narkotika, als Immunornodulatoren mit immunstimulierender oder immunsuppressiver Wirkung, als Krebstherapeutika, Entzündungshemmer, als Antirheumatika, zur Unterdrückung von Organabstoßung nach Transplantationen, als Diuretika, Anorektika, als Mittel gegen Diarrhoe, Obstipation, Ileus, Pruritis, als Anästhetika oder als neuroprotektive Wirkstoffe.

Die in den Ansprüchen angeführten Verbindungen sind daher potentiell verwendbar zur Behandlung von akuten und chronischen Schmerzen, von funktionellen intestinalerkrankungen, wie Diarrhoe, Abdominalschmerzen, Obstipation, Ileus, zur Behandlung von entzündlichen Intestinalerkrankungen, zur Behandlung von Säugetieren, speziell Menschen, zur Behandlung des Raynaud-Syndroms, zur Behandlung von Leiden bedingt durch Gefäßverengung, zur Behandlung von Dismenorrhoe, Angina pectoris, Herzinfarkt, Emphysem, Bronchialspasmen, chronisch obstruktiver Bronchitis, rheumatischer Beschwerden, Ödemen, Nephrosen, Nephritis in Verbindung mit rheumatischen Erkrankungen, zur Behandlung von Tumoren, Phaeochromozytom, der Addison-Krankheit, Leberzirrhose, chronischer Entzündungen des Dünn- und Dickdarms und Mastdarms, zur Behandlung von Pruritis, Psoriasis, Neurodermitis, zur Suchtentwöhnung von beispielsweise Opioiden, Kokain oder Alkohol, zur Behandlung von Übergewicht, oder zur Behandlung von psychischen Erkrankungen wie Dysphorie, Depression oder Schizophrenie.

Überraschend wurde auch gefunden, dass die Verbindungen der gegenständlichen Erfindung die Blut-Hirn-Schranke nicht oder nur in geringem Ausmaß zu überwinden vermögen, und ihnen daher eine spezielle Bedeutung hinsichtlich ihrer Verwendung als peripher wirksame Therapeutika zukommt, beispielsweise als Medikamente zur Schmerzbehandlung, zur Behandlung oder Vorbeugung der Obstipation verursacht durch Analgetika, zur Behandlung oder Vorbeugung von postoperativem lleus, postpartum Ileus, zur Rheumatherapie, zur Unterdrückung von Organabstoßung nach Transplantationen bei Säugetieren, speziell Menschen, als auch zur Behandlung von Erektionsstörungen. Der limitierte Zugang zum Zentralnervensystem geht einher mit einer sehr verringerten Nebenwirkungsrate, was zentrale Nebenwirkungen wie z.B. Übelkeit, Erbrechen, Sedation, Benommenheit, Verwirrtheit, Atemdepression und Sucht betrifft.

### Herstellung der Verbindungen

Die Verbindungen gemäß der vorliegenden Erfindung, welche durch die Formel (I) oder (la) repräsentiert werden, können mithilfe der folgenden Methoden gewonnen werden:
Ausgehend von Thebain der Formel (II), 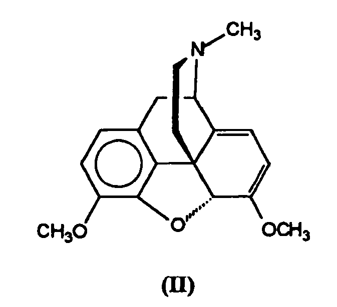
wird diese Verbindung mit Dialkylsulfaten, Fluorsulfonsäurealkylestem, Alkylsulfonsäurealkylestern, Arylsulfonsäurealkylestem, Alkylhalogeniden, Aralkylhalogeniden, Alkylsulfonsäurearalkylestem, Arylsulfonsäurearalkylestern, Arylalkenylhalogeniden, Chlorameisensäureestern oder Ähnlichem umgesetzt in Lösungsmitteln wie Tetrahydrofuran, 1,2-Dimethoxyethan, Diethylether oder Ähnlichem in Gegenwart einer starken Base wie n-Butyllithium, Lithiumdiethylamid, Lithiumdüsopropylamid oder Ähnlichem bei niedrigen Temperaturen (-20° C bis -80° C) (s. Boden et al., J. Org. Chem., Vol. 47, S. 1347-1349, 1982; Schmidhammer et al., Helv. Chim. Acta, Vol. 71, S. 642-647, 1988; Gates et al., J. Org. Chem., Vol. 54, S. 972-975, 1984) um die Verbindungen der Formel (III) erhalten, worin R₃ C₁-C₆-Alkyl ist; C₂-C₆-Alkenyl; C₇-C₁₆-Arylalkyl, worin Aryl C₆-C₁₀-Aryl ist und Alkyl C₁-C₆-Alkyl ist; C₈-C₁₆-Arylalkenyl, worin Aryl C₆-C₁₀-Aryl und Alkenyl C₂-C₆-Alkenyl ist; Alkoxyalkyl, worin Alkoxy C₁-C₆-Alkoxy und Alkyl C₁-C₆-Alkyl ist; CO₂(C₁-C₆-Alkyl); CO₂H; CH₂OH.

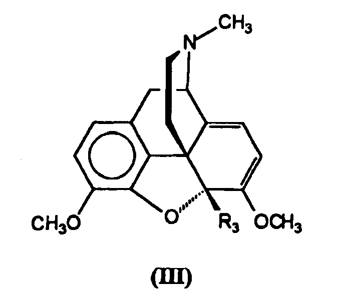

Die Verbindungen der Formel (III) oder Thebain (Formel (II)) können in die entsprechenden 14-Hydroxycodeinone der Formel (IV) umgewandelt werden, 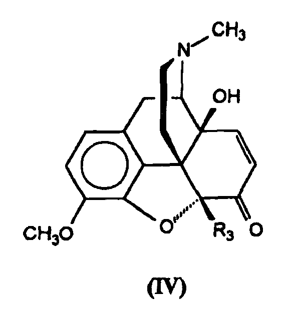 worin R₃ Wasserstoff; C₁-C₆-Alkyl; C₂-C₆-Alkenyl; C₇-C₁₆-Arylalkyl, worin Aryl C₆-C₁₀-Aryl ist und Alkyl C₁-C₆-Alkyl ist; C₈-C₁₆-Arylalkenyl, worin Aryl C₆-C₁₀-Aryl und Alkenyl C₂-C₆-Alkenyl ist; Alkoxyalkyl, worin Alkoxy C₁-C₆-Alkoxy und Alkyl C₁-C₆-Alkyl ist; CO₂ (C₁-C₆-Alkyl) darstellt. Diese Reaktion wird mit Perameisensäure (s. H. Schmidhammer et al., Helv. Chim. Acta, Vol. 71, 1801-1804, 1988), m-Chlorperbenzoesäure oder Ähnlichem durchgeführt, bei Temperaturen zwischen 0°C und 60°C. Die bevorzugte Methode ist die Reaktion mit Perameisensäure bei 0°C bis 40°C.

Diese 14-Hydroxycodeinone der Formel (IV) werden in der Folge mit Dialkylsulfaten, Alkylhalogeniden, Alkenylhalogeniden, Alkinylhalogeniden, Arylalkylhalogeniden, Arylalkenylhalogeniden, Arylalkinylhalogeniden oder Chloroformiaten umgesetzt in Lösungsmitteln wie N,N-Dimethylformamid (DMF) oder Tetrahydrofuran (THF) in Gegenwart einer starken Base wie Natriumhydrid, Kaliumhydrid oder Natriumamid, um die Verbindungen der Formel (V) zu erhalten, 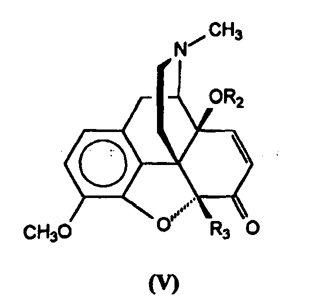 worin R₃ wie oben definiert ist; und R₂ C₁-C₆-Alkyl; C₂-C₆-Alkenyl; C₂-C₆-Alkinyl; C₄-C₁₆-Cycloalkylalkyl, worin Cycloalkyl C₃-C₁₀-Cycloalkyl ist und Alkyl C₁-C₈-Alkyl ist; C₅-C₁₆-Cycloalkylalkenyl, worin Cycloalkyl C₃-C₁₀-Cycloalkyl ist und Alkenyl C₂-C₆-Alkenyl ist; C₅-C₁₆-Cycloalkylalkinyl, worin Cycloalkyl C₃-C₁₀-Cycloalkyl ist und Alkinyl C₂-C₆-Alkinyl ist; C₇-C₁₆-Arylalkyl, worin Aryl C₆-C₁₀-Aryl ist und Alkyl C₁-C₆-Alkyl ist; C₈-C₁₆-Arylalkenyl, worin Aryl C₆-C₁₀-Aryl und Alkenyl C₂-C₆-Alkenyl ist; C₈-C₁₆-Arylalkinyl,
worin Aryl C₆-C₁₀-Aryl ist und Alkinyl C₂-C₆-Alkinyl; C₁-C₆-Alkanoyl; C₃-C₆-Alkenoyl; C₃-C₆-Alkinoyl; C₇-C₁₆-Arylalkanoyl, worin Aryl C₆-C₁₀-Aryl und Alkanoyl C₁-C₆-Alkanoyl; C₉-C₁₆-Arylalkenoyl, worin Aryl C₆-C₁₀-Aryl ist und Alkenoyl C₃-C₆-Alkenoyl ist; C₉-C₁₆-Arylalkinoyl, worin Aryl C₆-C₁₀-Aryl ist und Alkinoyl C₃-C₆-Alkinoyl ist, darstellt.

Diese Verbindungen werden anschließend mittels katalytischer Hydrierung über einem Katalysator wie Pd/C, PdO, Pd/Al₂O₃, Pt/C, PtO₂, Pt/Al₂O₃, oder Ähnlichem in Lösungsmitteln wie Alkoholen, Alkohol/Wasser-Gemischen, Eisessig oder Ähnlichem 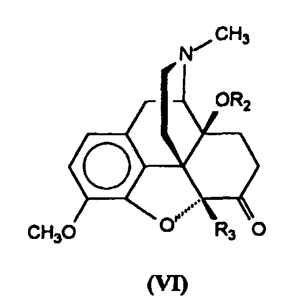 reduziert zu Verbindungen der Formel (VI),
worin R₂ und R₃ wie oben definiert sind.

Die anschließende N-Demethylierung wird mit Chloroformiaten oder Bromcyan in Lösungsmitteln wie 1,2-Dichlormethan, Chloroform oder Ähnlichen durchgeführt, und man erhält Verbindungen der Formel (VII). 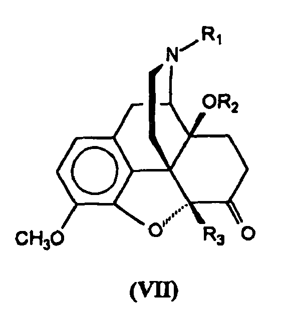 worin R₁CO₂CH(Cl)CH₃, CO₂CH=CH₂, CO₂CH₂CCl₃, CO₂CH₂CH₃, CO₂Ph, CN oder Ähnliches darstellt; und R₂ und R₃ wie oben definiert sind.

Die Carbamate der Formel (VII) werden entweder durch Rückflußerhitzen in Alkoholen (im Falle von 1-Chlorethylcarbamaten) oder durch Zugabe von Hydrogenhalogeniden bzw. Halogenen und anschließendes Rückflußerhitzen in Alkoholen (im Falle von Vinylcarbamaten) gespalten, die Cyanamide der Formel (VII) durch saure oder alkalische Hydrolyse, wodurch N-Nor-Verbindungen der Formel (VIII) erhalten werden, 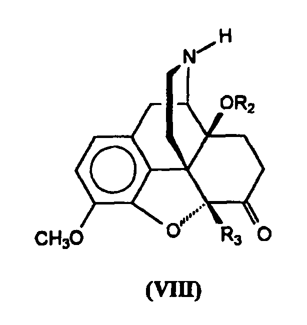 in denen R₂ und R₃ wie oben definiert sind.

Die N-Alkylierung der Verbindungen der Formel (VIII) erreicht man mit Alkylhalogeniden, Dialkylsulfaten, Alkenylhalogeniden, Alkinylhalogeniden, Cycloalkylalkylhalogeniden, Cydoalkenylalkylhalogeniden, Arylalkylhalogeniden, Arylalkenylhalogeniden, Arylalkinylhalogeniden oder Ähnlichem in Lösungsmitteln wie Dichlormethan, Chloroform oder N,N-Dimethylformamid in Gegenwart einer Base wie Natriumhydrogencarbonat, Kaliumcarbonat, Triethylamin oder Ähnlichem und erhält somit Verbindungen der Formel (IX), 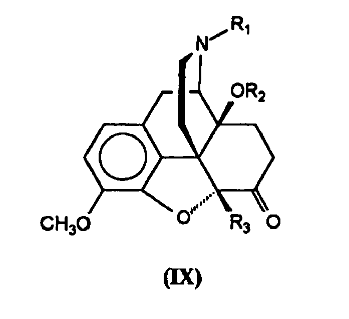 worin R₂ und R₃ wie oben definiert sind; und R₁ C₁-C₆-Alkyl; C₂-C₆-Alkenyl; C₂-C₆-Alkinyl; C₄-C₁₆-Cycloalkylalkyl, worin Cycloalkyl C₃-C₁₀-Cycloalkyl ist und Alkyl C₁-C₆-Alkyl ist; C₅-C₁₆-Cycloalkylalkenyl, worin Cycloalkyl C₃-C₁₀-Cycloalkyl ist und Alkenyl C₂-C₆-Alkenyl ist; C₅-C₁₆-Cycloalkylalkinyl, worin Cycloalkyl C₃-C₁₀-Cycloalkyl ist und Alkinyl C₂-C₆-Alkinyl ist; C₇-C₁₆-Arylalkyl, worin Aryl C₆-C₁₀-Aryl ist und Alkyl C₁-C₆-Alkyl ist; C₈-C₁₆-Arylalkenyl, worin Aryl C₆-C₁₀-Aryl und Alkenyl C₂-C₆-Alkenyl ist; C₈-C₁₆-Arylalkinyl, worin Aryl C₆-C₁₀-Aryl ist und Alkinyl C₂-C₆-Alkinyl darstellt.

Etherspaltung dieser Verbindungen der Formel (IX) mit Bortribromid (in einem Lösungsmittel wie Dichlormethan oder Chloroform) bei 0°C, 48% Bromwasserstoffsäure (Rückflusserhitzen), mit Natriumalkanthiolaten (in einem Lösungsmittel wie N,N-Dimethylformamid) oder mit anderen allgemein bekannten Etherspaltungsreagenzien, gibt phenolische Verbindungen der Formel (X), 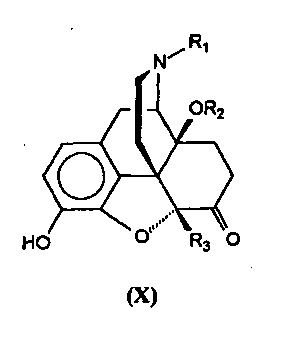 in denen R₁, R₂ und R₃ wie oben definiert sind.

Die 3-O-Alkylierung der Verbindungen der Formel (X) erreicht man mit Alkylhalogeniden, Dialkylsulfaten, Alkenylhalogeniden, Alkinylhalogeniden, Cycloalkylalkylhalogeniden, Cycloalkylalkenylhalogeniden, Arylalkylhalogeniden, Arylalkenylhalogeniden, Arylalkinylhalogeniden oder Ähnlichem in Lösungsmitteln wie Dichlormethan, Chloroform, Aceton oder N,N-Dimethylformamid in Gegenwart einer Base wie Natriumhydrogencarbonat, Kaliumcarbonat, Triethylamin oder Ähnlichem; 3-O-Acylierung der Verbindungen der Formel (X) erreicht man mit Carbonsäurehalogeniden, Carbonsäureanhydriden oder Ähnlichem in Lösungsmitteln wie Dichlormethan, Chloroform, Aceton, N,N-Dimethylformamid, Pyridin oder Ähnlichem und erhält somit Verbindungen der Formel (XI); 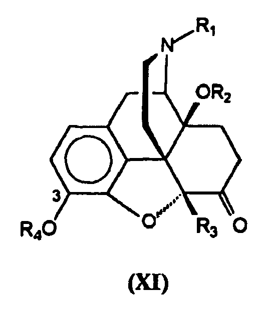 worin R₁, R₂ und R₃ wie oben definiert sind; und R₄ C₁-C₆-Alkyl; C₂-C₆-Alkenyl; C₂-C₆-Alkinyl; C₄-C₁₆-Cydoalkylalkyl, worin Cycloalkyl C₃-C₁₀-Cycloalkyl ist und Alkyl C₁-C₆-Alkyl ist; C₅-C₁₀-Cycloalkylalkenyl, worin Cycloalkyl C₃-C₁₀-Cycloalkyl ist und Alkenyl C₂-C₆-Alkenyl ist; C₅-C₁₆-Cycloalkylalkinyl, worin Cycloalkyl C₃-C₁₀-Cycloalkyl ist und Alkinyl C₂-C₆-Alkinyl ist; C₇-C₁₆-Arylalkyl, worin Aryl C₆-C₁₀-Aryl ist und Alkyl C₁-C₆-Alkyl ist; C₆-C₁₆-Arylalkenyl, worin Aryl C₆-C₁₀-Aryl und Alkenyl C₂-C₆-Alkenyl ist; C₈-C₁₆-Arylalkinyl,
worin Aryl C₆-C₁₀-Aryl ist und Alkinyl C₂-C₆-Alkinyl darstellt; C₁-C₆-Alkanoyl; C₃-C₆-Alkenoyl; C₃-C₆-Alkinoyl; C₇-C₁₆-Arylalkanoyl, worin Aryl C₆-C₁₀-Aryl und Alkanoyl C₁-C₆-Alkyl; C₉-C₁₈-Arylalkenoyl, worin Aryl C₆-C₁₀-Aryl ist und Alkenoyl C₃-C₆-Alkenoyl ist; C₈-C₁₆-Arylalkinoyl, worin Aryl C₆-C₁₀-Aryl ist und Alkinoyl C₃-C₆-Alkinoyl ist.

Eine alternative Route beginnt mit Verbindungen der Formel (XII), in denen R₁ und R₃ wie oben definiert sind (s. Weiss et al., J. Amer. Chem. Soc., Vol. 77, S. 5891, 1955; lijima et al., J. Med. Chem., Vol. 21, S. 398-400, 1978; Coop et al., J. Org. Chem., Vol. 63, S. 4392-4396, 1998; Schmidhammer et al., Helv. Chim. Acta, Vol. 71, S. 1801-1804, 1988; Schmidhammer et al., Helv. Chim. Acta, Vol. 73, S. 1986-1990, 1990).

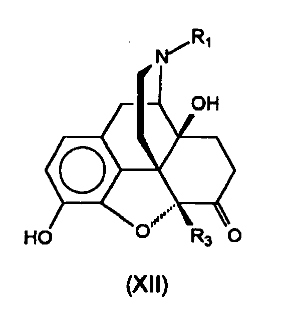

Die Ketone der Formel (XII) werden in Gegenwart von Methansulfonsäure oder Ähnlichem mit Ethylenglykol (als Reagenz und Lösungsmittel) zu Verbindungen der Formel (XIII) umgesetzt, 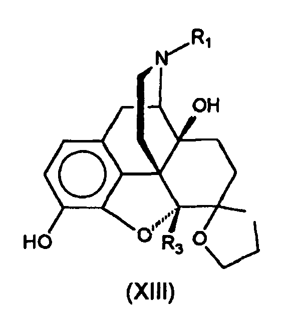 in denen R₁ und R₃ wie oben definiert sind.

Die Einführung einer Schutzgruppe wie z.B. Benzyl, Trityl oder Silyl an der 3-Hydroxygruppe erreicht man durch 3-O-Benzylierung, 3-O-Tritylierung oder 3-0-Silylierung der Verbindungen der Formel (XIII) mit Benzylhalogeniden, Tritylhalogeniden Trialkylhalogensilanen in Lösungsmitteln wie Dichlormethan, Chloroform, Aceton oder N,N-Dimethylformamid in Gegenwart einer Base wie Natriumhydrogencarbonat, Kaliumcarbonat, Triethylamin oder Ähnlichem oder mittels Phasentransferkatalyse und erhält somit Verbindungen der Formel (XIV), 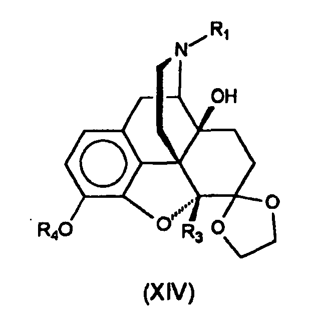 in denen R₁ und R₃ wie oben definiert sind und R₄ eine Schutzgruppe wie z. B. Benzyl, Trityl, Tri-(C1-C6-Alkyl)silyl oder Tris-(C7-C16-Arylalkyl)silyl ist oder eine andere leicht abzuspaltende Schutzgruppe ist.

Diese 14-Hydroxyverbindungen werden in der Folge mit Dialkylsulfaten, Alkylhalogeniden, Alkenylhalogeniden, Alkinylhalogeniden, Arylalkylhalogeniden, Arylalkenylhalogeniden, Arylalkinylhalogeniden oder Chloroformiaten umgesetzt in Lösungsmitteln wie N,N-Dimethylformamid (DMF) oder Tetrahydrofuran (THF) in Gegenwart einer starken Base wie Natriumhydrid, Kaliumhydrid oder Natriumamid, um die Verbindungen der Formel (XV) zu erhalten, 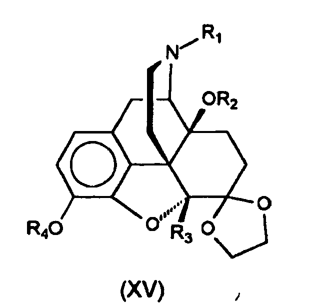 worin R₁, R₂, R₃ und R₄ wie oben definiert sind. Wenn R₂ und R₄ Benzyl sind, können Verbindungen der Formel (XIII) direkt mit 2 Äquivalenten Benzylbromid in DMF in Gegenwart von Natriumhydrid umgesetzt werden, zu 3,14-O-Dibenzylderivaten der Formel (XV), in der R₂ und R₄ Benzyl sind, und R₁ und R₃ wie oben definiert.

Die saure Abspaltung der 3-O-Schutzgruppe und der Ketalfunktion der Verbindungen mit der Formel (XV) wird in einem Schritt mit einer Säure wie Salzsäure in Methanol, Tetrafluoroborsäure in Dichlormethan oder Trifluoressigsäure durchgeführt, und man erhält Verbindungen der Formel (X) (s. 1. Route),

Alternativ dazu kann man, wenn R₄ in Verbindungen der Formel (XV) Benzyl ist, durch Hydrogenolyse der 3-O-Benzylbindung mit Wasserstoffgas in Gegenwart eines Katalysators wie Pd/C, PdO, Pd/Al₂O₃, Pt/C, PtO₂, Pt/Al₂O₃, oder Ähnlichem in Lösungsmitteln wie Alkoholen, Alkohol/Wasser-Gemischen, Eisessig oder Ähnlichem, gefolgt von saurer Hydrolyse der Ketalfunktion in Position 6 mit z.B. Methanol und konzentrierter Salzsäure Verbindungen der Formel (X) erhalten (s. 1. Route), 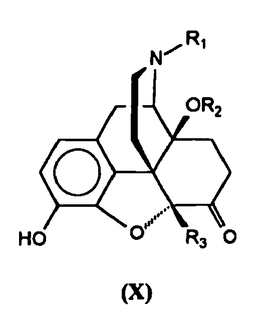 in denen R₁, R₂ und R₃ wie oben definiert sind.

Die Verbindungen der Formel (X) werden gemäß dem ersten Schema zu erfindungsgemäßen Verbindungen der Formel (I) umgesetzt.

Verbindungen der Formel (I) können in Verbindungen der Formel (la), in denen R₅ Wasserstoff; Hydroxy; C₁-C₆-Alkyloxy; C₂-C₁₀-Alkyloxyalkoxy, worin Alkyloxy C₁-C₄ ist und Alkoxy C₁-C₆-Alkyloxy C₁-C₆-Alkyloxy ist; C₂-C₆-Alkenyloxy; C₂-C₆-Alkinyloxy; C₄-C₁₆-Cycloalkylalkyloxy, worin Cycloalkyl C₃-C₁₀ ist und Alkyl C₁-C₆ ist; C₅-C₁₆-Cycloalkylalkenyloxy, worin Cycloalkyl C₃-C₁₀ ist und Alkenyl C₂-C₆ ist; C₅-C₁₆-Cycloalkylalkinyloxy, worin Cycloalkyl C₃-C₁₀ ist und Alkinyl C₂-C₆ ist; C₇-C₁₆-Arylalkyloxy, worin Aryl C₆-C₁₀-Aryl ist und Alkyl C₁-C₆-Alkyl ist; C₈-C₁₆-Arylalkenyloxy,
worin Aryl C₆-C₁₀-Aryl und Alkenyl C₂-C₆-Alkenyl ist; C₈-C₁₆-Arylalkinyloxy, worin Aryl C₆-C₁₀-Aryl ist und Alkinyl C₂-C₆-Alkinyl; C₁-C₈-Alkanoyloxy; C₇-C₁₆-Arylalkanoyloxy, worin Aryl C₆-C₁₀-Aryl ist und Alkanoyloxy C₁-C₆-Alkylanoyloxy ist, folgendermaßen überführt werden:
Verbindungen der Formel (I) können in Verbindungen der Formel (XVI) überführt werden, indem man bei ersteren eine reduktive Öffnung der 4,5-Etherbrücke mit z.B. Zink in einem Alkohol wie Methanol oder Ethanol (bevorzugte Temperatur 40-80 °C) in Gegenwart von Ammoniumchlorid vornimmt. 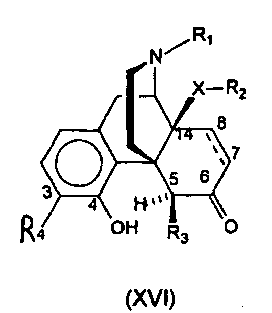
(R₁, R₂ und R₃ sind wie oben angegeben; für R₄ s. Formel (I))

Verbindungen der Formel (XVI) können in die erfindungsgemäßen Verbindungen der Formel (la) in denen R₅ C₁-C₆-Alkyloxy; C₂-C₁₀-Alkyloxyalkoxy, worin Alkyloxy C₁-C₄ ist und Alkoxy C₁-C₆-Alkyloxy C₁-C₆-Alkyloxy ist; C₂-C₆-Alkenyloxy; C₂-C₆-Alkinytoxy; C₄-C₁₆-Cycloalkylalkyloxy, worin Cycloalkyl C₃-C₁₀ ist und Alkyl C₁-C₆ ist; C₅-C₁₆-Cycloalkylalkenyloxy, worin Cycloalkyl C₃-C₁₀ ist und Alkenyl C₂-C₆ ist; C₅-C₁₆-Cycloalkylalkinyloxy, worin Cycloalkyl C₃-C₁₀ ist und Alkinyl C₂-C₆ ist; C₇-C₁₆-Arylalkyloxy, worin Aryl C₆-C₁₀-Aryl ist und Alkyl C₁-C₆-Alkyl ist; C₈-C₁₆-Arylalkenyloxy,
worin Aryl C₆-C₁₀-Aryl und Alkenyl C₂-C₆-Alkenyl ist; C₈-C₁₆-Arylalkinyloxy, worin Aryl C₆-C₁₀-Aryl ist und Alkinyl C₂-C₆-Alkirlyl; C₁-C₆-Alkanoyloxy; C₇-C₁₆-Arylalkanoyloxy, worin Aryl C₆-C₁₀-Aryl ist und Alkanoyloxy C₁-C₆-Alkylanoyloxy ist, und R₁, R₂ und R₃ wie oben angegeben sind und R₄ in Formel (I) angegeben ist, überführt werden durch Alkylierung oder Acylierung nach allgemein üblichen Methoden.
Verbindungen der Formel (XVI) können in die erfindungsgemäßen Verbindungen der Formel (I) in denen R₅ Wasserstoff ist, überführt werden durch Entfernung der Hydroxylgruppe in Position 4. Das kann z.B. geschehen durch Bildung des entsprechenden Phenyltetrazolylethers in Gegenwart einer Base wie Kaliumcarbonat in einem Lösungsmittel wie N,N-Dimethylformamid und anschließende katalytische Hydrierung des Phenyltetrazolylethers z.B. über einem Katalysator wie Pd/C in einem Alkohol oder Eisessig bei Temperaturen zwischen 20 und 100 °C (s. z.B. Schmidhammer et al. J. Med. Chem. 1984, 27, S. 1575-1579).

Verbindungen der Formel (I) oder (Ia) können in quartäre Ammoniumsalze der Formel (IA) oder (IAa) umgewandelt werden durch Alkylierung mit einem entsprechenden Alkylhalogenid oder einem anderen Alkylierungsmittel in Lösungsmitteln wie N,N-Dimethylformamid, Acetonitril, Aceton, Alkoholen, Tetrahydrofuran, Ether, Ethylacetat etc. oder in einem Lösungsmittelgemisch. Die Anordnung des eintretenden Substituenten ist bevorzugt axial (s. M. A. lorio et al., Eur. J. Med. Chem. - Chim. Ther. 1984, 19, 301-303).

Die folgenden Beispiele beschreiben die Herstellung die erfindungsgemäßen Verbindungen im Detail.

### Beispiel 1

### Synthese des 17-Allyl-4,5α-epoxy-3-methoxy-14β-(3-phenylpropyloxy)morphinan-6-on Hydrochlorids (Verbindung 1·HCl).

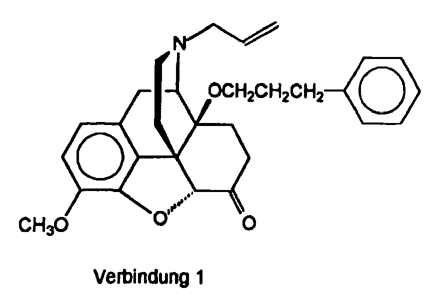

NaH (0,97 g, 40,4 mmol) (erhalten aus 1,62 g 60% NaH-Dispersion in Öl durch Waschen mit Petrolether) wurde einer Lösung von 14-Hydroxycodeinon (lijima et al. J. Med. Chem. 1978, 21, S. 398) (3,00 g, 9,6 mmol) in 100 ml wasserfreiem N,N-Dimethylformamid unter N₂ bei 0° C (Badtemperatur) unter Rühren zugefügt. Nach 20 min wurde Cinnamylbromid (2,28 g, 11,6 mmol) zugesetzt und das Gemisch zunächst für 30 min bei 0° C (Badtemperatur), dann bei nachlassender Kühlung für 4.5 Stunden weiter gerührt. Zum Beenden der Reaktion wurden Eisstückchen zugesetzt, bis keine Wasserstoffentwicklung mehr festzustellen war und das Gemisch auf 100 ml Wasser gegossen. Das abgeschiedene Produkt wurde mit Dichlormethan (3 x 100 ml) extrahiert, die vereinigten organischen Phasen mit 250 ml gesättigter Natriumchloridlösung gewaschen und über Natriumsulfat getrocknet. Nach Entfernen des Lösungsmittels unter vermindertem Druck wurde ein braunes Öl erhalten. Dieses wurde aus Methanol kristallisiert. Ausbeute 2,49 g (60%) farblose Kristalle von 14-Cinnamyloxycodeinon (= 7,8-Didehydro-4,5α-epoxy-3-methoxy-17-methyl-14β-{[(E)-3-phenylprop-2-enyl]oxy}morphinan-6-on. Fp. 216-219 °C; ¹H -NMR (DMSO-d₆, δ in ppm): δ 7,46-7,17 (m, 5 arom. H), 7,19 (d, 1 olef. H, CH-8, J = 10.2 Hz), 6,74 (d, 1 arom. H, J = 8.3 Hz), 6,65 (d, 1 arom. H, J = 8.3 Hz), 6,51-6,59 (m, 1 olef. H, -CH=CH-Ph), 6,34 (trans) und 6,26 (cis) (2 ps-d, 1 olef. H, -CH=CH-Ph), 6,18 (d, 1 olef. H, CH-7, J = 10.2 Hz), 4,85 (s, CH-5), 4,26 und 4,11 (2 dxdxd, 2 H, C-14-OCH₂-), 3,73 (s, CH₃O), 2,36 (s, CH₃N).

Ein Gemisch von 14-Cinnamyloxycodeinon (6,27 g, 14,6 mmol) und 0,32 g Pd/C (10%) in 200 ml Eisessig wurde 3 h bei 30 psi und Raumtemperatur hydriert. Der Katalysator wurde abfiltriert und mit 50 ml Eisessig gewaschen. Das Filtrat wurde eingedampft, Wasser zugesetzt und mit konzentriertem Ammoniak alkalisiert und mit Dichlormethan (3 x 50 ml) extrahiert. Die vereinigten organischen Phasen wurden mit gesättigter Natriumchloridlösung gewaschen (3 x 70 ml), über Natriumsulfat getrocknet und eingedampft. Der ölige Rückstand (6,27 g) wurde aus Methanol kristallisiert. Ausbeute 4,93 g (78%) farblose Kristalle 4,5α-Epoxy-3-methoxy-17-methyt-14β-(3-phenylpropyloxy)morphinan-6-on (= 14-O-(3-Phenylpropyl)oxycodon). Fp. 119-121 °C; ¹H -NMR (CDCl₃, δ in ppm): δ 7,35-7,15 (m, 5 arom. H), 6,68 (d, 1 arom. H, J = 8,2 Hz), 6,60 (d, 1 arom. H, J = 8,2 Hz), 4,63 (s, CH-5), 3,89 (s, CH₃O), 2,34 (s, CH₃N).

Ein Gemisch von 4,5α-Epoxy-3-methoxy-17-methyl-14β-(3-phenylpropyloxy)morphinan-6-on (= 14-O-(3-Phenylpropyl)oxycodon) (5,00 g, 11.50 mmol), Natriumhydrogencarbonat (6,8 g, 80,91 mmol) und Chlorameisensäure-1-chlorethylester (7,5 ml, 67,5 mmol) in 30 ml 1,2-Dichlorethan wurde unter Feuchtigkeitsausschluß bei 60°C (Badtemperatur) unter Rühren rückflußerhitzt. Nach 17 h wurde vom anorganischen Rückstand filtriert, mit Dichlormethan gewaschen und das Filtrat eingedampft. Das entstandene Carbamat wurde nicht isoliert, sondern in 100 ml Methanol gelöst und eine Stunde lang rückflußerhitzt. Die Lösung wurde eingedampft und der Eindampfrückstand, ein farbloses Schaumharz (6,08 g) aus Aceton kristallisiert. Ausbeute 4,91 g (93%) 4,5β-Epoxy-3-methoxy-14β-(3-phenylpropyloxy)morphinan-6-on Hydrochlorid als farblose Kristalle. Fp. 215-218 °C; IR (KBr): 1730 (CO) cm⁻¹; ¹H-NMR (CDCl₃): δ 10,55 und 8,67 (2 s, breit, 2 H, ⁺NH₂), 7,28-7,10 (m, 5 arom. H), 6,75 (d, 1 arom. H, J = 8,2 Hz), 6,71 (d, 1 arom. H, J = 8,2 Hz), 4,41 (s, C5-H), 3,89 (s, CH₃O); El-MS: m/z 419 (M⁺).

Ein Gemisch von 4,5α-Epoxy-3-methoxy-14β-(3-phenylpropyloxy)morphinan-6-on Hydrochlorid (2,00 g, 4,76 mmol), Kaliumcarbonat (4,00 g, 28,9 mmol) und Allylbromid (0,56 ml, 6,62 mmol) in wasserfreiem N,N-Dimethylformamid (15 ml) wurde unter Feuchtigkeitsausschluß und unter Stickstoff bei 80°C (Badtemperatur) 7 h lang gerührt. Nach Filtration vom anorganischen Rückstand, der mit je 50 ml Dichlormethan dreimal gewaschen wurde, wurde das Filtrat unter vermindertem Druck eingedampft. Der ölige Rückstand wurde in 300 ml Dichlormethan gelöst, mit Wasser (3x 200 ml) und gesättigter Natriumchloridlösung (4 x 200 ml) gewaschen, über Natriumsulfat getrocknet und eingedampft. Das erhaltene braune Öl, wurde mittels Säulenchromatographie (Kieselgel; CH₂Cl₂ / MeOH / konz. NH₄OH 250 : 2 : 0,5) gereinigt, der ölige Eindampfrückstand in 50 ml Ether gelöst und durch Zugabe von etherischer HCl bis zur sauren Reaktion gefällt. Das Produkt wurde aus Isopropanol umkristallisiert. Ausbeute 1,50 g (69%) der Verbindung 1.HCl als farblose Kristalle. Fp. 129-130 °C; MS (Cl): 460 (M⁺+1); IR (KBr): 1725 (C=O) cm⁻¹; ¹H -NMR (DMSO-D₆, δ in ppm): δ 8,98 (s, breit, HN⁺), 7,30-7,18 (m, 5 arom. H), 6,89 (d, 1 arom. H, J = 8,4 Hz), 6,83 (d, 1 arom. H, J = 8,4 Hz), 5,89 (m, 1 olef. H), 5,69 (m, 2 olef. H), 4,94 (s, CH-5), 3,81 (s, CH₃O).

### Beispiel 2

### Synthese des 17-Cyclobutylmethyl-4,5α-epoxy-3-methoxy-14β-(3-phenylpropyloxy)morphinan-6-on Hydrochlorids (Verbindung 2·HCl).

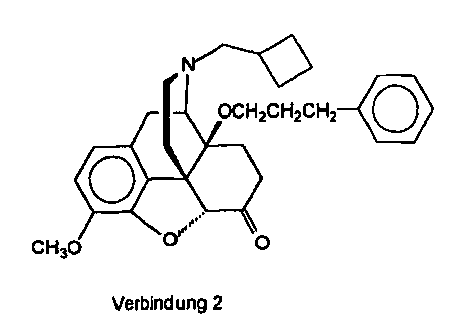

Ein Gemisch von 4,5α-Epoxy-3-methoxy-14β-(3-phenylpropyloxy)morphinan-6-on Hydrochlorid (500 mg, 1,19 mmol), Kaliumcarbonat (1,00 g, 7,24 mmol) und Cyclobutylmethylbromid (0,6 ml, 5,34 mmol) in wasserfreiem N,N-Dimethylformamid (7 ml) wurde unter Feuchtigkeitsausschluß und unter Stickstoff bei 80°C (Badtemperatur) 24 h lang gerührt. Nach Filtration vom anorganischen Rückstand, der mit je 20 ml Dichlormethan dreimal gewaschen wurde, wurde das Filtrat unter vermindertem Druck eingedampft. Der ölige Rückstand wurde in 100 ml Dichlormethan gelöst, mit Wasser (3x 100 ml) und gesättigter Natriumchloridlösung (4 x 100 ml) gewaschen, über Natriumsulfat getrocknet und eingedampft. Der Eindampfrückstand (520 mg braunes Öl) wurde mittels Säulenchromatographie (Kieselgel; CH₂Cl₂ / MeOH / konz. NH₄OH 250 : 2 : 0,5) gereinigt, der ölige Eindampftückstand in 10 ml Ether gelöst und durch Zugabe von etherischer HCl gefällt. Ausbeute 0,40 g (69%) der Verbindung 2.HCl als farblose Kristalle. Fp. 128-133 °C; MS (Cl): 488 (M⁺+1); IR (KBr): 1728 (C=O) cm⁻¹; ¹H -NMR (DMSO-d₆, δ in ppm): δ 8,48 (s, breit, HN⁺), 7,30-7,15 (m, 5 arom. H), 6,87 (d, 1 arom. H, J = 8,4 Hz), 6,77 (d, 1 arom. H, J = 8,4 Hz), 4,93 (s, CH-5), 3,81 (s, CH₃O).

### Beispiel 3

### Synthese des 17-Cyclopropylmethyl-4,5α-epoxy-3-methoxy-14β-(3-phenylpropyloxy)morphinan-6-on Hydrochlorids (Verbindung 3·HCl).

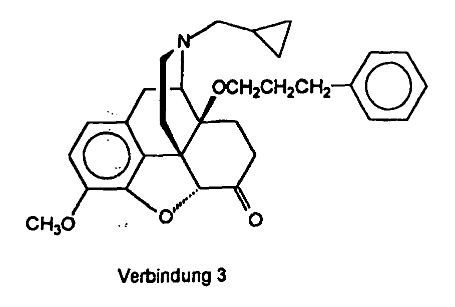

Ein Gemisch von 4,5α-Epoxy-3-methoxy-14β-(3-phenylpropyloxy)morphinan-6-on Hydrochlorid (1,5 g, 3,57 mmol), Kaliumcarbonat (3,00 g, 21,7 mmol) und Cyclopropylmethylbromid (1,0 ml, 6,62 mmol) in wasserfreiem N,N-Dimethylformamid (10 ml) wurde unter Feuchtigkeitsausschluß und unter Stickstoff bei 80°C (Badtemperatur) 4 h lang gerührt. Nach Filtration vom anorganischen Rückstand, der mit je 10 ml Dichlormethan dreimal gewaschen wurde, wurde das Filtrat unter vermindertem Druck eingedampft. Der ölige Rückstand wurde in 50 ml Dichlormethan gelöst, mit Wasser (3x 50 ml) und gesättigter Natriumchloridlösung (4x 50 ml) gewaschen, über Natriumsulfat getrocknet und eingedampft. Der Eindampfrückstand (1,42 g gelbes Öl) wurde mittels Säulenchromatographie (Kieselgel; CH₂Cl₂ / MeOH / konz. NH₄OH 250 : 2 : 0,5) gereinigt, der ölige Eindampfrückstand in 50 ml Ether gelöst und durch Zugabe von etherischer HCl bis zur sauren Reaktion gefällt. Ausbeute 0,60 g (53%) der Verbindung **3.**HCl als farblose Kristalle. Fp. 130-133 °C; MS (El): 473 (M⁺); IR (KBr): 1725 (C=O) cm⁻¹; ¹H -NMR (DMSO-d₆, δ in ppm): δ 8,30 (s, breit, HN⁺), 7,31-7,18 (m, 5 arom. H), 6,88 (d, 1 arom. H, J = 8,4 Hz), 6,78 (d, 1 arom. H, J = 8,4 Hz), 4,96 (s, CH-5), 3,81 (s, CH₃O), 0,80-0,40 (m, 5 cyclopropyl H).

### Beispiel 4

### Synthese des 4,5α-Epoxy-3-methoxy-17-(2-phenylethyl)-14β-(3-phenylpropyloxy)morphinan-6-on Hydrochlorids (Verbindung 4·HCl).

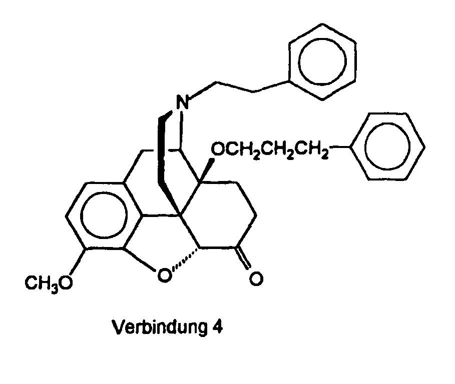

Ein Gemisch von 4,5α-Epoxy-3-methoxy-14β-(3-phenylpropyloxy)morphinan-6-on Hydrochlorid (1,0 g, 2,38 mmol), Kaliumcarbonat (3,00 g, 21,7 mmol) und 2-Phenylethylbromid (1,19 ml, 8,8 mmol) in wasserfreiem N,N-Dimethylformamid (10 ml) wurde unter Feuchtigkeitsausschluß und unter Stickstoff bei 80°C (Badtemperatur) 12 hlang gerührt. Nach Filtration vom anorganischen Rückstand, der mit je 50 ml Dichlormethan dreimal gewaschen wurde, wurde das Filtrat unter vermindertem Druck eingedampft. Der ölige Rückstand wurde in 300 ml Dichlormethan gelöst, mit Wasser (3x 200 ml) und gesättigter Natriumchloridlösung (4x 200 ml) gewaschen, über Natriumsulfat getrocknet und eingedampft. Der Eindampfrückstand (960 mg braunes Öl) wurde mittels Säulenchromatographie (Kieselgel; CH₂Cl₂ / MeOH / konz. NH₄OH 250 : 2 : 0,5) gereinigt, der ölige Eindampfrückstand in 50 ml Ether gelöst und durch Zugabe von etherischer HCl bis zur sauren Reaktion gefällt. Ausbeute 0,45 g (36%) der Verbindung **4**.HCl als farblose Kristalle. Fp. 128-130 °C; MS (Cl): 524 (M⁺+1); IR (KBr): 1726 (C=O) cm⁻¹, ¹H -NMR (DMSO-d₆, δ in ppm): δ 8,84 (s, breit, HN⁺), 7,39-7,17 (m, 10 arom. H), 6,89 (d, 1 arom. H, J = 8,2 Hz), 6,79 (d, 1 arom. H, J = 8,2 Hz), 4,95 (s, CH-5), 3,81 (s, CH₃O).

### Beispiel 5

### Synthese des 4,5α-Epoxy-17-[2-((R,S)-tetrahydrofuran-2-yl)methyl]-3-methoxy-14β-(3-phenylpropyloxy)morphinan-6-on Hydrochlorids (Verbindung 5·HCl).

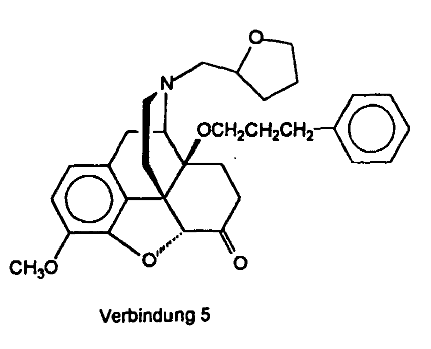

Ein Gemisch von 4,5α-Epoxy-3-methoxy-14β-(3-phenylpropyloxy)morphinan-6-on Hydrochlorid (1,0 g, 2,38 mmol), Kaliumcarbonat (0,6 g, 4,34 mmol) und Tetrahydrofurfurylchlorid (0,5 ml, 4,59 mmol) in wasserfreiem N,N-Dimethylformamid (10 ml) wurde unter Feuchtigkeitsausschluß und unter Stickstoff bei 150°C (Badtemperatur) 15 h lang gerührt. Das Gemisch wurde gekühlt, mit 50 ml Wasser versetzt und mit Dichlormethan extrahiert (3 x 30 ml). Die vereinigten organischen Phasen wurden mit Wasser (3x 60 ml) und gesättigter Natriumchloridlösung (4 x 100 ml) gewaschen, über Natriumsulfat getrocknet und eingedampft. Der Rückstand (1,3 g braunes Öl) wurde mittels Säulenchromatographie (Kieselgel; CH₂Cl₂ / MeOH / konz. NH₄OH 250 : 1,5 : 0,5 → 250: 3,5 : 0,5) gereinigt, der ölige Eindampfrückstand in 50 ml Ether gelöst und durch Zugabe von etherischer HCl bis zur sauren Reaktion gefällt Ausbeute 0,57 g (48%) der Verbindung **5**.HCl als farblose Kristalle. Fp. 121-123 °C; MS (Cl): 504 (M'+1); IR (KBr): 1727 (C=O) cm⁻¹; ¹H-NMR (DMSO-d₆, δ in ppm): δ 8,18 und 7,64 (s, breit, HN⁺), 7.08-6,96 (m, 5 arom. H), 6,68-6,52 (m, 2 arom. H), 4,75 und 4,73 (2 s, CH-5), 3,79 und 3,81 (2 s, CH₃O).

### Beispiel 6

### Synthese des 17-Allyl-4,5α-epoxy-3-hydroxy-14β-(3-phenylpropyloxy)morphinan-6-on Hydrochlorid) (Verbindung 6·HCl).

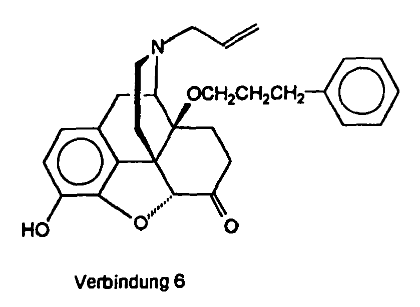

Eine Lösung von Verbindung **1**.HCl (1,50 g, 3,02 mmol) in 5 ml 48% HBr wurde 15 min rückflußerhitzt. Danach wurde die Lösung abgekühlt, auf ca. 50 ml Eis gegossen, mit konz. NH₄OH alkalisiert und mit Dichlormethan (3 x 80 ml)extrahiert Die vereinigten organischen Phasen wurden mit gesättigter Natriumchloridlösung (4 x 50 ml) gewaschen, über Natriumsulfat getrocknet und eingedampft. Der Rückstand (1,3 g braunes Öl) wurde mittels Säulenchromatographie (Kieselgel; CH₂Cl₂ / MeOH / konz. NH₄OH 250 : 2 : 0,5) gereinigt, der ölige Eindampfrückstand in 50 ml Ether gelöst und durch Zugabe von etherischer HCl bis zur sauren Reaktion gefällt. Ausbeute 0,5 g (34%) der Verbindung **6**.HCl als farblose Kristalle. Fp. > 250 °C (Zers.); MS (CI): 446 (M⁺+1); IR (KBr): 1725 (C=O) cm⁻¹; ¹H -NMR (DMSO-d₆, δ in ppm): g 9,51 (s, OH), 8,81 (s, breit, HN⁺), 7,30-7,16 (m, 5 arom. H), 6,71 (d, 1 arom. H, J = 8,0 Hz), 6,64 (d, 1 arom. H, J = 8,0 Hz), 5,92 (m, 1 olef. H), 5,61 (m, 2 olef. H), 4,87 (s, CH-5).

### Beispiel 7

### Synthese des 17-Cyclobutylmethyl-4,5α-epoxy-3-hydroxy-14β-(3-phenylpropyloxy)morphinan-6-on Hydrochlorids (Verbindung 7·HCl).

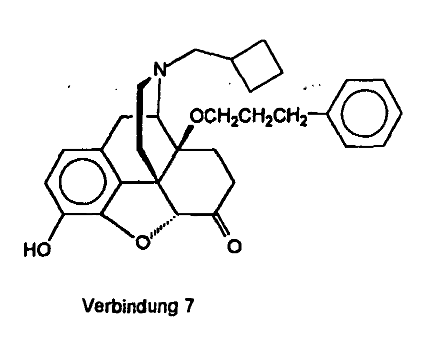

Eine Lösung von Verbindung **2**.HCl (336 mg, 0,64 mmol) in 5 ml 48% HBr wurde 15 min rückflußerhitzt. Danach wurde die Lösung abgekühlt, auf ca. 50 ml Eis gegossen, mit konz. NH₄OH alkalisiert und mit Dichlormethan (3 x 50 ml)extrahiert. Die vereinigten organischen Phasen wurden mit gesättigter Natriumchloridlösung (4 x 50 ml) gewaschen, über Natriumsulfat getrocknet und eingedampft. Der Rückstand (210 mg braunes Schaumharz) wurde mittels Säulenchromatographie (Kieselgel; CH₂Cl₂ / MeOH / konz. NH₄OH 250 : 2 : 0,5) gereinigt, der ölige Eindampfrückstand in 50 ml Ether gelöst und durch Zugabe von etherischer HCl bis zur sauren Reaktion gefällt. Ausbeute 79 mg (34%) der Verbindung **7**.HCl als farblose Kristalle. Fp. > 227 °C (Zers.); MS (Cl): 474 (M⁺+1); IR (KBr): 1725 (C=O) cm⁻¹; ¹H -NMR (DMSO-d₆, δ in ppm): δ 9.51 (s, OH), 8.30 (s, breit, HN⁺), 7,29-7,18 (m, 5 arom. H), 6,70 (d, 1 arom. H, J = 8,4 Hz), 6,63 (d, 1 arom. H, J = 8,4 Hz), 4,86 (s, CH-5).

### Beispiel 8

### Synthese des 17-Cyclopropylmethyl-4,5α-epoxy-3-hydroxy-14β-(3-phenylpropyloxy)morphinan-6-on Hydrochlorids (Verbindung 8·HCl).

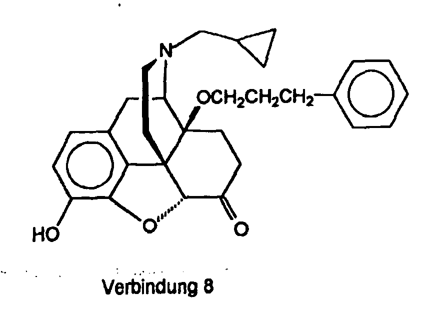

Eine Lösung von Verbindung **3.**HCl (600 mg, 1,26 mmol) in 5 ml 48% HBr wurde 12 min rückflußerhitzt. Danach wurde die Lösung abgekühlt, auf ca. 50 ml Eis gegossen, mit konz. NH₄OH alkalisiert und mit Dichlormethan (3 x 50 ml) extrahiert. Die vereinigten organischen Phasen wurden mit gesättigter Natriumchloridlösung (4 x 50 ml) gewaschen, über Natriumsulfat getrocknet und eingedampft. Das erhaltene braune Öl wurde mittels Säulenchromatographie (Kieselgel; CH₂Cl₂ /MeOH / konz. NH₄OH 250 : 2 : 0,5) gereinigt, der ölige Eindampfrückstand in 50 ml Ether gelöst und durch Zugabe von etherischer HCI bis zur sauren Reaktion gefällt. Umkristallisation aus Isopropanol gab 200 mg (34%) der reinen Verbindung **8**.HCI als farblose Kristalle. Fp. 175-178 °C; MS (CI): 460 (M⁺+1); IR (KBr): 1725 (C=O) cm⁻¹: ¹H -NMR (DMSO-d₆, δ in ppm): δ 9.52 (s, OH), 8,20 (s, breit, HN⁺), 7,30-7,18 (m, 5 arom. H), 6,71 (d, 1 arom. H, J = 8,0 Hz), 6,64 (d, 1 arom. H, J = 8,0 Hz), 4,89 (s, CH-5).

### Beispiel 9

### Synthese des 4,5α-Epoxy-3-hydroxy-17-(2-phenylethyl)-14β-(3-phenylpropyloxy)morphinan-6-on Hydrochlorids (Verbindung 9·HCl).

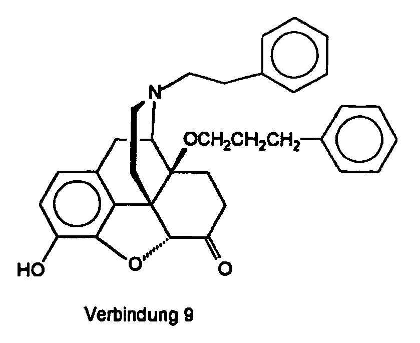

Eine Lösung von Verbindung **4**.HCl (1,79 g, 3,2 mmol) in 5 ml 48% HBr wurde 15 min rückflußerhitzt. Danach wurde die Lösung abgekühlt auf ca. 50 ml Eis gegossen, mit konz. NH₄OH alkalisiert und mit Dichlormethan (3 x 60 ml) extrahiert. Die vereinigten organischen Phasen wurden mit gesättigter Natriumchloridlösung (4 x 60 ml) gewaschen, über Natriumsulfat getrocknet und eingedampft. Das erhaltene braune Öl (1,02 g) wurde mittels Säulenchromatographie (Kieselgel; CH₂Cl₂ / MeOH / konz. NH₄OH 250 : 2 : 0,5) gereinigt, der ölige Eindampfrückstand in 50 ml Ether gelöst und durch Zugabe von etherischer HCl bis zur sauren Reaktion gefällt Ausbeute 247 mg (15%) der reinen Verbindung **9**.HCl als farblose Kristalle. Fp. >218 °C (Zers.); MS (Cl): 510 (M⁺+1); IR (KBr): 1725 (C=O) cm⁻¹; ¹H -NMR (DMSO-d₆, δ in ppm): δ 9,51 (s. OH), 8,84 (s, breit, HN⁺), 7,39-7,17 (m, 10 arom. H), 6,72 (d, 1 arom. H, J = 8,4 Hz), 6,66 (d, 1 arom. H, J = 8,4 Hz), 4,87 (s, CH-5).

### Beispiel 10

### Synthese des 4,5α-Epoxy-17-[2-((R,S)-tetrahydrofuran-2-yl)methyl]-3-hydroxy-14β-(3-phenylpropyloxy)morphinan-6-on Hydrochlorids (Verbindung 10·HCl).

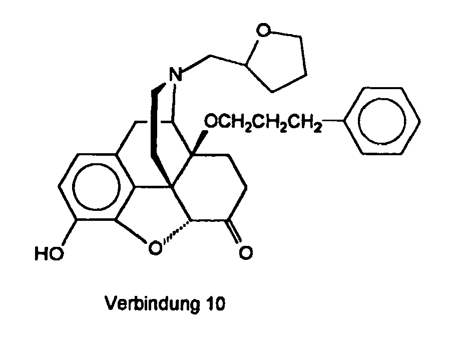

Eine Lösung von Verbindung **5**.HCl (600 mg, 1,11 mmol) in 5 ml 48% HBr wurde 15 min rückflußerhitzt. Danach wurde die Lösung abgekühlt, auf ca. 50 ml Eis gegossen, mit konz. NH₄OH alkalisiert und mit Dichlormethan (3 x 60 ml) extrahiert. Die vereinigten organischen Phasen wurden mit gesättigter Natriumchloridlösung (4 x 60 ml) gewaschen, über Natriumsulfat getrocknet und eingedampft. Das erhaltene braune Öl (0,52 g) wurde mittels Säulenchromatographie (Kieselgel; CH₂Cl₂ / MeOH / konz. NH₄OH 250 : 2 : 0,5) gereinigt, der ölige Eindampfrückstand in 50 ml Ether gelöst und durch Zugabe von etherischer HCl bis zur sauren Reaktion gefälit. Ausbeute 210 mg (36%) der Verbindung **10**.HCl als farblose Kristalle. Fp. 170-172 °C; MS (Cl): 490 (M⁺+1); IR (KBr): 1725 (C=O) cm⁻¹; ¹H -NMR (DMSO-d₆, δ in ppm): δ 9,45 (s, OH), 8,45 und 7,85 (s, breit, HN⁺), 7,30-7,21 (m, 5 arom. H), 6,70-6,65 (m, 2 arom. H), 4,90 und 4,87 (2 s, CH-5).

### Beispiel 11

### Synthese des 4,5α-Epoxy-3-hydroxy-14β-(3-phenylpropyloxy)-17-propylmorphinan-6-on Hydrochlorids (Verbindung 11·HCl).

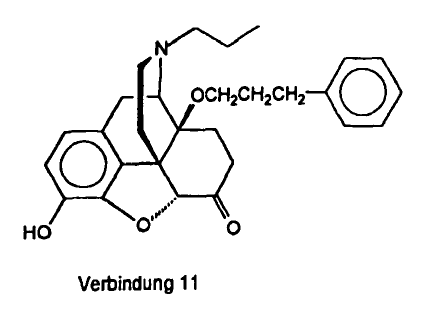

Ein Gemisch von Verbindung **6**.HCl (308 mg, 0,64 mmol) und 35 mg 10 % Pd/C Katalysator in 50 ml Methanol wurde 3 h bei 30 psi und Raumtemperatur hydriert. Der Katalysator wurde abfiltriert und mit 50 ml Methanol gewaschen. Das Filtrat wurde eingedampft. Der Eindampfrückstand, ein farbloses Schaumharz, wurde in 30 ml Ether gelöst und durch Zugabe von etherischer HCl bis zur sauren Reaktion als Hydrochlorid gefällt. Ausbeute: 295 mg (95%) farblose Kristalle der Verbindung **11.**HCl. Fp. 162-163 °C; MS (Cl): 460 (M⁺+₁); IR (KBr): 1725 (C=O) cm-¹; ¹H -NMR (DMSO-d₆, δ in ppm): δ 9,51 (s, OH), 8,50 (s, breit, HN⁺), 7,30-7,18 (m, 5 arom. H), 6,75 (d, 1 arom. H, J = 8,4 Hz), 6,64 (d, 1 arom. H, J = 8,4 Hz), 4,86 (s, CH-5), 0,94 (t, CH₃CH₂CH₂, J = 7 Hz).

### Beispiel 12

### Synthese des 17-Cydopropylmethyl-4,5α-epoxy-3-hydroxy-14β-[(2-methylbenzyl)oxy]-morphinan-6-on Hydrochlorids (Verbindung 12.HCl).

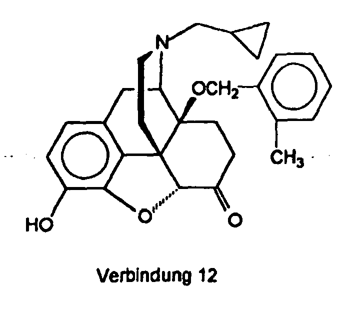

Eine Lösung von Tritylbromid (3,8 g, 11,86 mmol), Triethylamin (2,41 ml, 15,99 mmol), DMAP (14 mg, 0,21 mmol) und 17-Cyclopropylmethyl-4,5α-epoxy-3,14β-dihydroxymorphinan-6-spito-2'-(1,3-dioxolan) (Schmidhammer et al. Heterocycles 1998, 49, 489-497) (3,0 g, 7,78 mmol) in CH₂Cl₂ (80 ml) wurde 24 h rückflußerhitzt, gekühlt und mit H₂O (2 x 100 ml) gewaschen. Die vereinigten organischen Phasen wurden eingedampft und der Rückstand (7,11 g gelbliche Kristalle) aus MeOH (15 ml) umkristallisiert. Ausbeute 4,15 g (85%) 17-Cyclopropylmethyl-4,5α-epoxy-14β-hydroxy-3-triphenylmethoxymorphinan-6-spiro-2'-(1,3-dioxolan). Fp 203-205 °C ; ¹H NMR (CDCl₃) δ 7.50-7.15 (m, 15 arom. H), 6,60 (d, 1 arom. H, J = 8,2 Hz), 6,30 (d, 1 arom. H, J = 8,2 Hz), 6,13 (s, breit, OH), 4,37 (s, CH-5), 4,12-3,80 (m, 4H, OCH₂CH₂O); Cl-MS: m/z 628 (M⁺+1).

NaH (312 mg, 13,34 mmol) (erhalten aus 520 mg einer 60% NaH-Dispersion in Öl durch Waschen mit Petrolether) wurde einer Lösung von 17-Cyclopropylmethyl-4,5α-epoxy-14β-hydroxy-3-triphenylmethoxymorphinan-6-spiro-2'-(1,3-dioxolan) (2,0 g, 3,18 mmol) in wasserfreiem N,N-Dimethylformamid (20 ml) unter N₂ bei 0° C (Badtemperatur) unter Rühren zugesetzt. Nach 20 min wurde 2-Methylbenzylbromid (0,64 ml, 5,4 mmol) zugefügt und das resultierende Gemisch bei Raumtemperatur 20 h gerührt. Überschüssiges NaH wurde durch Zugabe von kleinen Eisstückchen zerstört und dann das Gemisch mit H₂O (80 ml) verdünnt und mit CH₂Cl₂ (1 x 40 ml, 2 x 30 ml) extrahiert. Die vereinigten organischen Phasen wurden mit H₂O (3 x 100 ml) gewaschen, getrocknet (Na₂SO₄), und eingedampft. Der Rückstand (1,8 g orangefarbenes Öl) wurde mittels Säulenchromatographie (Kieselgel; CH₂Cl₂/MeOH/conc. NH₄OH 250 : 2 : 0,5) gereinigt. Es wurden 480 mg eines gelblichen Öls erhalten, welche aus MeOH kristallisiert wurden. Ausbeute 304 mg (13%) reines 17-Cyclopropylmethyl-4,5α-epoxy-14β-[(2-methylbenzyl)oxy]-3-(triphenylmethoxy)morphinan-6-spiro-2'-(1,3-dioxolan). Fp 180-182 °C; ¹H-NMR (Me₂SO-*d*₆): δ 7,30-7,15 (m, 19 arom. H), 6,31 (d, 1 arom. H, J = 8,2 Hz), 6,23 (d, 1 arom. H, J = 8.2 Hz), 4,68 (d, 1 H, OCH₂Ar, J = 10,2 Hz), 4,22 (d, 1H, OCH₂Ar, J = 10,2 Hz), 4,17 (s, CH-5), 4,05-3,50 (m, 4H, OCH₂CH₂O), 2,33 (s, 3H, MePh): CI-MS: *m*/*z* 733 (M⁺+1).

Eine Lösung von 17-Cyclopropylmethyl-4,5α-epoxy-14β-[(2-methylbenzyl)oxy]-3-(triphenylmethoxy)morphinan-6-spiro-2'-(1,3-dioxolan) (300 mg, 0,67 mmol) in MeOH (5 ml) und konz. HCI (0.5 ml) wurde 10 h rückflußerhitzt Nach dem Abkühlen mit Eis wurde die Lösung mit konz. NH₄OH alkalisiert und mit CH₂Cl₂ (1 x 30 ml, 2 x 20 ml) extrahiert. Die vereinigten organischen Phasen wurden mit H₂O gewaschen, über Na₂SO₄ getrocknet und eingedampft. Der Rückstand (165 mg gelbliches Öl) wurde mittels Säulenchromatographie (Kieselgel; CH₂Cl₂/MeOH/conc. NH₄OH 250:2:0.5) gereinigt. Es wurden 80 mg eines farblosen Öls erhalten, das in Ether gelöst und durch Zugabe von etherischer HCl bis zur sauren Reaktion als Hydrochlorid gefällt wurde. Ausbeute 70 mg (23%) reine Verbindung **12**.HCl. Fp 198-200 °C; ¹H NMR (Me₂SO-*d₆*): δ 9,50 (s, OH), 8,60 (s, ⁺NH), 7,67-7,22 (m, 4 arom. H), 6,74 (d, 1 arom. H, J = 8 Hz), 6,69 (d, 1 arom. H, J = 8 Hz), 5,16 (s, C-5 H), 2,31 (s, 3H, MePh); Cl-MS: *m*/*z* 446 (M⁺+1).

### Beispiel 13

### Synthese des 14β-[(2-Chlorbenzyl)oxy]-17-(cyGopropylmethyl)-4,5α-epoxy-3-hydroxymorphinan-6-on Hydrochlorids (Verbindung 13·HCl).

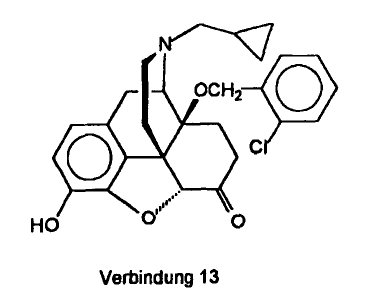

Ein Gemisch von 17-CyGopropylmethyl-4,5α-epoxy-3,14β-dihydroxymorphinan-G-spiro-2'-(1,3-dioxolan) (Schmidhammer et al. Heterocycles 1998, 49, 489-497) (6,9 g, 17,9 mmol), K₂CO, (6,7 g, 48,48 mmol), Benzylbromid (2,34 ml, 19,66 mmol) und wasserfreies N,N-Dimethylformamid (70 ml) wurde unter N₂ und bei Raumtemperatur 22 h gerührt. Das anorganische Material wurde abfiltriert und das Filtrat eingedampft. Der Rückstand wurde in CH₂Cl₂ (80 ml) gelöst, mit H₂O (7 x 50 ml) gewaschen, über Na₂SO₄ getrocknet und eingedampft. Der Rückstand (8,2 g farblose Kristalle) wurde aus MeOH umkristallisiert. Ausbeute 7,37 g (87%) 3-Benzyloxy-17-cyclopropylmethyl-4,5α-epoxy-14f-hydroxymorphinan-6-spiro-2'-(1,3-dioxolan). Fp 130-131 °C; ¹H NMR (CDCl₃): δ 7,42-7,27 (m, 5 arom. H), 6,75 (d, 1 arom. H, J = 8,3 Hz), 6,54 (d, 1 arom. H, J = 8,3 Hz), 5,17 (d, 1H, OCH₂Ph, J = 11,7 Hz), 5,10 (d, 1H, OCH₂Ph, J = 11,7 Hz), 4,58 (s, C-5 H), 4,19-3,73 (m, 4H, OCH₂CH₂O); Cl-MS: *m*/*z* 476 (M⁺+1).

Ein Gemisch von 3-(Benzyloxy)-17-(cyclopropylmethyl)-4,5α-epoxy-14β-hydroxy morphinan-6-spiro-2'-(1,3-dioxolane) (5,82 g, 12,2 mmol), NaH (1,47 g, 61,3 mmol; erhalten aus 2,45 g einer 60% NaH Dispersion in Öl durch Waschen mit Petrolether) and 100 ml wasserfreies N,N-Dimethylformamid wurde unter N₂ bei 0 - 5 °C (Badtemperatur) 25 min gerührt. 2-Chlorbenzylbromid (3,16 ml, 24,3 mmol) wurde zugefügt und das resultierende Gemisch 4 h (1 h bei 0 - 5° C, 3 h bei Raumtemperatur) gerührt. Überschüssiges NaH wurde durch Zugabe von kleinen Eisstückchen zerstört, und dann das Gemisch mit H₂O (350 ml) verdünnt und mit CH₂Cl₂ (1 x 250 ml, 3 x 100 ml) extrahiert. Die vereinigten organischen Phasen wurden mit H₂O (3 x 100 ml) und gesättigter Natriumchloridlösung (2 x 50 ml) gewaschen, getrocknet (Na₂SO₄), und eingedampft. Der Rückstand (8,3 g gelbliches Öl) wurde mittels Säulenchromatographie (Kieselgel; CH₂Cl₂/MeOH/conc. NH₄OH 250 : 4 : 0,5) gereinigt. Es wurden 4.75 g (65%) reines 3-(Benzyloxy)-14β-[(2-chlorobenzyl)oxy]-17-cyclopropylmethyl-4,5α-epoxymorphinan-6-spiro-2'-(1,3-dioxolan) als farbloses erhalten, welches für die nächste synthetische Stufe als solches verwendet wurde. ¹H-NMR (Me₂SO-d₆) δ 7,71-7,31 (m, 9 arom. H), 6,79 (d, 1 arom. H, J = 8,0 Hz), 6,57 (d, 1 arom. H, J = 8,0 Hz), 5,14 (d, 1H, OCH₂Ar, J = 11,6 Hz), 5,07 (d, 1H, OCH₂Ar, J = 11,6 Hz), 4,81 (d, 1H, OCH₂Ar, J = 12 Hz), 4,50 (s, C₅-H), 4,46 (d, 1H, OCH₂Ar, J = 12 Hz), 4,01-3,74 (m, 4H, OCH₂CH₂O).

Eine Lösung von 3-Benzyloxy-14β-[(2-chlorbenzyl)oxy]-17-cyclopropylmethyl-4,5α-epoxymorphinan-6-spiro-2'-(1,3-dioxolan) (5,50 g, 9,16 mmol) in MeOH (52 ml) und konz. HCl (23 ml) wurde 2,5 h rückflußerhitzt. Nach dem Abkühlen mit Eis wurde die Lösung mit konz. NH₄OH alkalisiert und mit CH₂Cl₂ (1 x 200 ml, 1 x 100 ml, 2 x 50 ml)extrahiert. Die vereinigten organischen Phasen wurden mit H₂O gewaschen, über Na₂SO₄ getrocknet und eingedampft. Der Rückstand (5,54 g gelbliches Öl) wurde mittels Säulenchromatographie (Kieselgel; CH₂Cl₂/MeOH/conc. NH₄OH 250 : 3 : 0,5) gereinigt und so 2,69 g (63%) reines 14β-[(2-Chlorobenzyl)oxy]-17-(cyclopropylmethyl)-4,5α-epoxy-3-hydroxymorphinan-6-on (Verbindung **13**) erhalten. Ein Teil wurde in Ether gelöst und mit etherischer HCl versetzt. Es wurde Verbindung **13·**HCl isoliert: Fp 165-168 °C; ¹H-NMR (Me₂SO-*d*₆) δ 8,63 (br. s, OH), 7,96 (s, 1H, ⁺NH), 7,50-7,30 (m, 4 arom. H), 6,74 (d, 1 arom. H, J = 8Hz) 6,69 (d,1 1 arom. H, J = 8 Hz). 5,19 (s, C₅-H), 4,87 (d, 1 H, OCH₂Ar, J =11,6 Hz), 4,81 (d, 1 H, OCH₂Ar, J =11,6 Hz); Cl-MS: *mlz* 466 (M⁺+1).

### Beispiel 14

### Synthese des 14β-Benzyloxy-17-cyclopropylmethyl-4,5α-epoxy-3-hydroxymorphinan-6-ons (Verbindung 14.HBr)

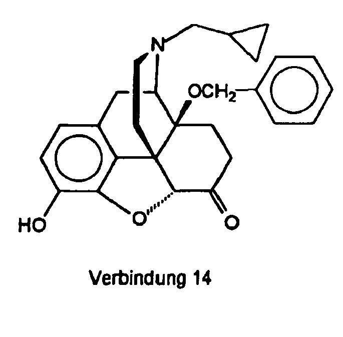

Eine Lösung von 3,14β-Dibenzyloxyl7-cyclopropylmethyl-4,5a-epoxymorphinan-6-spiro-2'-(1,3-dioxolan) (Schmidhammer et al. Heterocycles 1998, 49, 489-497) (10,0 g, 17,68 mmol) in MeOH (200 ml) / H₂O (250 ml) / konz. HCl (50 ml) wurde 4 h rückflußerhitzt. Nach dem Abkühlen mit Eis wurde die Lösung mit konz. NH₄OH alkalisiert und mit CH₂Cl₂ (1 × 100 ml, 2 x 25 ml) extrahiert. Die vereinigten organischen Phasen wurden mit H₂O gewaschen, über Na₂SO₄ getrocknet und eingedampft. Der Rückstand (8,48 g schwach braunes Schaumharz) wurde mittels Säulenchromatographie (Kieselgel; CH₂Cl₂/MeOH/conc. NH₄OH 90: 9: 1) gereinigt und so 6,28 g 3,14β-dibenzyloxy-17-cyclopropylmethyl-4,5α-epoxymorphinan-6-on (Schmidhammer et al. Heterocycles 1998, 49, 489-497) als farbloses Schaumharz und 1,14 g (18%) der Verbindung 14 als schwach gelbes Schaumharz erhalten. Ein Teil der Verbindung **14** wurde in üblicher Weise mittels 48% HBr in das Hydrobromid (Verbindung **14**.HBr) umgewandelt. Fp >210 °C (Zers.); ¹H NMR (Me₂SO-*d*₆) δ 9,48 (s, OH), 8,31 (s, ⁺NH), 7,56-7,32 (m, 5 arom. H), 6,72 (d, 1 arom. H, J = 8 Hz), 6,68 (d, 1 arom. H, J = 8 Hz), 5,10 (s, C-5 H), 4,70 (d, 1H, OCH₂Ph, J =10,4 Hz), 4,25 (d, 1H, OCH₂Ph, J = 10,4 Hz); Cl-MS: *m*/*z* 432 (M⁺+1).

### Beispiel 15

### Synthese des 14β-Butoxy-17-cyclopropylmethyl-4,5α-epoxy-3-hydroxymorphinan-6-on Hydrochlorids (Verbindung 15·HCl)

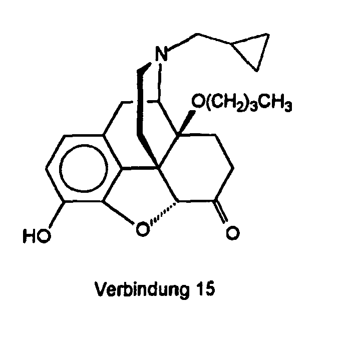

Zu einer eisgekühlten, gerührten Lösung von 3-Benzyloxy-17-cyclopropylmethyl-4,5α-epoxy-14β-hydroxymorphinan-6-spiro-2'-(1,3-dioxolan) (s. Beispiel 13) (10,70 g, 22,5 mmol) in 200 ml wasserfreiem N,N-Dimethylformamid wurde unter Stickstoff NaH (2,8 g, 118 mmol; aus 4,2 g 60%iger NaH-Dispersion durch Waschen mit Petrolether erhalten) hinzugefügt. Nach 15 min wurde trans-Crotylbromid (27 mmol, 3,2 ml einer 85%igen trans-Crotylbromidlösung) zugegeben und 3,5 h lang (1 h bei 0 - 5 °C, 2,5 h bei Raumtemperatur gerührt. Nach der Zerstörung von überschüssigem NaH durch Zugabe von kleinen Eisstücken wurde die Mischung auf 500 ml H₂O gegossen und mit CH₂Cl₂ (3 x 100 ml, 2 x 50 ml) extrahiert. Die vereinigten organischen Phasen wurden mit H₂O (3 x 200 ml) und ges. NaCl-Lösung gewaschen, über Na₂SO₄ getrocknet und eingedampft. Der Eindampfrückstand (13,85 g gelbes Öl) wurde mittels Säulenchromatographie (Kieselgel; CH₂Cl₂ / MeOH / konz. NH₄OH (250/3/0,5) gereinigt. Der Eindampfrückstand (7,11 g gelbes Öl) wurde in Ether gelöst und durch Zugabe von etherischer HCl wurde 3-Benzyloxy-14β-[(E)-(but-2enyl)oxy]-17-cyclopropylmethyl-4,5α-epoxymorphinan-6-spiro-2'-(1,3-dioxolan) als Hydrochlorid gefällt. Ausbeute: 6.85 g (54%). Fp 135 - 139 °C; ¹H-NMR (DMSO-d₆)): δ 8,11 (s, breit, ⁺NH), 7,50 - 7,25 (m, 5 arom. H), 6,92 (d, 1 arom. H, J = 8,4 Hz), 6,68 (d, 1 arom. H, J = 8,4 Hz), 5,79 (m, 2 olefin. H), 5,14 (s, 2 H, C₃OCH₂), 4,59 (s, C₅-H), 4,20 - 3,72 (m, 6 H, C₆-(OCH₂)₂, C₁₄OCH₂), 1,72 (m, 3 H, CH=CHCH₃); MS (Cl): m/z 530 (M⁺ + 1)

Ein Gemisch von 3-Benzyloxy-14β-[(E)-(but-2-enyl)oxy]-17-cyclopropylmethyl-4,5α-epoxymorphinan-6-spiro-2'-(1,3-dioxolan) Hydrochlorid (6,27 g, 11 mmol), 630 mg 10% Pd/C-Katalysator und 100 ml MeOH wurde bei Raumtemperatur und 30 psi 2 h hydriert. Anschließend wurde der Katalysator abfiltriert und das Filtrat im Vakuum eingedampft. Der Eindampfrückstand (rötlich gefärbte Kristalle) wurde mit rückflußkochendem MeOH behandelt. Ausbeute 4,17 g (79%) farblose Kristalle 14β-Butoxy-17-cyclopropylmethyl-4,5α-epoxy-3-hydroxymorphinan-6-spiro-2'-(1,3-dioxolan) Hydrochlorid. Fp 247 - 252 °C; ¹H-NMR (DMSO-d₆): δ 9,23 (s. 1 H, OH). 7,87 (s, breit, 1 H, ⁺NH), 6,68 (d, 1 arom. H, J = 8,2 Hz), 6,57 (d, 1 arom. H, J = 8,2 Hz), 4,49 (s, C₅-H), 4,11 - 3,55 (m, 4 H, C₆(OCH₂)₂), 0,95 (t, 3 H, CH₂CH₃, J = 7,4 Hz); MS (Cl): m/z 443 (M⁺ + 1).

Eine Lösung von 14β-Butoxy-17-cyclopropylmethyl-4,5α-epoxy-3-hydroxymorphinan-6-spiro-2'-(1,3-dioxolan) Hydrochlorid (4,07 g, 8,5 mmol) in 100 ml MeOH / H₂O / konz. HCl (50 : 85 : 15) wurde 4 h rückflußerhitzt. Nach Zugabe von H₂O (70 ml) und Alkalisierung mit konz. NH₄OH wurde mit CH₂Cl₂ (3 x 60 ml) extrahiert. Die vereinigten organischen Phasen wurden mit H₂O und ges. NaCl-Lösung gewaschen, über Na₂SO₄ getrocknet und eingedampft. Der Eindampfrückstand (3,63 g braunes Öl) wurde mittels Säulenchromatographie (Kieselgel, CH₂Cl₂ / MeOH / konz. NH₄OH (250 : 3,5 : 0,5)) gereinigt. Ein Teil des Eindampfrückstandes (2,71 g farbloses Schaumharz, 80%) wurde in Ether gelöst und durch Zugabe von etherischer HCl wurde Verbindung **15**·HCl (2.46 g) als Reinsubstanz erhalten. Fp 240°C (Zers.); IR (KBr): 1725 (CO) cm⁻¹; ¹H-NMR (DMSO-d₆): δ 9,50 (s, OH), 8,15 (s, breit, ⁺NH), 6,71 (d, 1 arom. H, J = 8,0 Hz), 6.65 (d, 1 arom. H. = 8,0 Hz), 4,93 (s, C₅-H), 0,95 (t, 3 H, CH₂CH₃, J = 7,2 Hz); MS (Cl): m/z 399(M⁺+1)

### Beispiel 16

### Synthese des 17-Cyclopropylmethyl-4,5α-epoxy-3-hydroxy-14β-[(3-methylbutyl)oxy]morphinan-6-on Hydrochlorids (Verbindung 16·HCl)

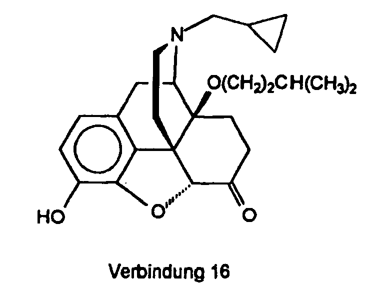

Zu einer eisgekühlten, gerührten Lösung von 3-Benzyloxy-17-cyclopropylmethyl-4,5α-epoxy-14β-hydroxymorphinan-6-spiro-2'-(1,3-dioxolan) (s. Beispiel 13) (4,0 g, 8,4 mmol) in 200 ml wasserfreiem N,N-Dimethylformamid wurde unter Stickstoff NaH (840 m g, 35 mmol; aus 1,4 g 60%iger NaH-Dispersion durch Waschen mit Petrolether erhalten) hinzugefügt. Nach 15 min wurde 3,3-Dimethylallylbromid (1,95 ml, 16,8 mmol) zugegeben und 19 h lang (1 h bei 0 - 5 °C, 18 h bei Raumtemperatur) gerührt. Nach der Zerstörung von überschüssigem NaH durch Zugabe von kleinen Eisstücken wurde die Mischung auf 200 ml H₂O gegossen und mit CH₂Cl₂ (1 x 70 ml, 3 x 40 ml) extrahiert. Die vereinigten organischen Phasen wurden mit H₂O (3 x 150 ml) und ges. NaCI-Lösung gewaschen, über Na₂SO₄ getrocknet und eingedampft. Der Eindampfrückstand (4,8 g gelbbraunes Öl) wurde mittels Säulenchromatographie (Kieselgel; CH₂Cl₂ /MeOH/ konz. NH₄OH 250 : 3 : 0,5) gereinigt.
Das erhaltene Öl wurde in Ether gelöst und durch Zugabe von etherischer HCI wurde 3-Benzyloxy-17-cyclopropylmethyl-4,5α-epoxy-14β-[(3-methylbut-2-enyl)oxy]morphinan-6-spiro-2'-(1,3-dioxolan) Hydrochlorid als Reinsubstanz erhalten. Ausbeute 3,81 g (78%). Fp 126 -129 °C; ¹H-NMR (DMSO-d₆): δ 8,15 (s, breit, ⁺NH), 7,45 - 7,25 (m, 5 arom. H), 6,92 (d, 1 arom. H, J = 8,0 Hz), 6,68 (d, 1 arom. H, J = 8,0 Hz), 5.51 (m, 1 olefin. H), 5,14 (s, 2 H, C₃OCH₂), 4,58 (s, C₅-H), 3,92 - 3,44 (m, 4 H, C₆(OCH₂)₂), 1.76 (s, 3 H, CH=CCH₃), 1,67 (s, 3 H, CH=CCH₃); MS (CI): m/z 544 (M⁺ + 1).

Ein Gemisch von 3-Benzyloxy-17-cyclopropylmethyl-4,5α-epoxy-14β-[(3-methylbut-2-enyl)oxy]morphinan-6-spiro-2'-(1,3-dioxolan) Hydrochlorid (4,01 g, 6,9 mmol), 400 mg 10%iger Pd/C-Katalysator und 100 ml MeOH wurde bei Raumtemperatur und 30 psi 2,5 h hydriert. Anschließend wurde der Katalysator abfiltriert und das Filtrat eingedampft. Der Eindampfrückstand wurde mit Ether behandelt, der unlösliche Anteil abfiltriert, und das Filtrat mit etherischer HCl versetzt und so 17-Cyclopropylmethyl-4,5α-epoxy-3-hydroxy-14β-[(3-methylbutyl)oxy]morphinan-6-spiro-2'-(1,3-dioxolan) Hydrochlorid als Reinsubstanz erhalten. Ausbeute 2,24 g (66%). Fp191 -193 °C; ¹H-NMR (DMSO-d₆): δ 9,22 (s, OH), 7,83 (s, breit, ⁺NH), 6,69 (d, 1 arom. H, J = 8,0 Hz), 6.58 (d, 1 arom. H, J = 8,0 Hz), 4,47 (s, C₅-H),
4,08 - 3,50 (m, 4 H, C₆(OCH₂)₂), 0,96 (d, 3 H, CHCH₃, J = 4,8 Hz), 0,92 (d, 3 H, CHCH₃, J = 4,8 Hz); MS (Cl): m/z 457 (M⁺ + 1).

Eine Lösung von 17-Cyclopropylmethyl-4,5α-epoxy-3-hydroxy-14β-[(3-methylbutyl)oxy]morphinan-6-spiro-2'-(1,3-dioxolan) Hydrochlorid (2,8 g, 5,7 mmol) in 110 ml MeOH / H₂O / konz. HCl (4 : 5:1) wurde 4,5 h rückflußerhitzt. Nach Zugabe von H₂O (80 ml) und Alkalisierung mit konz. NH₄OH wurde mit CH₂Cl₂ (1 x 80 ml, 3 x 50 ml) extrahiert. Die vereinigten organischen Phasen wurden mit H₂O und ges. NaCl-Lösung gewaschen, über Na₂SO₄ getrocknet und eingedampft. Der Eindampfrückstand (2,5 g braunes Öl) wurde mittels Säulenchromatographie (Kieselgel; CH₂Cl₂ / MeOH / konz. NH₄OH (250 : 3 : 0.5)) gereinigt. Der Eindampfrückstand (2,21 g weißes Schaumharz) wurde in Ether gelöst und durch Zugabe von etherischer HCl wurde Verbindung **16**.HCl als Reinsubstanz erhalten. Ausbeute 1,99 g (78%). Fp 168 -170 °C; IR (KBr): 1725 (CO) cm⁻¹; ¹H-NMR (DMSO-d₆): δ 9,50 (s, OH), 8,08 (s, breit, ⁺NH), 6,72 (d, 1 arom. H, J = 8,0 Hz), 6,66 (d, 1 arom. H, J = 8,0 Hz), 4,92 (s, C₅-H), 0,96 (d, 3 H, CHCH₃, J = 3,8 Hz), 0,93 (d, 3 H, CHCH₃, J = 3,8 Hz); MS (Cl): m/z 412 (M⁺ + 1).

### Beispiel 17

### Synthese des 4,5α-Epoxy-5β,17-dimethyl-14β-[(3-phenylpropyl)oxy]-3-[(prop-2-inyl)-oxy]morphinan-6-on Hydrochlorids (Verbindung 17.HCl)

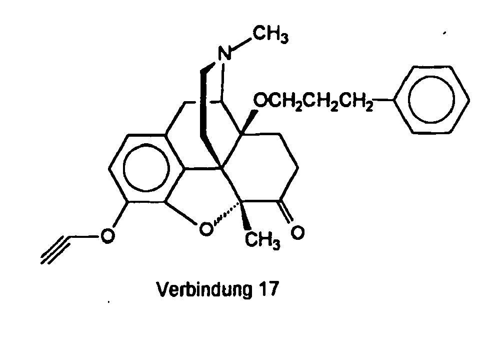

NaH (0,72 g, 30,0 mmol; erhalten aus 1,2 g 60% NaH-Dispersion in Öl durch Waschen mit Petrolether) wurde einer Lösung von 14-hydroxy-5-methylcodeinon (Schmidhammer et al. Helv. Chim Acta 1988, 73, S.642) (5,00 g, 15,27 mmol) in 30 ml wasserfreiem N,N-Dimethylformamid unter N₂ bei 0° C (Badtemperatur) unter Rühren zugefügt. Nach 25 min wurde eine Lösung von Cinnamylbromid (3,01 g, 15,27 mmol) in 20 ml N,N-Dimethylformamid zugetropft und das Gemisch 1,5 h bei 0° C (Badtemperatur) gerührt. Es wurden Eisstückchen zugesetzt, bis keine Wasserstoffentwicklung mehr festzustellen war, das Gemisch wurde auf 500 ml Wasser gegossen und mit Dichlormethan/Isopropanol (4:1) (1 x 150 ml, 3 x 50 ml) extrahiert. Die vereinigten organischen Phasen wurden mit H₂O (5 x 400 ml) und 1 x mit 250 ml gesättigter Natriumchloridlösung gewaschen, über Natriumsulfat getrocknet und eingedampft. Der Eindampfrückstand (7,59 orangefärbiges Öl) wurde aus Isopropanol/Ether (1:1) (20 ml) kristallisiert. Ausbeute 3,03 g (45%) farblose Kristalle von 14-Cinnamyloxy-5-methylcodeinon (= 7,8-Didehydro-4,5α-epoxy-3-methoxy-5β,17-dimethyl-14β-{[(E/Z)-3-phenylprop-2-enyl]oxy}morphinan-6-on). Fp. >150 °C (Zers.); IR (KBr): 1675 (CO) cm⁻¹; ¹H -NMR (DMSO-d₆, δ in ppm): δ 7,44-7,23 (m, 5 arom. H, 1 olef. H), 6,70 (d, 1 arom. H, J = 8.2 Hz), 6,62 (d, 1 arom. H, J = 8.2 Hz), 6,56 (d, 1 olef. H, -CH=CH-Ph, J = 15,7 Hz)), 6,33 (dt, 1 blef. H, -CH=CH-Ph, J = 15,7 Hz), 6,10 (d, 1 olef. H, J = 10.0 Hz), 4,26 und 4,11 (2 dd, 2 H, C-14-OCH₂-, J = 5,0 und 10,6 Hz), 3,70 (s, CH₃O), 2,36 (s, CH₃N), 1,61 (s, C5-CH₃); Cl-MS: m/z 444 (M⁺+1).

Ein Gemisch von 14-Cinnamyloxy-5-methylcodeinon (3,44 g, 7,76 mmol), 10% Pd/C-Katalysator (350 mg) und 80 ml EtOH wurden 2 h bei einer Temperatur von 50 °C und 50 psi hydriert. Der Katalysator wurde abfiltriert, das Filtrat eingedampft und der Rückstand (3,31 dunkles Öl) aus 10 ml EtOH kristallisiert. Ausbeute 2,52 g (73%) 4,5α-Epoxy-3-methoxy-5β,17-dimethyl-14β-[(3-phenylpropyl)oxy]morphinan-6-on (Verbindung **17a**). Fp. 126-128 °C; IR (KBr): 1720 (CO) cm⁻¹; ¹H -NMR (CDCl₃, δ in ppm): δ 7,32-7,17 (m, 5 arom. H), 6,67 (d, 1 arom. H, J = 8.2 Hz), 6,58 (d, 1 arom. H, J = 8.2 Hz), 3,87 (s, CH₃O), 2,34 (s, CH₃N), 1,65 (s, C5-CH₃); CI-MS: m/z 448 (M⁺+1).

Eine Lösung von 4,5α-Epoxy-3-methoxy-5β,17-dimethyl-14β-[(3-phenylpropyl)oxy]morphinan-6-on (2,23 g, 4,98 mmol) in 13 ml 48% HBr wurde 20 min rückflußerhitzt, dann abgekühlt und eingedampft. Der Eindampfrückstand wurde in 20 ml MeOH gelöst und eingedampft. Dieser Vorgang wurde 1 x wiederholt, wobei 2,48 g beige Kristalle erhalten wurden, welche aus 15 ml MeOH umkristallisiert wurden. Ausbeute 2,26 g (88%) 4,5α-Epoxy-3-hydroxy-5β,17-dimethyl-14β-[(3-phenylpropyl)oxy]morphinan-6-on Hydrobromid (Verbindung **17b.**HBr). Fp. > 270 °C (Zers.); IR (KBr): 1720 (CO) cm⁻¹; ¹H -NMR (DMSO-d₆, δ in ppm): δ 9,42 (s, OH), 8,58 (s, ⁺NH), 7,36-7,16 (m, 5 arom. H), 6,69 (d, 1 arom. H, J = 8,2 Hz), 6,64 (d, 1 arom. H, J = 8,2 Hz), 2,97 (s, CH₃N), 1,65 (s, C5-CH₃); Cl-MS: m/z 434 (M⁺+1).

Ein Gemisch aus 4,5α-Epoxy-3-hydroxy-5β,17-dimethyl-14β-[(3-phenylpropyl)oxy]morphinan-6-on Hydrobromid (380 mg, 0,88 mmol), Kaliumcarbonat (370 mg, 2,68 mmol), Propargylbromid (0,20 ml, 2,66 mmol) und 25 ml Aceton wurde unter N₂ 7 h rückflußerhitzt. Das anorganische Material wurde abfiltriert, das Filtrat eingedampft und der Rückstand (460 mg orangefarbenes Öl) mittels Säulenchromatographie (Kieselgel; CH₂Cl₂ / MeOH / konz. NH₄OH 250 : 2 : 0,5) gereinigt Der Eindampfrückstand (350 mg gelbes Öl) wurde in Ether gelöst und mit etherischer HCl versetzt, wobei 220 mg (49%) der Verbindung **17**.HCl erhalten wurden. Fp. >130-133 °C; IR (KBr): 1727 (CO) cm⁻¹;¹H -NMR(DMSO-d₆, δ in ppm): 8.938 (s, ⁺NH), 7,34-7,19 (m, 5 arom. H), 6,92 (d, 1 arom. H, J = 8,4 Hz), 6,78 (d, 1 arom. H, J = 8,4 Hz), 4,81 (d, 2 H, HCCCH₂O-C3, J =1 Hz), 3,59 (d, 1 H, HCCCH₂O-C₃, J = 1 Hz). 2,94 (s, CH₃N), 1,53 (s, C5-CH₃); Cl-MS: m/z 472 (M⁺+1).

### Beispiel 18

### Synthese des 14β-[(3-Chlorbenzyl)oxy]-4,5α-epoxy-17-methyl-3-[(prop-2-inyl)oxy]-morphinan-6-on Hydrochlorids (18·HCl)

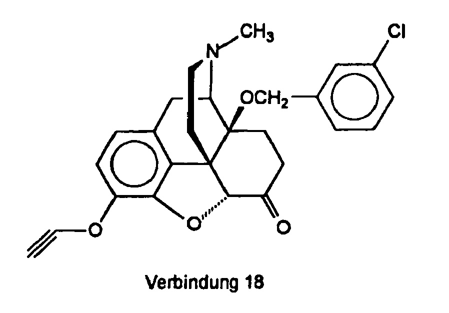

NaH (1,60 g, 66,76 mmol; aus 2,67 g 60%iger NaH-Dispersion durch Waschen mit Petrolether erhalten) wurde einer Lösung von 4,5α-Epoxy-14β-hydroxy-3-methoxy-17-methylmorphinan-6-spiro-2'-(1,3-dioxolan) (Laster et al. Tetrahedron 1965, 21. S. 771-778) (6,00 g. 16,69 mmol) in 100 ml wasserfreiem N,N-Dimethylformamid unter N₂ bei 0_{°} C (Badtemperatur) unter Rühren zugefügt. Nach 15 min wurde 3-Chlorbenzylbromid (4,4 ml, 33,38 mmol) zugegeben und 24 h lang (1 h bei 0 - 5 °C, 23 h bei Raumtemperatur) gerührt. Nach der Zerstörung von überschüssigem NaH durch Zugabe von kleinen Eisstücken wurde die Mischung auf 300 ml H₂O gegossen und mit CH₂Cl₂ (1 x 100 ml, 3 x 40 ml) extrahiert. Die vereinigten organischen Phasen wurden mit H₂O (4 x 200 ml) und ges. NaCl-Lösung gewaschen, über Na₂SO₄ getrocknet und eingedampft. Der Eindampfrückstand (13,36 g gelbes Öl) wurde aus MeOH kristallisiert und umkristallisiert. Ausbeute 4,29 g (53%) 14β-[(3-Chlorbenzyl)oxy]-4,5α-epoxy-3-methoxy-17-methylmorphinan-6-spiro-2'-(1,3-dioxolan) als farbloses Kristallisat. Fp. 131 - 133 °C; ¹H-NMR (DMSO-d₆): δ 7,58-7,26 (m, 4 arom. H), 6,74 (d, 1 arom. H, J = 8.1 Hz), 6,60 (d, 1 arom. H, J = 8,1 Hz), 4,55 (d, 1 H, C₁₄OCH₂, J = 11,8 Hz), 4.31 (d, 1 H, C₁₄OCH₂, J = 11,8 Hz), 4,42 (s, C₅-H), 4,10 - 3,61 (m, 4 H, C₆(OCH₂)₂), 3,78 (s, OCH₃), 2.30 (s, NCH₃); MS (Cl): m/z 485 (M⁺ + 1).

Ein Gemisch von 14β-[(3-Chlorbenzyl)oxy]-4,5α-epoxy-3-methoxy-17-methylmorphinan-6-spiro-2'-(1,3-dioxolan) (3,00 g, 6,2 mmol), Natriumethanthiolat (2,32 g, 27.6 mmol) und 30 ml wasserfreiem N,N-Dimethylformamid wurde 5 h lang unter N₂ bei 130 °C (Badtemperatur) gerührt und dann auf 400 ml H₂O gegossen und mit CH₂Cl₂ (1 x 100 ml, 3 x 60 ml) extrahiert. Die vereinigten organischen Phasen wurden mit H₂O (4 x 200 ml) und ges. NaCl-Lösung gewaschen, über Na₂SO₄ getrocknet und eingedampft. Der Eindampfrückstand (3,36 g dunkelbraunes Öl 14β-[(3-Chlorbenzyl)oxy]-4,5α-epoxy-3-hydroxy-17-methylmorphinan-6-spiro-2'-(1,3-dioxolan)) konnte nicht zur Kristallisation gebracht werden. Daher wurde ein Teil (2,8 g) ohne weitere Reinigung zur Darstellung von 14β-[(3-Chlorbenzyl)oxy]-4,5α-epoxy-3-hydroxy-17-methylmorphinan-6-on verwendet. Der Rest (414 mg) wurde mittels Säulenchromatographie (Kieselgel; CH₂Cl₂ / MeOH / konz NH₄OH (250:2:0,5) gereinigt. Der Eindampfrückstand (352 mg gelbes Öl) wurde in Ether gelöst und durch Zugabe von etherischer HCl wurde 14β-[(3-Chlorbenzyl)oxy]-4,5α-epoxy-3-hydroxy-17-methylmorphinan-6-spiro-2'-(1,3-dioxolan) Hydrochlorid (250 mg) als Reinsubstanz gefällt. Fp. 202-205°C; ¹H-NMR (DMSO-d₆): δ 9,28 (s, OH), 8,92 (s, breit, ⁺NH), 7,60 - 7,36 (m, 4 arom. H), 6,70 (d, 1 arom. H, J = 8,2 Hz), 6,60 (d, 1 arom. H, J = 8,2 Hz), 4,59 (s, C₅-H), 4,76 (d, 1 H, C₁₄OCH₂, J = 11, 9 Hz), 4,57 (d, 1 H, C₁₄OCH₂, J = 11, 9 Hz), 4,11 - 3,61 (m, 4 H, C₆(OCH₂)₂), 2,88 (d, ⁺NCH₃, J = 4,6 Hz); MS (Cl): m/z 471 (M⁺+1).

Eine Lösung von ungereinigtem 14β-[(3-Chlorbenzyl)oxy]-4,5a-epoxy-3-hydroxy-17-methylmorphinan-6-spiro-2'-(1,3-dioxolan) (2,8 g dunkelbraunes Öl) in 100 ml MeOH/H₂O/ konz. HCl (4 : 5 : 1) wurde 7 h lang rückflußerhitzt Nach Zugabe von Eiswasser und Alkalisierung mit konz. NH₄OH wurde mit CH₂Cl₂ (3 x 60 ml) extrahiert. Die vereinigten organischen Phasen wurden mit H₂O (3 x 100 ml) und ges. NaCl-Lösung gewaschen, über Na₂SO₄ getrocknet und eingedampft. Vom Eindampfrückstand (1,94 g braunes Schaumharz 14β-[(3-Chlorbenzyl)oxy]-4,5α-epoxy-3-hydroxy-17-methylmorphinan-6-on) wurde ein Teil (1,50 g) ohne weitere Reinigung für weitere Synthesen verwendet. Der Rest (443 mg) wurde mittels Säulenchromatographie (Kieselgel; CH₂Cl₂ / MeOH / konz. NH₄OH (250:4:0,5) gereinigt. Der Eindampfrückstand (353 mg gelbes Schaumharz) wurde in Ether gelöst und durch Zugabe von etherischer HCI wurde 14β-[(3-Chlorbenzyl)oxy]-4,5α-epoxy-3-hydroxy-17-methylmorphinan-6-on Hydrochlorid (335 mg) als Reinsubstanz gefällt. Fp. 205 - -210°C; IR (KBr): 1724 (CO) cm⁻¹; ¹H-NMR (DMSO-d₆): δ 9,55 (s, OH), 9,15 (s, breit, ⁺NH), 7,70 - 7,25 (m, 4 arom. H), 6,73 (d, 1 arom. H, J = 8 Hz), 6,67 (d, 1 arom. H, J = 8 Hz), 5,10 (s, C₅-H), 4,87 (d, 1 H, OCH₂), J = 12,4 Hz), 4,68 (d, 1 H, OCH₂), J = 12,4 Hz), 2,93 (d, ⁺NCH₃, J = 4,6 Hz); MS (Cl): m/z 427(M⁺+1).

Eine Gemisch von ungereinigtem 14β-[(3-Chlorbenzyl)oxy]-4,5α-epoxy-3-hydroxy-17-methylmorphinan-6-on (500 mg braunes Schaumharz), K₂CO₃ (485 mg, 3,51 mmol) und Propargylbromid (0,26 ml, 3,51 mmol) und 40 ml Aceton (mit 0.5% H₂O) wurde unter N₂ 3 h lang rückflußerhitzt. Nach dem Abkühlen wurde das anorganische Material abfiltriert, mit CH₂Cl₂ gewaschen und das Filtrat eingedampft. Der Eindampfrückstand (538 mg braunes Öl) wurde mittels Säulenchromatographie (Kieselgel; CH₂Cl₂ /MeOH / konz. NH₄OH (250:2:0.5)) gereinigt. Der Eindampfrückstand (337 mg gelbes Öl) wurde in Ether gelöst und durch Zugabe von etherischer HCl wurde **18·**HCl als Reinsubstanz gefällt. Ausbeute 347 mg (74%). Fp. 145 -150 °C; IR (KBr): 1717 (CO), 2118 (C≡C) cm⁻¹; ¹H-NMR (DMSO-d₆): δ 9,21 (s, breit. ⁺NH). 7,71 -7,32 (m, 4 arom. H), 6,96 (d, 1 arom. H, J = 8,4 Hz), 6,82 (d, 1 arom. H, J = 8,4 Hz), 5,20 (s, C₅-H), 4,89 (d, 1 H, C₁₄OCH₂, J = 12,1 Hz ),4,69 (d, 1 H, C₁₄OCH₂, J = 12,1 Hz), 4,84 (d, 2 H, C₃OCH₂, J = 2,2 Hz), 3,58 (t, 1 H, C≡CH, J = 2,2 Hz), 2,94 (d, ⁺NCH₃, J = 4,4 Hz); MS (CI): m/z 465 (M⁺ + 1).

### Beispiel 19

### Synthese des 4,5α-Epoxy-17-ethyl-3-methoxy-14β-[(3-phenylpropyl)oxy]morphinan-6-ons

### (Verbindung 19)

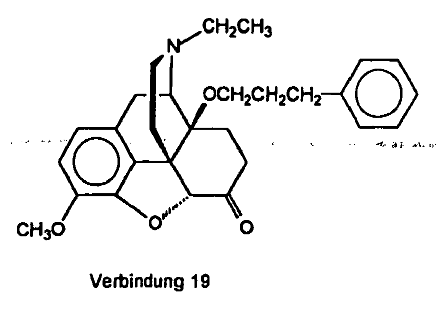

Ein Gemisch von 4,5α-Epoxy-3-methoxy-14β-[(3-phenylpropyl)oxy]morphinan-6-on Hydrochlorid (s. Beispiel 1) (4,0 g, 8,77 mmol), K₂CO₃ (8,0 g, 57,89 mmol), Ethyliodid (0,99 ml, 12,40 mmol) und 50 ml wasserfreiem N,N-Dimethylformamid wurde 5 h lang unter N₂ bei 80 °C (Badtemperatur) gerührt. Dann wurde vom anorganischen Rückstand abfiltriert, das Filtrat eingedampft und der Rückstand (4,7 g braunes Öl) mittels Säulenchromatographie (Kieselgel; CH₂Cl₂/MeOH/konz. NH₄OH: 8 I: (250/2/0,5)); 0,5 I: (250/3/0,5), (250/4/0,5), (250/6/0,5), (250/8/0,5), (250/10/0,5)) gereinigt. Der Eindampfrückstand (1,3 g, hellgelbes Öl) wurde aus MeOH kristallisiert. Ausbeute 1,2 g (30 %) Verbindung **19**. Fp. 95-101 °C; IR (KBr): 1719 (C=O) cm⁻¹; ¹H-NMR (DMSO-d₆): δ 7,30-7,13 (m, 5 arom. H), 6,73 (d, J = 8,2 Hz, 1 arom. H), 6,64 (d, J = 8,2 Hz, 1 arom. H), 4,76 (s, 1 H, C₅-H), 3,78 (s, 3 H, OCH₃), 0,96 (t, 3 H, N-CH₂-CH₃), J = 7 Hz); MS (Cl): m/z 448 (M⁺+1).

### Beispiel 20

### Synthese des 4,5α-Epoxy-17-ethyl-3-hydroxy-14β-[(3-phenylpropyl)oxy]morphinan-6-on Hydrochlorids (Verbindung 20.HCl)

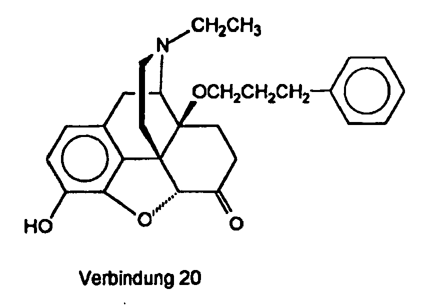

Eine Lösung von Verbindung **19** (1,0 g, 2,23 mmol) in 48%iger HBr (10 ml) wurde 13 Minuten lang rückflusserhitzt. Nach dem Abkühlen wurde mit Wasser (50 ml) verdünnt, Eis zugesetzt, mit konz. NH₄OH alkalisiert und mit CH₂Cl₂ (5 x 50 ml) extrahiert. Die vereinigten organischen Phasen wurden mit Wasser (3 x 50 ml) und NaCl-Lösung (1x 50 ml) gewaschen, über Na₂SO₄ getrocknet und eingedampft Der Eindampfrückstand (1,3 g, braunes Schaumharz) wurde mittels Säulenchromatographie (Kieselgel; CH₂Cl₂/MeOH/konz. NH₄OH (250:2:0,5)) gereinigt. Der Eindampfrückstand (448 mg gelbes Schaumharz) wurde in Ether gelöst, etherische HCl zugesetzt und Verbindung **20**.HCl isoliert. Ausbeute: 380 mg (36%). Fp. 178-180 °C; IR (KBr): 1724 (C=O) cm⁻¹; ¹H-NMR (DMSO-d₆): δ 9,14 (s, OH), 7,29-7,03 (m, 5 arom. H), 6,56 (d, 1 arom. H, J = 8 Hz), 6,50 (d, 1 arom. H, J = 8 Hz), 4,69 (s, C₅-H), 0,96 (t, 3 H, N-CH₂-CH₃), J = 7,2 Hz); MS (Cl): m/z 434 (M⁺+1).

### Beispiel 21

### Synthese des 4,5α-Epoxy-3-hydroxy-14β-[(3-methylbutyl)oxy)-17-propylmorphinan-6-on Hydrochlorids (Verbindung 21.HCl)

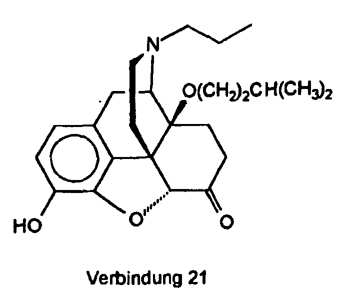

Eine Löung von Naloxon·HCl·2H₂O (20,0 g, 50,0 mmol) und Methansulfonsäure (3,9 ml, 60,0 mmol) in 150 ml Ethylenglykol wurde bei einer Badtemperatur von 80-90 °C 18 h lang unter N₂ gerührt. Nach Zugabe von 200 ml Eiswasser und Alkalisierung mit konz. NH₄OH erfolgte Extraktion mit CH₂Cl₂ (2 x 100 ml, 2 x 50 ml). Die vereinigten organischen Phasen wurden mit H₂O (2 x 100 ml) und ges. NaCl-Lösung gewaschen, über Na₂SO₄ getrocknet und eingedampft. Der Eindampfrückstand (beige Kristalle) wurde mit siedendem MeOH behandelt, wobei 16.52 g (89%) farblose Kristalle von 4,5α-Epoxy-3,14β-dihydroxy-17-(prop-2-enyl)morphinan-6-spiro-2'-(1,3-dioxolan) erhalten wurden. Fp. 244 - 247 °C;¹H-NMR (DMSO-d₆): δ 8.87 (s, C₃-OH), 6,54 (d, 1 arom. H, J = 8 Hz), 6,44 (d, 1 arom. H, J = 8 Hz), 5,93 - 5,70 (m, 1 olef. H), 5,27 - 5,04 (m, 2 olef. H), 4,79 (s, C₁₄-OH), 4,33 (s, C₅-H), 4,11 -3,61 (m, 4 H, C₆(OCH₂)₂); MS (Cl): m/z 372 (M⁺ + 1).

Ein Gemisch, bestehend aus 4,5α-Epoxy-3,14β-dihydroxy-17-(prop-2-enyl)morphinan-6-spiro-2'-(1,3-dioxolan) (16,0 g, 43,1 mmol), K₂CO₃ (16.2 g, 117 mmol), Benzylbromid (5,63 ml, 47,4 mmol) und 80 ml wasserfreiem N,N-Dimethylformamid, wurde unter N₂ 24 h lang bei Raumtemperatur gerührt. Das anorganische Material wurde abfiltriert, mit CH₂Cl₂ gespült und das Filtrat eingedampft Der Eindampfrückstand (weißes Kristallisat) wurde zur Entfernung des Lösungsmittels und des Benrylbromids 1 h lang mit rückflußkochendem Ether behandelt. Das abgekühlte Gemisch wurde abfiltriert, das verbleibende Kristallisat mit wenig kaltem Ether nachgewaschen und dann mit rückflußkochendem MeOH behandelt. Es wurden 12,53 g (63%) 3-Benryloxy-4,5α-epoxy-14β-hydroxy-17-(prop-2-enyl)morphinan-6-spiro-2'-(1,3-dioxolan) als farblose Kristalle erhalten. Fp. 142 - -144 °C; ¹H-NMR (DMSO-d₆): δ 7,50 - 7,22 (m, 5 arom. H), 6,77 (d, 1 arom. H, J = 8,0 Hz), 6,56 (d, 1 arom. H, J = 8,0 Hz), 5,95 - 5,68 (m, 1 H, 1 olef. H), 5,30 - 5,01 (m, 4 H, 2 olef. H, C₃OCH₂), 4,81 (s, OH), 4,40 (s, C₅-H), 4,08 - 3,65 (m, 4 H, C₆(OCH₂)₂); MS (Cl): m/z 463 (M⁺ + 1).

NaH (650 mg, 27 mmol) (erhalten aus 1,1 g 60% NaH-Dispersion in Öl durch Waschen mit Petrolether) wurde einer Lösung von 3-Benzyloxy-4,5a-epoxy-14β-hydroxy-17-(prop-2-enyl)morphinan-6-spiro-2'-(1,3-dioxolan) (5,00 g, 10,83 mmof) in 40 ml wasserfreiem N,N-Dimethylformamid unter N₂ bei 0° C (Badtemperatur) unter Rühren zugefügt. Nach 15 min wurde 3,3-Dimethylallylbromid (1,90 ml, 16,25 mmol) zugesetzt und das Gemisch zunächst für 1 h bei 0° C (Badtemperatur), dann bei nachlassender Kühlung für 4 Stunden weiter gerührt. Zum Beenden der Reaktion wurden Eisstückchen zugesetzt, bis keine Wasserstoffentwicklung mehr festzustellen war. Das Gemisch wurde auf 100 ml Wasser gegossen und mit Dichlormethan (3 x 100 ml) extrahiert. Die vereinigten organischen Phasen wurden mit H₂O (3 x 100 ml) und 250 ml gesättigter Natriumchloridlösung gewaschen, über Natriumsulfat getrocknet und eingedampft. Der Eindampfrückstand (6,02 g gelbes Öl) wurde mittels Säulenchromatographie (Kieselgel; CH₂Cl₂/MeOH/konz. NH₄OH (250 : 2 : 0.5)) gereinigt Der Eindampfrückstand (5,01 g hellgelbes Öl) wurde in Ether gelöst und durch Zugabe von etherischer HCl reines 3-Benzyloxy-4,5α-epoxy-14β-[(3-methylbut-2-enyl)oxy]-17-(prop-2-enyl)morphinan-6-spiro-2'-(1,3-dioxolan) Hydrochlorid gefällt. Ausbeute 4,83 g (79%); Fp. 122 - 124 °C; ¹H-NMR (DMSO-d₆): δ 8,61 (s, breit, ⁺NH), 7,50- 7,25 (m, 5 arom. H), 6,92 (d, J = 8,4 Hz, 1 arom. H), 6,70 (d, ³J = 8,4 Hz, 1 arom. H), 5,99-5,41 (m, 4 olefin. H), 5,14 (s, C₃-OCH₂), 4,57 (s, C₅-H), 1,75 (s, 3 H, CH=CCH₃), 1,66 (s, 3 H, CH=CCH₃); MS (Cl): m/z 531 (M⁺+1).

Ein Gemisch von 3-Benryloxy-4,5α-epoxy-14β-[(3-methylbut-2-enyl)oxy]-17-(prop-2-enyl)morphinan-6-spiro-2'-(1,3-dioxolan) Hydrochlorid (4,59 g, 8,11 mmol), 460 mg 10% Pd/C-Katalysator und 100 ml MeOH wurde bei Raumtemperatur und 30 psi 4 h lang hydriert. Anschließend wurde der Katalysator abfiltriert und das Filtrat im Vakuum eingedampft. Der Eindampfrückstand (schwachgraue Kristalle) wurde aus MeOH/Et₂O umkristallisiert. Ausbeute 3,54 g (91%) reines 4,5α-Epoxy-3-hydroxy-14β-[(3-methylbutyl)oxy]-17-propylmorphinan-6-spiro-2'-(1,3-dioxolan) Hydrochlorid. Fp. 176 - 185 °C; ¹H-NMR (DMSO-d₆): δ 9,22 (s, OH), 8,12 (s, breit, 1 H, ⁺NH), 6,68 (d, J = 8,0 Hz, 1 arom. H), 6,58 (d, J = 8,0 Hz, 1 arom. H), 4,46 (s, 1 H, C₅-H), 4,11-3,62 (m, 4 H, C₆(OCH₂)₂), 0,99 - 0,85 (m, 9 H, N⁺(CH₂)₂CH₃, CH(CH₃)₂); MS (Cl): m/z 445 (M⁺ + 1).

Eine Lösung von 4,5α-Epoxy-3-hydroxy-14β-[(3-methylbutyl)oxy]-17-propylmorphinan-6-spiro-2'-(1,3-dioxolan) Hydrochlorid (3,40 g, 7,1 mmol) in 40 ml MeOH/H₂O/konz. HCl (50 : 85 : 15) wurde 4 h lang rückflußerhitzt Nach Zugabe von H₂O (50 ml) und Alkalisierung mit konz. NH₄OH wurde mit CH₂Cl₂ (3 x 60 ml) extrahiert. Die vereinigten organischen Phasen wurden mit H₂O (2 x 100 ml) und ges. NaCl-Lösung gewaschen, über Na₂SO₄ getrocknet und eingedampft. Der Eindampfrückstand (2,61 g dunkelbraunes Öl) wurde mittels Säulenchromatographie (Kieselgel; CH₂Cl₂ / MeOH / konz. NH₄OH (250 : 3:0.5)) gereinigt. Vom Eindampfrückstand (1,77 g weißes Schaumharz, 62%) wurde ein Teil (1,39 g) in Ether gelöst und durch Zugabe von etherischer HCl **21**.HCl (1.42 g) als Reinsubstanz gefällt. Fp. 175 - 185 °C (Zers.); IR (KBr): 1725 (CO) cm⁻¹; ¹H-NMR (DMSO-d₆): δ 9.52 (s, OH), 8,33 (s, breit. ⁺NH), 6,70 (d, 1 arom. H, J = 8.2 Hz), 6,65 (d, 1 arom. H, J = 8,2 Hz), 4,91 (s, C₅-H), 1,00 - 0,85 (m, 9 H, N⁺(CH₂)₂CH₃, CH(CH₃)₂); MS (Cl): m/z 401 (M⁺ + 1).

### Beispiel 22

### Synthese des 5β-Benzyl-14-methoxycodeinon Hydrochlorids (= 5β-Benzyl-7,8-didehydro-4,5α-epoxy-3,14β-dimethoxy-17-methyl-morphinan-6-on Hydrochlorid) (Verbindung 22.HCl)

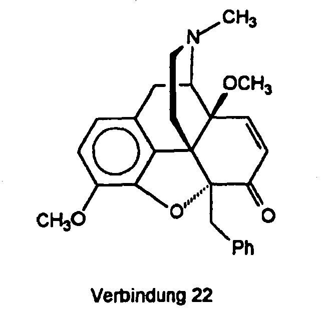

NaH (1,2 g, 50 mmol) (erhalten aus 2,0 g 60% NaH-Dispersion in Öl durch Waschen mit Petrolether) wurde einer Lösung von 5β-Benzyl-1 4-hydroxycodeinon (M. Gates et al. J. Org. Chem. 1989, 54, S. 972-974) (7,95 g, 19,7 mmol) in 100 ml wasserfreiem N,N-Dimethylformamid unter N₂ bei 0° C (Badtemperatur) unter Rühren zugefügt. Nach 15 min wurde Dimethylsulfat (2,43 ml, 25,6 mmol) zugesetzt und das Gemisch 1 h lang bei 0° C (Badtemperatur) gerührt. Zum Beenden der Reaktion wurden Eisstückchen zugesetzt, bis keine Wasserstoffentwicklung mehr festzustellen war. Das Gemisch wurde auf 250 ml Wasser gegossen und mit Dichlormethan (3 x 100 ml) extrahiert. Die vereinigten organischen Phasen wurden mit H₂O (1 x 100 ml, 3 x 60 ml) und gesättigter Natriumchloridlösung gewaschen, über Natriumsulfat getrocknet und eingedampft. Vom Eindampfrückstand (8,3 g gelbbraunes Öl) wurde ein Teil unverändert weiter verwendet, der Rest (500 mg) mittels Säulenchromatographie (Kieselgel; CH₂Cl₂ / MeOH / konz. NH₄OH (250:2:0,5) gereinigt. Der Eindampfrückstand (210 mg gelbes Öl) wurde in Ether gelöst und durch Zugabe von etherischer HCl reine Verbindung **22**.HCl gefällt. Ausbeute 148 mg. Fp. 164 - 166 °C; IR (KBr): 1680 (CO) cm⁻¹; ¹H-NMR (DMSO-d₆): δ 9,47 (s, breit, ⁺NH), 7,37-7,09 (m, 5 arom. H), 7,04 (d, 1 H, C₈-H, J = 10,0 Hz), 6,84 (d, 1 arom. H, J = 8,4 Hz), 6,74 (d, 1 arom. H, J = 8,4 Hz), 6,29 (d, 1 H, C₇-H, J = 10,0 Hz), 3,76 (s, 3 H, C₃OCH₃), 3,59 (d, 1 H, C₅-CH₂, J = 15,1 Hz), 3,29 (d, 1 H, C₅-CH₂), J = 15,1 Hz), 2,98 (s, 3 H, C₁₄-OCH₃), 2,88 (d, 3 H, ⁺NCH₃, J = 4,6 Hz); MS (CI): m/z 419 (M⁺ + 1).

### Beispiel 23

### Synthese des 5β-Benzyl-4,5a-epoxy-3,14β-dimethoxy-17-methylmorphinan-6-ons (Verbindung 23)

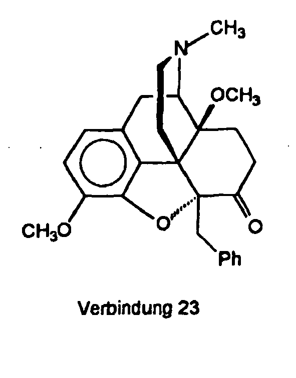

Ein Gemisch der ungereinigten Verbindung **22** (6,96 g, 16,7 mmol gelbbraunes Öl), 0,7 g 10%iger Pd/C-Katalysator und 150 ml MeOH wurde bei Raumtemperatur und 30 psi 3 h lang hydriert. Anschließend wurde der Katalysator abfiltriert und das Filtrat im Vakuum eingedampft. Der Eindampfrückstand (hellbraunes Schaumharz) wurde aus isopropanol kristallisiert, wobei 3,14 g (45%) reine Verbindung **23** erhalten wurden. Fp. 136 -138 °C; IR (KBr): 1725 (CO) cm⁻¹; ¹H-NMR (CDCl₃): δ 7,35 - 7,18 (m, 5 arom. H), 6,66 (d, 1 arom. H, J = 8,2 Hz), 6,57 (d, 1 arom. H, J = 8,2 Hz), 3,90 (s, C₃-OCH₃), 3,48 (s, 2 H, C₅-CH₂), 3,34 (s, C₁₄-OCH₃), 2,42 (s, NCH₃); MS (Cl): m/z 421 (M⁺ + 1).

### Beispiel 24

### Synthese des 5β-Benzyl-4,5α-epoxy-3-hydroxy-14β-methoxy-17-methylmorphinan-6-on Hydrochlorids (Verbindung 24.HCl)

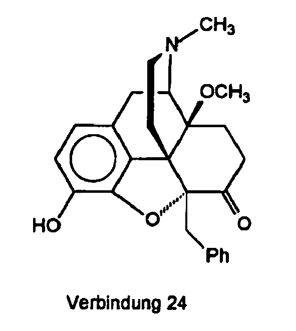

Eine Lösung von Verbindung **23** (2,90 g, 6,9 mmol) in 40 ml 48%iger Bromwasserstoffsäure wurde 15 min lang rückflußerhitzt. Anschließend wurde die Lösung gekühlt und eingedampft. Der Eindampfrückstand wurde in MeOH gelöst und wieder eingedampft. Das resultierende Schaumharz (2,50 g) wurde in die Base überführt und mittels Säulenchromatographie (Kieselgel; CH₂Cl₂ / MeOH / konz. NH₄OH (250:3:0.5) gereinigt. Der Eindampfrückstand (2,27 g gelbes Öl) wurde in Ether gelöst, und mit etherischer HCl wurde die reineVerbindung **24.**HCl gefällt. Ausbeute 2,30 g (75%); Fp. 222 - 224 °C; IR (KBr): 1725 (CO) cm⁻¹; ¹H-NMR (DMSO-d₆): δ 9,45 (s, OH), 9,38 (s, breit, ⁺NH), 7,37 - 7,09 (m, 5 arom. H), 6,70 (d, 1 arom. H, J = 8,2 Hz), 6,62 (d, 1 arom. H, J = 8,2 Hz), 3,41 (s, OCH₃), 2,89 (d, 3 H, ⁺NCH₃, J = 3,4 Hz); MS (Cl): m/z 407 (M⁺ + 1).

### Beispiel 25

### Synthese des 4-Hydroxy-3-methoxy-17-methyl-14-[(3-phenylpropyl)oxy]-morphinan-6-ons (Verbindung 25)

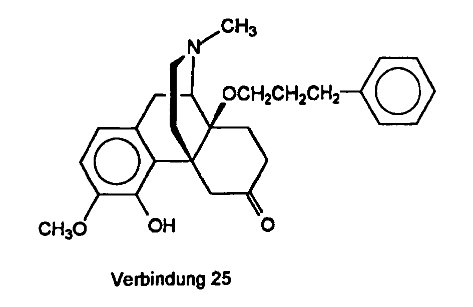

Ein Gemisch von 4,5α-Epoxy-3-methoxy-17-methyl-14-[(3-phenylpropyl)oxy]-morphinan-6-on (= 14-O-(3-Phenylpropyl)oxycodon) (s. Beispiel 1) (600 mg, 1,4 mmol), Ammoniumchlorid (0,97 g, 18,0 mmol) und 30 ml MeOH wurde unter Rühren rückflußerhitzt. Dann wurde innerhalb von 5 Minuten aktiviertes Zinkpulver (0,94 g, 14,0 mmol) in Portionen zugegeben und das Gemisch 6 h rückflußerhitzt. Dann wurde vom anorganischen Rückstand abfiltriert, das Filtrat eingedampft, mit konz. NH₄OH alkalisiert, mit CH₂Cl₂ (3 x 40 ml) extrahiert, mit H₂O gewaschen, über Na₂SO₄ getrocknet und eingedampft. Der Eindampfrückstand (605 mg) wurde aus wenig MeOH kristallisiert. Ausbeute 562 mg (86 %) Verbindung 25. Fp. > 258 °C Zers.); IR (KBr): 3145 (OH), 1734 (CO) cm⁻¹; ¹H-NMR (CDCl₃): δ 7.37 - 7,05 (m, 5 arom.H). 6.64 (d, 1 arom. H, J = 8,3 Hz), 6,54 (d, 1 arom. H, J = 8.3 Hz), 3,79 (s, OCH₃), 2,30 (s, NCH₃); MS(Cl): m/z 436 (M⁺+1).

### Beispiel 26

### Synthese des 3,4-Dimethoxy-17-methyl-14-[(3-phenylpropyl)oxy]morphinan-6-on Salicylats (Verbindung 26.C₇H₆O₃)

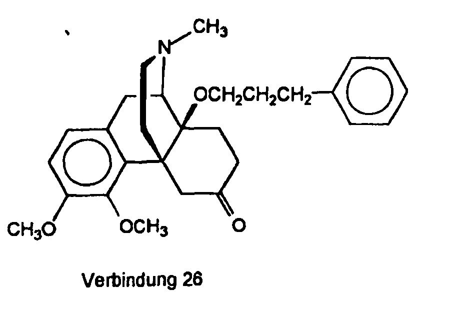

Ein Gemisch von Verbindung **25** (200 mg, 0,5 mmol), K₂CO₃ (420 mg, 3,0 mmol) und Phenyltrimethylammoniumchlorid (254 mg, 1,5 mmol) und 50 ml wasserfreiem N,N-Dimethylformamid wurde 9 h bei 80 °C (Badtemperatur)unter N₂ gerührt. Dann wurde vom anorganischen Rückstand abfiltriert, das Filtrat eingedampft und der Eindampfrückstand mittels Säulenchromatographie (Kieselgel; CH₂Cl₂/MeOH/konz. NH₄OH (93 : 7 : 1) gereinigt. Der Eindampfrückstand (250 mg Schaumharz) wurde in MeOH gelöst und mit Salicylsäure versetzt. Es wurden 102 mg (41 %) der Verbindung **26**. C₇H₆O₃ isoliert. Fp. 110 -115 °C; IR(KBr): 1720 (CO) cm⁻¹; ¹H-NMR (CDCl₃): δ 7,29 - 7,18 (m, 5 arom. H), 6,75 (s, 2 arom. H), 3,93 (s, C₄-OCH₃), 3,80 (s, C₃-OCH₃), 2,31 (s, NCH₃); MS(Cl): m/z 450 (M⁺+1).

### Beispiel 27

### Synthese des 14β-Benzyloxy-4-hydroxy-3-methoxy-17-methylmorphinan-6-on Hydrochlorids (Verbindung 27.HCl)

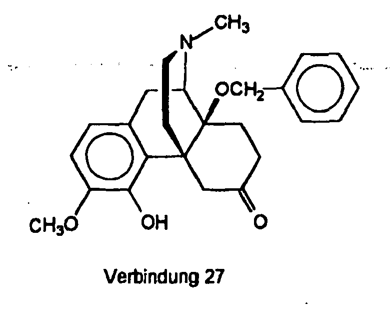

Ein Gemisch von 4,5α-Epoxy-14β-hydroxy-3-methoxy-17-methylmorphinan-6-spiro-2'-(1,3-dioxolan) (Lester et al. 1965, 21, S. 771) (6,0 g, 16,69 mmol), NaH (2,16 g, 90,0 mmol, gewonnen aus 3,6 g 60%iger Dispersion durch Waschen mit Petrolether) und 150 ml wasserfreiem N,N-Dimethylformamid wurde bei 0 °C (Badtemperatur) unter N₂ 20 min lang gerührt. Nach der Zugabe von Benzylbromid (4,0 ml, 33,39 mmol) wurde weitere 16 h lang bei Raumtemperatur gerührt. Dann wurden Eisstückchen zugegeben, bis die H₂-Entwicklurig beendet war, mit Wasser (100 ml) verdünnt und mit CH₂Cl₂ (1 x 250 ml, 3 x 150 ml, 1 x 100 ml) extrahiert. Die vereinigten organischen Phasen wurden mit Wasser (4 x 250 ml) und NaCl-Lösung (1 x 250 ml) gewaschen, über Na₂SO₄ getrocknet und eingedampft. Der Eindampfrückstand (7,47 g rotbraunes Öl) wurde aus MeOH (12 ml) kristallisiert. Isoliert wurden 6,01 g farbloses 4,5α-Epoxy-14β-benzyloxy-3-methoxy-17-methylmorphinan-6-spiro-2'-(1,3-dioxolan). Die Mutterlauge wurde eingedampft und mittels Säulenchromatographie (Kieselgel; CH₂Cl₂/MeOH/konz. NH₄OH: 2 (250/2/0.5); je **1** I: (250/4/0,5), (250/6/0,5), (250/8/0,5), (250/10/0.5)) gereinigt. Der Eindampfrückstand (551 mg, hellgelbes Öl) wurde aus MeOH (1 ml) kristallisiert. Gesamtausbeute 6,32 g (84%) 4,5α-Epoxy-14β-benzyloxy-3-methoxy-17-methylmorphinan-6-spiro-2'-(1,3-dioxolan). Fp. 121-124 °C; ¹H-NMR (DMSO-d₆): δ 7,44-7,26 (m, 5 arom. H), 6,74 (d, 1 arom. H, J=8,2 Hz), 6,60 (d, 1 arom. H J = 8,2 Hz), 4,55 (d, 1 aliph. H, 14-O-CH₂-C₆H₅, J = 11,2 Hz), 4,41 (s, C₅-H), 4,31 (d, 1 aliph. H, 14-O-CH₂-C₆H₅, J = 11,2 Hz), 4,02-3,67 (m, 4 H, OCH₂CH₂O), 3,77 (s, OCH₃); 2,31 (s, NCH₃); MS (Cl): m/z 450 (M⁺+1).

Eine Lösung von 4,5α-Epoxy-14β-benzyloxy-3-methoxy-17-methylmorphinan-6-spiro-2'-(1,3-dioxolan) (957 mg, 2,13 mmol) in MeOH (15 ml) und 2N HCl (22,5 ml) wurde 4 h lang rückflusserhitzt. Nach dem Abkühlen wurde mit Wasser (20 ml) verdünnt, mit konz. NH₄OH alkalisiert und mit CH₂Cl₂ (1x 100 ml, 1x 50 ml, 3x 25 ml) extrahiert. Die vereinigten organischen Phasen wurden mit Wasser (1x 100 ml) und NaCl-Lösung (1x 100 ml) gewaschen, über Na₂SO₄ getrocknet und eingedampft. Der Eindampfrückstand (879 mg dunkelgelbes Öl) wurde aus MeOH kristallisiert. Ausbeute 801 mg (89 %) 14β-Benzyloxy-4,5a-epoxy-3-methoxy-17-methylmorphinan-6-on. Fp. 136-138 °C; IR (KBr): 1721 (CO) cm⁻¹; ¹H-NMR (CDCl₃): δ 7,45-7,24 (m, 5 arom. H), 6,68 (d, 1 arom. H, J = 8,3 Hz), 6,62 (d, 1 arom. H, J = 8,3 Hz), 4,77 (d, 1 H, OCH₂Ph, J = 10,3 Hz), 4,64 (s, CH-5), 4,37 (d, 1 H, OCH₂Ph, J = 10,3 Hz), 3,88 (s, CH₃O), 2,39 (s, CH₃N); MS (CI): m/z 406 (M+⁺1).

Zu einem rückflußkochendem Gemisch von 14β-Benryloxy-4,5α-epoxy-3-methoxy-17-methylmorphinan-6-on (870 mg, 2,15 mmol), NH₄Cl (1,4 g, 26,21 mmol) und MeOH (40 ml) wurde unter Rühren vorsichtig portionsweise Zinkstaub (insgesamt 1,4 g, 21,45 mmol) zugegeben. Das Gemisch wurde 2 h lang rückftusserhitzt. Dann wurde vom anorganischen Material abfiltriert, das Filtrat eingedampft, der Eindamptrückstand mit Wasser (40 ml) versetzt, mit einem Gemisch aus CH₂Cl₂ und isopropanol (3:1) (3x je 40 ml) extrahiert, die vereinigten organischen Phasen mit Wasser (1x 20 ml) und NaCl-Lösung (1x 20 ml) gewaschen, über Na₂SO₄ getrocknet und eingedampft. Der Eindampfrückstand (961 mg, weißes Schaumharz) wurdemittels Säulenchromatographie (Kieselgel; CH₂Cl₂/MeOH/konz. NH₄OH (250:4:0,5)) gereinigt. Der Eindampfrückstand (750 mg) wurde in Ether gelöst und mit etherischer HCl versetzt. Ausbeute 730 mg (77 %) Verbindung **27.**HCl. Fp. > 220-224 °C (Zers.); IR (KBr): 1714 (C=O) cm⁻¹; ¹H-NMR (DMSO-d₆): δ 9,26 (s, breit, ⁺NH), 8,84 (s, C4-OH), 7,55-7,31 (m, 5 arom. H), 6,91 (d, 1 arom. H, J = 8,2 Hz), 6,68 (d, 1 arom. H, J = 8,2 Hz), 4,87 (d, 1 aliph. H, OCH₂C₆H₅), J = 11,6 Hz), 4,66 (d, 1 aliph. H, OCH₂C₆H₅), J = 11,6 Hz), 3,76 (s, C₃-OCH₃), 2,88 (d, 3 H, ⁺N-CH₃). MS (CI): m/z 408 (M⁺+1).

### Beispiel 28

### Synthese des 14β-Benzloxy-3,4-dimethoxy-17-methylmorphinan-6-on Hydrochlorids (Verbindung 28.HCl)

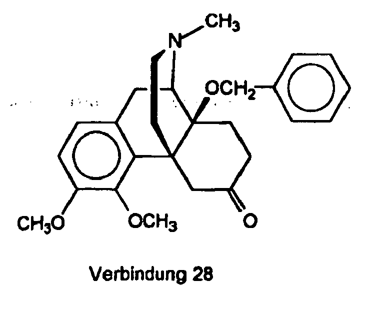

Ein Gemisch aus **27**.HCl (343 mg, 0,84 mmol), K₂CO₃ (353 mg, 2,52 mmol) und Phenyltrimethylammoniumchlorid (285 mg, 1,68 mmol) und N,N-Dimethylformamid (25 ml) wurde bei 80 °C (Badtemperatur) unter N₂ 4 h lang gerührt. Dann wurde vom anorganischen Material abfiltriert, das Filtrat eingedampft, der Eindampfrückstand (rotbraunes Öl) in CH₂Cl₂ (50 ml) gelöst, mit Wasser (2 x 20 ml) und NaCl-Lösung (1 x 20 ml) gewaschen, über Na₂SO₄ getrocknet und eingedampft. Der Eindampfrückstand (470 mg braunes Öl) wurde mittels Säulenchromatographie (Kieselgel; CH₂Cl₂/MeOH/konz. NH₄OH: je 1 (250/2/0,5), (25013/0,5), (250/410,5), (250/6/0,5), (250/8/0,5)) gereinigt Der Eindampfrückstand (280 mg, gelbes Öl) wurde in Ether gelöst und mittels etherischer HCl als Verbindung **28**.HCl gefällt. Ausbeute 251 mg (66 %); Fp. 140-145 °C; IR (KBr): 1710 (C=O) cm⁻¹; ¹H-NMR (DMSO-d₆): δ 9,27 (s, breit, ⁺NH), 7,53-7,30 (m, 5 arom. H), 7,02 (d, 1 arom. H, J = 8,4 Hz), 6,91 (d, 1 arom. H, J = 8,4 Hz), 4,84 (d, 1 aliph. H, OCH₂C₆H₅), J = 11 Hz), 4,64 (d, 1 aliph. H, OCH₂C₆H₅), J = 11 Hz), 3,78 und 3,82 (2 s, 2 OCH₃), 2,87 (s, ⁺N-CH₃); MS (Cl): m/z 422 (M⁺+1).

### Beispiel 29

### Synthese des 4-Hydroxy-3-methoxy-17-methyl-14β-[(2-naphthylmethyl)oxy]morphinan-6-on Hydrochlorids (Verbindung 29.HCl)

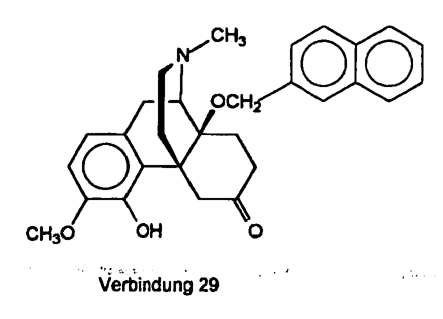

Ein Gemisch von 4,5α-Epoxy-14β-hydroxy-3-methoxy-17-methylmorphinan-6-spiro-2'-(1,3-dioxolan) (Lester et al. 1965, 21, S. 771) (1,96 g, 5,45 mmol), NaH (682 mg, 28,43 mmol, gewonnen aus 1,14 g 60%iger Dispersion durch Waschen mit Petrolether) und 40 ml wasserfreiem N,N-Dimethylformamid wurde bei 0 °C (Badtemperatur) unter N₂ 45 min lang gerührt. Nach Zugabe von 2-Brommethylnaphthalin (98%ig, 2,31 g, 10,9 mmol) wurde weitere 2 h lang bei 0 °C (Badtemperatur) gerührt. Dann wurden Eisstückchen zugegeben, bis die H₂-Entwicklung beendet war, mit Wasser (50 ml) verdünnt und mit CH₂Cl₂ (3 x 50 ml, 2 x 25 ml) extrahiert. Die vereinigten organischen Phasen wurden mit Wasser (4 x 50 ml) und NaCl-Lösung (1 x 250 ml) gewaschen, über Na₂SO₄ getrocknet und eingedampft. Der kristalline Eindampfrückstand (1,26 g) wurde zur Reinigung zweimal mit je 10 ml rückflußkochendem MeOH behandelt. Ausbeute 960 mg (35%) 4,5α-Epoxy-3-methoxy-17-methyl-14β-[(2-naphthylmethyl)oxy]morphinan-6-spiro-2'-(1,3-dioxolan). Fp. 184-185 °C; ¹H-NMR (DMSO-d₆): δ 7,93-7,89 (m, 4 arom. H), 7,62-7,48 (m, 3 arom. H), 6,75 (d, 1 arom. H, J = 8,2 Hz), 6,62 (d, 1 arom. H, J = 8,2 Hz), 4,72 (d, 1 aliph. H, OCH₂Naphtyl, J = 11,4 Hz), 4,47 (d, 1 aliph. H, OCH₂Naphtyl, J = 11,4 Hz), 4,43 (s, C₅-H), 4,06-3,68 (m, 4 H, OCH2CH2O), 3,78 (s, OCH₃), 2,34 (s, N-CH₃); MS (Cl): m/z 500 (M⁺+1).

Eine Lösung von 4,5α-Epoxy-3-methoxy-17-methyl-14β-[(2-naphthylmethyl)oxy]-morphinan-6-spiro-2'-(1,3-dioxolan) (2,3 g, 4,60 mmol) in MeOH (30 ml) und 2N HCl (45 ml) wurde 4 h lang rückflusserhitzt. Nach dem Abkühlen wurde mit Wasser (40 ml) verdünnt, mit konz. NH₄OH alkalisiert und mit CH₂Cl₂ (1 x 200 ml, 1 x 100 ml, 3 x 50 ml) extrahiert. Die vereinigten organischen Phasen wurden mit Wasser (1 x 200 ml) und NaCl-Lösung (1 x 200 ml) gewaschen, über Na₂SO₄ getrocknet und eingedampft. Der Eindampfrückstand (1,84 g dunkelgelbes Öl) wurde aus MeOH kristallisiert. Ausbeute 1,63 g (78%) 4,5α-Epoxy-3-methoxy-17-methyl-14β-[(2-naphthylmethyl)oxy]morphinan-6-on. Fp. 195-199 °C; IR (KBr): 1724 (vC=O), cm⁻¹; ¹H-NMR (DMSO-d₆): δ 8,00-7,90 (m, 4 arom. H), 7,68-7,48 (m, 3 arom. H), 6,77 (d, 1 arom. H, J = 8,2 Hz), 6,70 (d, 1 arom. H, J = 8,2 Hz), 4,88 (s, C₅-H), 4,84 (d, 1 aliph. H, 14OCH₂-Naphthyl, J = 11,2 Hz), 4,64 (d, 1 aliph. H, 14OCH₂-Naphthyl, J = 11,2 Hz), 3,80 (s, 3OCH₃), 2,37 (s, N-CH₃): MS (Cl) m/z 456 (M⁺+1).

Zu einem rückflußkochendem und gerührtem Gemisch von 4,5α-Epoxy-3-methoxy-17-methyl-14β-[(2-naphthylmethyl)oxy]-morphinan-6-on (1,0 g, 2,20 mmol), NH₄Cl (1,75 g, 32,71 mmol) und MeOH (42 ml) wurde portionsweise Zinkstaub (insgesamt 1,75 g, 26,76 mmol) zugegeben. Das Gemisch wurde 3 h lang rückflusserhitzt Dann wurde vom anorganischen Rückstand abfiltriert, mit MeOH gewaschen, das Filtrat eingedampft, der Eindampfrückstand in Wasser (40 ml) versetzt, mit konz. NH₄OH alkalisiert, mit CH₂Cl₂ (1 x 70 ml, 2 x 50 ml) extrahiert. Die vereinigten organischen Phasen wurden mit Wasser (1 x 40 ml, 1 x 30 ml) und NaCl-Lösung (1 x 30 ml) gewaschen, über Na₂SO₄ getrocknet und eingedampft. Der Eindampfrückstand (1,09 g, hellbraunes Schaumharz) wurde mittels Säulenchromatographie (Kieselgel; CH₂Cl₂/MeOH/konz. NH₄OH: 1,5 I: (250/2/0,5); je 0,5 I: (250/3/0,5), (250/4/0,5), (250/6/0,5)) gereinigt. Der Eindampfrückstand (836 mg beiges Schaumharz) wurde in Ether gelöst und mit etherischer HCl Verbindung **29** als Hydrochlorid (**29**.HCl) ausgefällt. Ausbeute 729 mg (68%); Fp. 195-197 °C; IR (KBr): 1710 (C=O)cm⁻¹; ¹H-NMR (DMSO-d₆): δ 9,24 (s, breit, ⁺NH), 8,86 (s, 4-OH), 8.02-7,91 (m, 4 arom. H, Naphthyl), 7,70-7,48 (m, 3 arom. H, Naphthyl), 6,91 (d, 1 arom. H, J = 8,2 Hz), 6,69 (d, 1 arom. H, J = 8,2 Hz), 5,01 (d, 1 aliph. H, 14OCH₂-Naphthyl, J = 12 Hz), 4,82 (d, 1 aliph. H, 14OCH₂-Naphthyl, J = 12 Hz), 3,77 (s, 3OCH₃), 2,90 (ps-d, ⁺NCH₃); MS (CI) m/z 458 (M⁺+1).

### Beispiel 30

### Synthese des 3,4-Dimethoxy-17-methyl-14β-[(2-naphtylmethyl)oxy]morphinan-6-on Hydrochlorids (Verbindung 30.HCl)

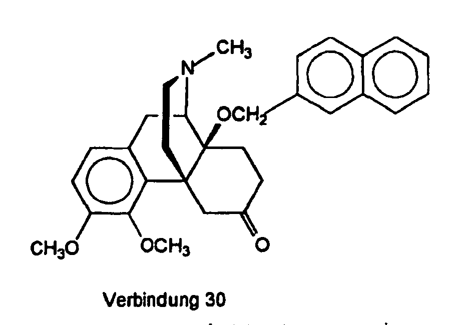

Ein Gemisch aus **29.**HCl (316 mg, 0,64 mmol), K₂CO₃ (304 mg, 2,17 mmol), Phenyltrimethylammoniumchlorid (260 mg, 1,53 mmol) und N,N-Dimethylformamid (25 ml) wurde bei 80°C (Badtemperatur) unter N₂ 2 h lang gerührt. Dann wurde vom anorganischen Material abfiltriert, das Filtrat eingedampft, der Eindampfrückstand (rotbraunes Öl) in CH₂Cl₂ (50 ml) aufgenommen, mit Wasser (2 x 20 ml) und NaCl-Lösung (1 x 20 ml) gewaschen, über Na₂SO₄ getrocknet und eingedampft. Der Eindampfrückstand (237 mg braunes Öl) wurde mittels Säulenchromatographie (Kieselgel; CH₂Cl₂/MeOH/konz. NH₄OH: 1,5 I: (250/2/0,5); je 0,51: (250/3:0,5), (250/4/0,5), (250/6/0,5), (250/8/0,5)) gereinigt. Der Eindampfrückstand (265 mg beiges Schaumharz) wurde in Ether gelöst und mit etherischer HCl Verbindung 30 als Hydrochlorid (**30.**HCl) ausgefällt. Ausbeute 257 mg (80%); Fp. 165-170 °C; IR (KBr): 1712 (C=O) cm⁻¹; ¹H-NMR (DMSO-d₆): δ 9,27 (s, breit, ⁺NH), 8,03-7,91 (m, 4 arom. H), 7,70-7,51 (m, 3 arom. H), 7,05 (d, 1 arom. H, J = 8,6 Hz), 6,97 (d, 1 arom. H, J = 8,6 Hz), 5,01 (d, 1 aliph. H, 14OCH₂-Naphthyl, J = 11,6 Hz), 4,83 (d, 1 aliph. H, 14OCH₂-Naphthyl, J = 11,6 Hz), 3,84 und 3,79 (2 s, 2 OCH₃), 2,90 (d, ⁺NCH₃); MS (Cl): m/z 472 (M⁺+1).

### Beispiel 31

### Synthese des 4-Hydroxy-3-methoxy-5β,17-dimethyl-14β-[(3-phenylpropyl)oxy]morphinan-6-on Hydrochlorids (Verbindung 31.HCl)

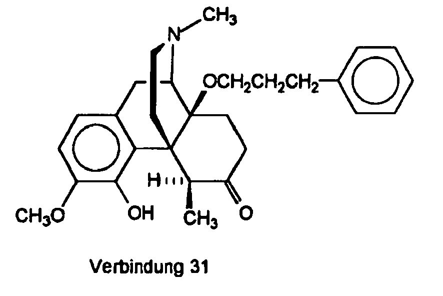

Zu einem rückflußkochendem und gerührten Gemisch von 4,5α-Epoxy-3-methoxy-5β,17-dimethyl-14β-[(3-phenylpropyl)oxy]morphinan-6-on (s. Beispiel 17) (3,7 g, 8,27 mmol), NH₄Cl (4,5 g, 83,87 mmol) und EtOH (50 ml) wurde portionsweise Zinkstaub (insgesamt 5,5 g, 84,12 mmol) zugegeben. Das Gemisch wurde 47 h lang rückflusserhitzt. Dann wurde vom anorganischen Rückstand abfiltriert, mit EtOH gewaschen, das Filtrat eingedampft, der Eindampfrückstand (6,35 g weißes Schaumharz) mit Wasser (200 ml) versetzt, mit konz. NH₄OH alkalisiert, mit CH₂Cl₂ (1 x 100 ml, 4 x 50 ml) extrahiert. Die vereinigten organischen Phasen wurden mit Wasser (4 x 200 ml) und NaCl-Lösung (1 x 200 ml) gewaschen, über Na₂SO₄ getrocknet und eingedampft. Der Eindampfrückstand (3,93 g gelbes Öl) wurde mittels Säulenchromatographie (Kieselgel; CH₂Cl₂/MeOH/konz. NH₄OH (250/2/0,5)) gereinigt. Es wurden 2,99 (80%) der reinen Verbindung **31** als gelbes Öl erhalten. 500 mg davon wurden in Ether gelöst und mit etherischer HCl Verbindung 31 als Hydrochlorid (**31**.HCl) ausgefällt. Fp. > 210 °C (Zers.); IR (KBr): 1700 (C=O) cm⁻¹; ¹H-NMR (DMSO-d₆): δ 9,64 (s, C4-OH), 8,88 (s, breit, ⁺NH), 7,33-7,17 (m, 5 arom. H), 6,91 (d, 1 arom. H, J = 8,2 Hz), 6,70 (d, 1 arom. H, J = 8,2 Hz), 3,78 (s, 30CH₃), 2,80 (d, ⁺NCH₃, J = 3,4 Hz); 1,28 (d, C₅-CH₃, J = 6,6 Hz); MS (Cl): m/z 450 (M⁺+1).

### Beispiel 32

### Synthese des 3,4-Dimethoxy-5β,17-dimethyl-14β-[(3-phenylpropyl)oxy]morphinan-6-on Hydrochlorids (Verbindung 32.HCl)

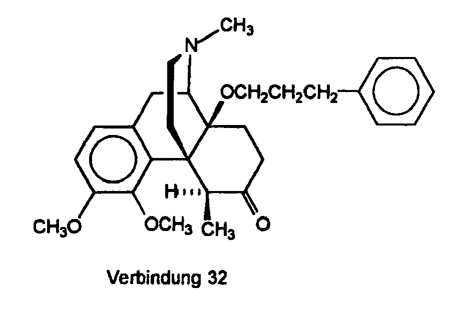

Verbindung 31 (Base) 1,55 g, 3,44 mmol) wurde in 20 g 40%iger Tetrabutylammoniumhydroxid-Lösung bei Raumtemperatur unter N₂ gelöst. Dann wurde eine Lösung von Dimethylsulfat (0,48 g, 3,8 mmol) in 15 ml Dichlormethan zugefügt. Das Gemisch wurde 1.2,5 h bei Raumtemperatur unter.N₂ heftig gerührt und dann 300 ml Wasser und 50 ml Dichlormethan zugesetzt. Die wässrige Phase wurde mit Dichlormethan (2 x 50 ml) extrahiert. Die vereinigten organischen Phasen wurden mit konz. Ammoniak (1 x 50 ml), Wasser (6 x 200 ml) und ges. Natriumchloridlösung (1 x 200 ml) gewaschen, über Natriumsulfat getrocknet und eingedampft. Der Eindampfrückstand (1,25 g gelbes Öl) wurde mittels Säulenchromatographie (Kieselgel; CH₂Cl₂/MeOH/konz. NH₄OH (25072/0.5)) gereinigt Es wurden 1,0 g (53%) der reinen Verbindung 32 als helles Öl erhalten. 810 mg davon wurden in Ether gelöst und mit etherischer HCI Verbindung **32** als Hydrochlorid (**32**.HCl) ausgefällt Fp. 135-138 °C; IR (KBr): 1700 (C=O) cm⁻¹; ¹H-NMR (DMSO-d₆): δ 9,36 (s. breit, ⁺NH), 7,31-7,21 (m, 5 arom. H), 7,05 (d, 1 arom. H, J = 8,4 Hz), 6,95 (d, 1 arom. H, J = 8,4 Hz), 3,80 und 3,79 (2 s, 2 OCH₃), 2,81 (d, ⁺NCH₃, J = 4,8 Hz); 1,25 (d, C₅-CH₃, J = 7 Hz); MS (CI): m/z 464 (M⁺+1).

### Beispiel 33

### Synthese des 14β-Ethoxy-4-hydroxy-3-methoxy-5β,17-dimethylmorphinan-6-ons (Verbindung 33)

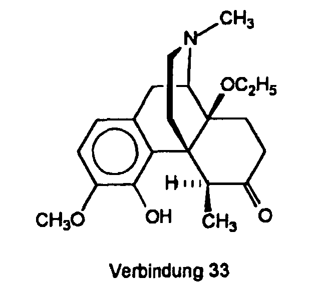

Zu einem rückflußkochendem und gerührtem Gemisch von 4,5α-Epoxy-14β-ethoxy-3-methoxy-5β,17-dimethylmorphinan-6-on (Schmidhammer et al. Helv. Chim. Acta 1990, 73, S. 1784-1787) (3,0 g, 8,4 mmol), NH₄Cl (15,0 g, 280,1 mmol) und MeOH (30 ml) wurde portionsweise Zinkstaub (insgesamt 15,0 g, 229,4 mmol) zugegeben. Das Gemisch wurde 24 h lang rückflusserhitzt. Dann wurde vom anorganischen Rückstand abfiltriert, mit MeOH gewaschen, das Filtrat eingedampft, der Eindampfrückstand mit verd. NH₄OH alkalisiert, mit einem Gemisch aus CH₂Cl₂ und MeOH (2:1) (1 x 100 ml, 3 x 30 ml) extrahiert. Die vereinigten organischen Phasen wurden über Na₂SO₄ getrocknet und eingedampft. Der Eindampfrückstand (2,82 g braunes Schaumharz) wurde aus MeOH kristallisiert und so 684 mg der reinen Verbindung **33** erhalten. Die Mutterlauge wurde eingedampft und der Eindampfrückstand (1,895 g braunes Schaumharz) für die Herstellung von Verbindung **34** ohne weitere Reinigung verwendet Verbindung **33**: Fp. 122-129 °C; IR (KBr): 3480 (OH), 1715 (C=O) cm⁻¹; ¹H-NMR (CDCl₃): δ 6,67 (d, 1 arom.

H, J = 8,0 Hz), 6,57 (d, 1 arom. H, J = 8,0 Hz), 3,82 (s, OCH₃), 2,32 (s, NCH₃), 1,54 (s, C₅-CH₃), 1,18 (t, CH₃CH₂, J = 5,7 Hz); MS (CI): m/z 360 (M⁺+1).

### Beispiel 34

### Synthese des 14β-Ethoxy-3,4-dimethoxy-5β,17-dimethylmorphinan-6-on Salicylats (Verbindung 34.C₇H₆O₃)

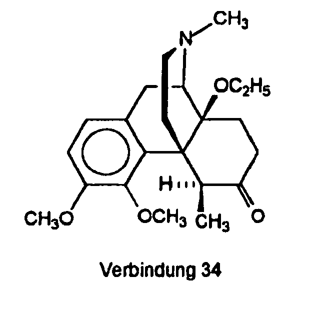

Ein Gemisch aus Verbindung **33** (1,895 g, 5,3 mmol) (Eindampfrückstand der Mutterlauge, s. oben), K₂CO₃ (2,12 g, 15,3 mmol), Phenyltrimethylammoniumchlorid (1,8 g, 10,5 mmol) und wasserfreies N,N-Dimethylformamid (20 ml) wurde bei 80 °C (Badtemperatur) unter N₂ 3 h lang gerührt. Dann wurde vom anorganischen Material abfiltriert, mit Dichlormethan gewaschen und das Filtrat eingedampft. Der Eindampfrückstand wurde in Dichlormethan gelöst, mit Wasser (2 x 50 ml) ges. NaCl-Lösung (1 x 50 ml) gewaschen, über Natriumsulfat getrocknet und eingedampft. Der Eindampfrückstand (2,11 g dunkelbraunes Öl) wurde mittels Säulenchromatographie (basisches Aluminiumoxid, Aktivitätsstufe III; Dichlormethan) gereinigt. Der Eindampfrückstand wurde mit Salicylsäure in Diisopropylether als Verbindung **34.** C₇H₆O₃ kristallisiert. Ausbeute 1,32 g (49%). Fp. 135-141 °C; IR (KBr): 3400 (⁺NH, OH), 1710 (C=O), 1630 (CO₂⁻) cm⁻¹; ¹H-NMR (CDCl₃): δ 7,88 (m, ⁺NH), 7,27 (m, 2 arom. H), 6,81 (m, 4 arom. H), 3,86 (s, OCH₃), 3,83 (s, OCH₃), 2,84 (s, ⁺NCH₃), 1,40 (d, C₅-CH₃, 5,8 Hz), 1,17 (t, CH₃CH₂, J = 5,3 Hz); MS (Cl): m/z 374 (M⁺+1).

### Beispiel 35

### Synthese des 14β-Benzyloxy-3,4-dimethoxy-5β,17-dimethylmorphinan-6-ons (Verbindung 37)

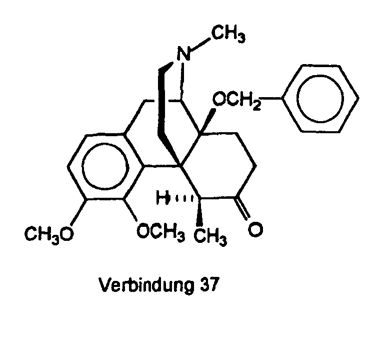

Zu einem rückflußkochendem und gerührtem Gemisch von 4,5α-Epoxy-3-methoxy-5β,17-dimethyl-14β-hydroxymorphinan-6-on (Schmidhammer et al. Helv. Chim. Acta 1988,71, S. 1801-1804) (4,17 g, 12,66 mmol), NH₄Cl (7,0 g, 130,87 mmol) und EtOH (50 ml) wurde portionsweise Zinkstaub (insgesamt 10,5 g, 160,6 mmol) zugegeben. Das Gemisch wurde 68 h lang rückflusserhitzt. Dann wurde vom anorganischen Rückstand abfiltriert, mit EtOH gewaschen, das Filtrat eingedampft, der Eindampfrückstand (7,65 g weißes Schaumharz) mit Wasser (150 ml) versetzt, mit konz. NH₄OH alkalisiert, mit CH₂Cl₂/Isopropanol (3:1) (2 x 80 ml, 1 x 60 ml, 1 x 40 ml) extrahiert. Die vereinigten organischen Phasen wurden mit Wasser (3 x 70 ml) und NaCI-Lösung (1 x 70 ml) gewaschen, über Na₂SO₄ getrocknet und eingedampft. Der Eindampfrückstand (5,02 g braunes Öl) wurde aus 8 ml CH₂Cl₂/Isopropanol (5:3) kristallisiert zu Verbindung **35** (4,14β-Dihydroxy-3-methoxy-5β,17-dimethylmorphinan-6-on) (3,39 g, 81%). 200 mg davon wurden in Aceton gelöst und mit etherischer HCl 135 mg der Verbindung **35**.HCl gefällt: Fp. > 185 °C (Zers.); IR (KBr): 3200 (OH), 1700 (C=O)cm⁻¹. Analytische Daten der Base Verbindung **35**: ¹H-NMR (DMSO-d₆): δ 8,43 (s, C₄-OH), 6,77 (d, 1 arom. H, J = 8,2 Hz), 6,57 (d, 1 arom. H, J = 8,2 Hz), 4,49 (s; C₁₄-OH), 3.74 (s, C₃-OCH₃), 2,25 (s, NCH₃); 1,30 (d, C₅-CH₃, J = 7,4 Hz); MS (CI): m/z 332 (M⁺+1).

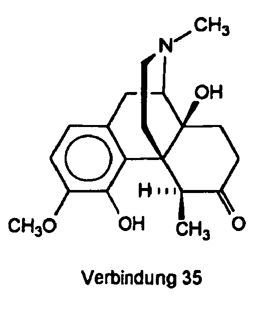

Verbindung **35** (Base) (9,5 g, 28,67 mmol) wurde in 45 g 40%iger Tetrabutylammoniumhydroxid-Lösung bei Raumtemperatur unter N₂ gelöst. Dann wurde eine Lösung von Dimethylsulfat (2,75 ml (=3,67 g), 29,06 mmol) in 50 ml Dichlormethan zugefügt. Das Gemisch wurde 5 h bei Raumtemperatur unter N₂ heftig gerührt und dann 100 ml Wasser zugesetzt. Die wässrige Phase wurde mit Dichlormethan (3 x 50 ml) extrahiert. Die vereinigten organischen Phasen wurden mit 2 M Ammoniaklösung (1 x 70 ml), Wasser (12 x 100 ml) und ges. Natriumchloridlösung (1 x 100 ml) gewaschen, über Natriumsulfat getrocknet und eingedampft. Der Eindampfrückstand (8,16 g helles Öl) wurde aus 4 ml MeOH kristallisiert. Ausbeute 5,06 g (51%) 14β-Hydroxy-3,4-dimethoxy-5β,17-dimethylmorphinan-6-on (Verbindung **36**). Fp. 122-124 °C; IR (KBr): 1704 (C=O) cm⁻¹; ¹H-NMR (CDCl₃): δ 6,78 (s, 2 arom. H), 4,31 (s, C14-OH), 3,87 und 3,82 (2 s, 2,OCH₃), 2,32 (s, NCH₃,), 1,28 (d, C₅-CH₃, J = 6,8 Hz); MS (Cl): m/z 346 (M⁺+1).

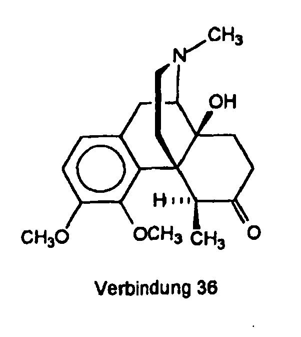

Ein Gemisch aus Verbindung **36** (7,42 g, 21,48 mmol), 2,20 ml Methansulfonsäure (3.26 g, 33.93 mmol) und 100 ml Diethylenglykol wurde 1 h lang unter N₂ und bei einer Badtemperatur von 80 °C gerührt Dann wurde das Gemisch auf 600 ml Eiswasser gegossen, mit konzentriertem Ammoniak alkalisiert und mit Dichlormethan extrahiert (1 x 200 ml, 4 x 50 ml). Die vereinigten organischen Phasen wurden mit Wasser (5 x 200 ml) und gesättigter Natriumchloridlösung (1 x 200 ml) gewaschen, über Natriumsulfat getrocknet und eingedampft. Der Eindampfrückstand (8.13 g rötliche Kristalle) wurde aus 8 ml Methanol umkristallisiert. Ausbeute 4,91 g (59 %) 14β-Hydroxy-3,4-dimethoxy-5β,17-dimethylmorphinan-6-spiro-2'-(1,3-dioxolan). Fp.155-157 °C; ¹H-NMR (CDCl₃): δ 6,76 (s, 2 arom. H), 4,36 (s, C14-OH), 3,91-3,57 (m, 4 H, OCH₂CH₂O-C), 3,83 und 3,74 (2 s, 2 CH₃O), 2,30 (s, CH₃N), 1,35 (d, C5-CH₃, J = 5,2 Hz); MS (Cl): m/z 390 (M⁺ + 1).

NaH (0,13 g, 5,41 mmol) (erhalten aus 0,21 g 60% NaH-Dispersion in Öl durch Waschen mit Petrolether) wurde einer Lösung von 14β-Hydroxy-3,4-dimethoxy-5β,17-dimethylmorphinan-6-spiro-2'-(1,3-dioxolan)(1,00 g, 2,57 mmol) in 100 ml wasserfreiem N,N-Dimethylformamid unter N₂ bei 0° C (Badtemperatur) unter Rühren zugefügt Nach 15 min wurde Benzylbromid (0,88 g, 5,14 mmol) zugesetzt und das Gemisch 3 h bei Raumtemperatur gerührt. Dann wurde das überschüssige Natriumhydrid mit Eisstückchen zerstört, das Reaktionsgemisch auf 300 ml Wasser gegossen und mit Dichlormethan / Isopropanol (5 : 2) extrahiert (3 x 70 ml). Die vereinigten organischen Phasen wurden mit Wasser (5 x 200 ml) und einer gesättigten Natriumchloridlösung (1 x 200 ml) gewaschen, über Natriumsulfat getrocknet und eingedampft. Der Eindampfrückstand (1.65 g gelbliche Kristalle) wurde aus 2 ml Ethanol kristallisiert. Ausbeute 0,65 g (53 %) 14β-Benzyloxy-3,4-dimethoxy-5β,17-dimethylmorphinan-6-spiro-2'-(1,3-dioxolan). Fp. 201-202 °C; ¹H-NMR (CDCl₃): δ 7,49-7,23 (m, 5 arom. H), 6,77 (s, 2 arom. H), 4,61 (d, 1H, PhCH₂O, J = 10,5 Hz), 4,29 (d, 1H, PhCH₂O, J = 10,5 Hz), 3,88-3,56 (m, 4 H, OCH₂CH₂O), 3,83 und 3,76 (2 s, 2 CH₃O), 2,30 (s, CH₃N), 1,25 (d, C5-CH₃, J = 7,4 Hz); MS (Cl): m/z 480 (M⁺ + 1).

Eine Lösung von 14β-Benzyloxy-3,4-dimethoxy-5β,17-dimethylmorphinan-6-spiro-2'-(1,3-dioxolan) (0,50 g, 1,04 mmol) in 10 ml eines Gemisches von 5 ml Methanol, 4 ml Wasser und 1 ml konz. HCl wurde 30 Minuten lang rückflußerhitzt. Dann wurde das Gemisch auf 150 ml Eiswasser gegossen, mit konzentriertem Ammoniak alkalisiert und mit Dichlormethan / Isopropanol (4 : 1) extrahiert (2 x 50 ml, 2 x 25 ml). Die vereinigten organischen Phasen wurden mit Wasser (2 x 100 ml) und einer gesättigten Natriumchloridlösung (1 x 100 ml) gewaschen, über Natriumsulfat getrocknet und eingedampft. Der Eindampfrückstand (0,51 g helles Öl) wurde aus 1 ml Methanol kristallisiert. Ausbeute 0,34 g (76 %) Verbindung **37.** Fp. 166-167 °C; IR (KBr): 1695 (CO) cm⁻¹; ¹H-NMR (CDCl₃): δ 7,34-7,21 (m, 5 arom. H), 6,83 (d, 1 arom. H, J = 8,6 Hz), 6,78 (d, 1 arom. H, J = 8,6 Hz), 4,63 (d, 1 H, PhCH₂O, J = 10,8 Hz), 4,32 (d, 1 H, PhCH₂O, J = 10,8 Hz), 3,86 und 3,83 (2s, 2 CH₃O), 2,30 (s, CH₃N), 1,43 (d, C5-CH₃, J = 7,0 Hz); MS (Cl): m/z 436 (M⁺ + 1).

### Beispiel 36:

### Synthese des 4,5α-Epoxy-3-hydroxy-17,17-dimethyl-6-oxo-14β-[(3-phenylpropyl]oxy]morphinaniumiodids (Verbindung 38)

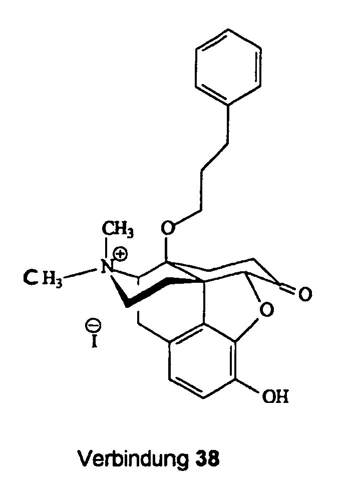

Eine Lösung von 4,5α-Epoxy-3-methoxy-17-methyl-14-[(3-phenylpropyl)oxy]-morphinan-6-on (= 14-O-(3-Phenylpropyl)oxycodon; s. Beispiel 1) (3,0 g, 6,90 mmol) in 48%iger HBr (30 ml) wurde 15 Minuten lang rückflusserhitzt. Nach Beendigung der Reaktion wurde mit Eis gekühlt, mit konz. NH₄OH alkalisiert und mit CH₂Cl₂ (5 x 50 ml) extrahiert Die vereinigten organischen Phasen wurden mit Wasser (3 x 50 ml) und ges. NaCl-Lösung (1 x 50 ml) gewaschen, über Na₂SO₄ getrocknet und eingedampft. Der Eindampfrückstand (3,5 g, braunes Öl) wurde mittels Säulenchromatographie (Kieselgel; CH₂Cl₂/MeOH/konz. NH₄OH: 6 I: (250/2/0,5); je 0,5 I: (250/3/0,5), (250/4/0,5), (250/6/0,5), (250/8/0,5)) gereinigt. Der Eindampfrückstand (4,5α-Epoxy-3-hydroxy-17-methyl-14β-[(3-phenylpropyl)oxy]morphinan-6-on, 1,36 g gelbes Öl, 47%) konnte nicht kristallisiert werden. Deshalb wurde ein Teil als Öl der nächsten Reaktion zugeführt, der Rest wie üblich in das Hydrochlorid (4,5α-Epoxy-3-hydroxy-17-methyl-14β-[(3-phenylpropyl)oxy]morphinan-6-on Hydrochlorid) überführt. Analytische Daten des Hydrochlorids: Fp. 185-189 °C; ¹H-NMR(DMSD-d₆): δ 9,50 (s, C₃-OH), 8,68 (s, breit, ⁺NH), 7,34-7,16 (m, 5 arom. H), 6,70 (d, 1 arom. H, J = 8,0 Hz), 6,65 (d, 1 arom. H, J = 8,0 Hz), 4,87 (s, C₅-H), 2,92 (d, ⁺NCH₃).

Eine Lösung von 4,5α-Epoxy-3-hydroxy-17-methyl-14β-[(3-phenylpropyl)oxy]morphinan-6-on (Öl, 257 mg, 0,61 mmol) und Methyliodid (0,38 ml, 6,11 mmol) in Acetonitril (10 ml) wurde 2 Tage lang bei 50 °C (Badtemperatur) unter N₂ gerührt Eine DC-Untersuchung zeigte, daß die Reaktion nicht abgeschlossen war. Deshalb wurde weiteres Methyliodid (0,38 ml, 6,11 mmol) zugegeben und weitere 3 Tage unter den selben Bedingungen gerührt. Dann wurde das Lösungsmittel abgedampft und der Eindampfrückstand (367 mg gelbes Schaumharz) mittels Säulenchromatographie (Kieselgel; CH₂Cl₂/MeOH (10:1)) gereinigt. Eindampfrückstand 240 mg (71%) amorphe Verbindung **38**. Fp. 164-170°C; IR (KBr):1723 (C=O) cm⁻¹; ¹H-NMR (DMSO-d₆): δ 9,57 (s, C3-OH), 7,3-7,16 (m, 5 arom. H), 6,67 (s, 2 arom. H, C₁-H, C₂-H), 4,88 (s, C₅-H), 3,56 (s, N⁺CH₃, axial), 3,21 (s, ⁺NCH₃, äquatorial); MS (Cl): m/z 434 (M⁺).

### Beispiel 37:

### Synthese des (17S)-4,5cc-Epoxy-17-ethyl-3-hydroxy-1 7-methyl-"xo-1 4β-[(3-phenylpropyl]oxy]morphinaniumiodids (Verbindung 39)

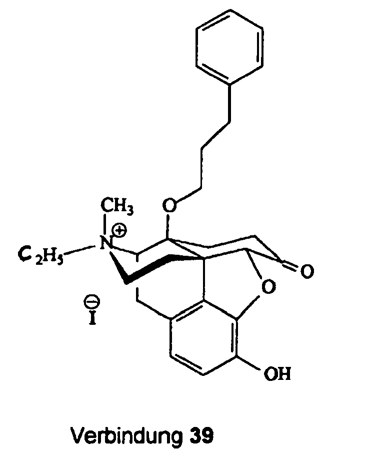

Eine Lösung von Verbindung **20** (s. Beispiel 20) (250 mg, 0,58 mmol) und Methyliodid (0,36 ml, 5,80 mmol) in Acetonitril (10 ml) wurde 6 Tage lang bei 50 °C (Badtemperatur) unter N₂ gerührt. Die Lösung wurde eingedampft und der Eindampfrückstand (320 mg hellbeiges Schaumharz) mittels Säulenchromatographie (Kieselgel; CH₂Cl₂/MeOH: je 1 I: (95/5), (92,5/7,5)) gereinigt. Eindampfrückstand (240 mg, 72%) amorphe Verbindung **39.** Fp. 171-176 °C; IR (KBr): 1723 (C=O) cm⁻¹; ¹H-NMR (DMSO-*d*₆): δ 9,55 (s, C3-OH), 7,31-7,16 (m, 5 arom. H), 6,68 (s, 2 arom. H, C₁-H, C₂-H), 4,87 (s, C₅-H), 3,44 (3 H, N⁺CH₃, axial), 1,34 (t, 3 H, ⁺NCH₂CH₃, J = 7 Hz); MS (Cl): m/z 448 (M⁺).

### Beispiel 38:

### Synthese des (17R)-4,5α-Epoxy-3-hydroxy-17-methyl-6-oxo-14β-[(3-phenylpropyl)oxy]-17-[(2-R,S-tetrahydrofurfuran-2-yl)methyl]morphinaniumiodids (Verbindung 40)

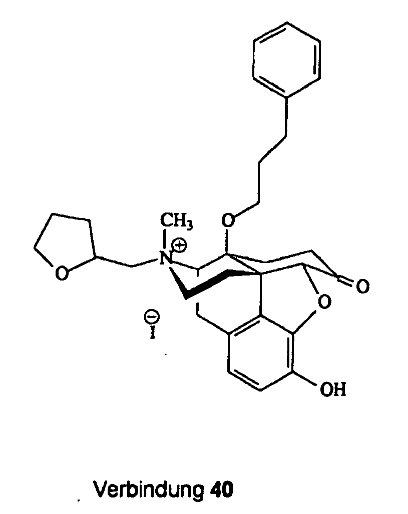

Eine Lösung von Verbindung **10** (s. Beispiel 10) (190 mg, 0,39 mmol) und Methyliodid (0,24 ml, 3,9 mmol) in Acetonitril (8 ml) wurde 10 Tage lang bei 40-50 °C (Badtemperatur) unter N₂ gerührt. Die Lösung wurde eingedampft und der Eindampfrückstand (248 mg beiges Schaumharz) mittels Säulenchromatographie (Kieselgel;CH₂Cl₂/MeOH: 2 I: (250/5); 1 I: (250/7,5)) gereinigt. Der resultierende Eindampfrückstand (70 mg gelbes Schaumharz, 28%) war reine Verbindung **40**. Fp. 156-159 °C; IR (KBr): 1724 (C=O) cm⁻¹; ¹H-NMR(DMSO-d₆): δ 9,53 (s, C3-OH), 7,30-7,16 (m, 5 arom. H), 6,68 (s, 2 arom. H, C₁-H, C₂-H), 4,85 (s, 1 H, C₅-H), 3,58 (s, ⁺NCH₃, axial); MS(CI): m/z 504 (M⁺).

### Beispiel 39

### Synthese des (17R)-17-Allyl-4,5α-epoxy-14β-ethoxy-3-hydroxy-17-methyl-6-oxomorphinaniumiodids (Verbindung 41)

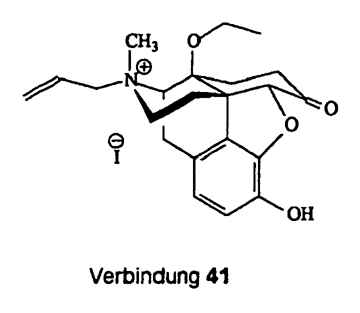

Eine Lösung von 14-O-Ethylnaloxon (Kobylecki et al. J. Med. Chem. 1982, 25, S. 116) (300 mg, 0,84 mmol) und Methyliodid (0,25 ml, 4,2 mmol) wurden in 8 ml Acetonitril 7 Tage bei 30 °C (Badtemperatur) gerührt und dann eingedampft. Der Eindampfrückstand (450 mg hellbraunes Öl) wurde mittels Säulenchromatographie (Kieselgel; CH₂Cl₂/MeOH (250:4)) gereinigt. Eindampfrückstand (110 mg, 35%) amorphe Verbindung **41**. Fp. 130-134 °C; IR (KBr): 1724 (CO) cm⁻¹; ¹H-NMR (DMSO-d₆): δ 9,54 (s, C3-OH), 6,70 (s, 2 arom. H), 6,32-6,07 (m, 1 olef. H), 5,84-5,63 (m, 2 olef. H), 4,92 (s, C5-H), 3,49 (s, ⁺NCH₃, axial), 1,24 (t, 3 H, OCH₂CH₃, J = 6,8 Hz); MS(Cl): m/z 370 (M⁺).

### Beispiel 40

### Synthese des (17R)-17-Allyl-4,5α-epoxy 3-hydroxy-14β-methoxy-17-methyl-6-oxomorphinaniumiodids (Verbindung 42)

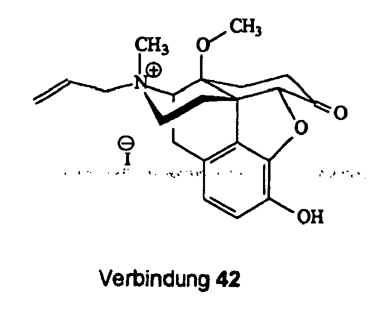

Eine Lösung von 14-O-Methylnaloxon (Kobylecki et al. J. Med. Chem. 1982, 25, S. 116) (230 mg, 0,67 mmol) und Methyliodid (0,21 ml, 3,37 mmol) wurde in 10 ml Acetonitril 7 Tage bei 40 °C (Badtemperatur) gerührt und dann eingedampft. Der Eindampfrückstand (341 mg hellbraunes Öl) wurde mittels Säulenchromatographie (Kieselgel; CH₂Cl₂/MeOH (250:4,5) → 250:9)) gereinigt. Eindampfrückstand (202 mg, 62%) amorphe Verbindung **42**. Fp. 178-181 °C; IR (KBr): 1722 (CO) cm⁻¹; ¹H-NMR (DMSO-d₆): δ 9,55 (s, C3-OH), 6,70 (s, 2 arom. H), 6,28-6,06 (m, 1 olef. H), 5,82-5,64 (m, 2 olef. H), 4,94 (s, C5-H), 3,45 (s, ⁺NCH₃, axial), 3,38 (s, CH₃O); MS(CI): m/z 356 (M⁺).

### Beispiel 43

### Synthese des (17S)-17-Allyl-4,5α-epoxy-3-hydroxy-14β-methoxy-17-methyl-6-oxomorphinaniumiodids (Verbindung 43)

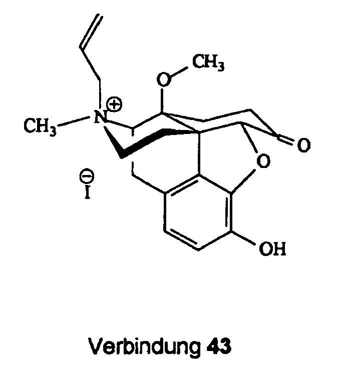

Eine Lösung von 14-O-Methyloxymorphon (Schmidhammer et al. J. Med. Chem. 1984, 27, S. 1575-1579) (210 mg, 0,67 mmol) und Allyliodid (0,28 ml, 3,35 mmol) wurden in 5 ml N,N-Dimethylformamid 7 Tage bei 40 °C (Badtemperatur) gerührt und dann eingedampft Der Eindampfrückstand (484 mg braunes Öl) wurde mittels Säulenchromatographie (Kieselgel; CH₂Cl₂/MeOH (250:5) → 250:12)) gereinigt. Der Eindampfrückstand (134 mg) wurde nochmals mittels Säulenchromatographie (Kieselgel; CH₂Cl₂/MeOH (250:4,5)) gereinigt. Eindampfrückstand 77 mg (27%) amorphe Verbindung **43**. Fp. >140 °C; IR (KBr): 1724 (CO) cm⁻¹; ¹H-NMR (DMSO-d₆): δ 9,57 (s, C3-OH), 6,68 (s, 2 arom. H), 6,28-6,08 (m, 1 olef. H), 5,75-5,63 (m, 2 olef. H), 4,95 (s, C5-H), 3,42 (s, CH₃O), 3,05 (s, ⁺NCH₃, äquatorial), MS(Cl): m/z 356 (M⁺).

### Beispiel 42

### Synthese des 4,5α-Epoxy-3-hydroxy-14β-methoxy-17,17-dimethyl-6-oxo-morphinaniumiodids (Verbindung 44)

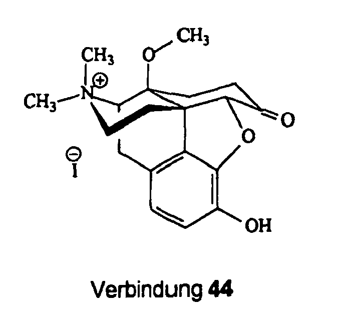

Eine Lösung von 14-O-Methyloxymorphon (Schmidhammer et al. J. Med. Chem. 1984, 27, S. 1575-1579) (200 mg, 0,63 mmol) und Methyliodid (0,32 ml, 5,07 mmol) in Acetonitril (10 ml) wurde 21 Stunden lang unter N₂ bei 50 °C (Badtemperatur) gerührt. Dann wurde nochmals Methyliodid (0,2 ml, 3,17 mmol) zugegeben, weitere 24 h lang unter den selben Bedingungen gerührt und eingedampft. Der Eindampfrückstand (324 mg, braunes Schaumharz) wurde aus MeOH (0,5 ml) kristallisiert und durch Umkristallisation aus MeOH gereinigt Ausbeute 244 mg (84 %) Verbindung **44**. Fp. 225-240 °C; IR (KBr): 1725 (C=O) cm⁻¹;¹H-NMR (DMSO-d₆): δ 9,55 (s, C₃-OH), 6,69 (s, 2 arom. H, C₁-H, C₂-H), 4,94 (s, C₅-H), 3,55 (s, ⁺NCH₃, axial), 3,38 (s, OCH₃), 3,22 (s, ⁺NCH₃, äquatorial); MS (CI): m/z 330 (M⁺).

### Beispiel 43

### Synthese des 5β-Benzyl-14β-(butyloxy)-4,5α-epoxy-3-hydroxy-17,17-dimethyl-6-oxomorphinaniumiodids (Verbindung 45)

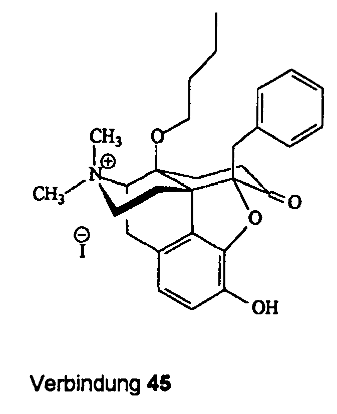

NaH (0,52 g, 21,6 mmol) (erhalten aus 0,86 g 60% NaH-Dispersion in Öl durch Waschen mit Petrolether) wurde einer Lösung von 5β-Benzyl-14-hydroxycodeinon (M. Gates et al. J. Org. Chem. 1989, 54, S.972-974) (2,90 g, 7,2 mmol) in 40 ml wasserfreiem N,N-Dimethylformamid unter N₂ bei 0 °C (Badtemperatur) unter Rühren zugefügt. Nach 20 min wurde trans-Crotylbromid (1,26 g, 9,3 mmol) zugesetzt und das Gemisch zunächst für 40 min bei 0 °C (Badtemperatur), dann bei Raumtemperatur für 2 h gerührt. Dann wurden Eisstückchen zugesetzt, bis keine Wasserstoffentwicklung mehr festzustellen war, das Gemisch auf 100 ml Wasser gegossen und mit Dichlormethan (1 x 100 ml, 5 x 50 ml) extrahiert. Die vereinigten organischen Phasen wurden mit gesättigter Natriumchloridlösung (6 x 250ml) gewaschen, über Natriumsulfat getrocknet, eingedampft und der Eindampfrückstand (3,25 g braunes Öl) mittels Säulenchromatographie (Kieselgel; CH₂Cl₂ / MeOH / konz. NH₄OH 250 : 6 : 0,5) gereinigt. Vom resultierenden gelblichen Öl (1,86 g, 56%) wurden 0,1 g in 5 ml Ether gelöst und etherische HCl zugefügt. Es wurden 0,1 g farblose Kristalle von 5β-Benzyl-14β-{[(E)-(but-2-enyl]oxy}-7,8-didehydro-4,5α-epoxy-3-methoxy-17-methylmorphinan-6-on Hydrochlorid isoliert. Fp. 125-130 °C; MS (Cl): 458 (M⁺+1); IR (KBr): 1684(C=O) cm⁻¹;¹H-NMR (DMSO-d₆, δ in ppm): 9,22 (s, breit, HN⁺), 7,28-7,13 (m, 5 arom. H; 1 olefin. H, CH-8), 6,84 (d, 1 arom. H, J = 8,4 Hz), 6,74 (d, 1 arom. H, J = 8,4 Hz), 6,23 (d, 1 olefin. H, CH-7, J = 10,2 Hz), 5,69-5,34 (m, 2 olefin. H), 3,75 (s. CH₃O), 2,91 (d, CH₃N⁺), 1,69-1,65 (d, 3 H, C14-OCH₂CHCHCH₃, J = 7,4 Hz).

Ein Gemisch von 5β-Benzyl-14β-{[(E)-(but-2-enyl]oxy}-7,8-didehydro-4,5α-epoxy-3-methoxy-17-methylmorphinan-6-on (1,75 g, 3,8 mmol) und 0,24 g Pd/C (10%) in 60 ml Methanol wurde 1,5 Stunden bei 35 psi und Raumtemperatur hydriert. Der Katalysator wurde abfiltriert und mit 20 ml Methanol gewaschen. Das Filtrat wurde eingedampft. Ausbeute 1,43 g (81 %) hellgelbes Öl, von dem 0,1 g in 5 ml Ether gelöst und durch Zugabe von etherischer HCl das Hydrochlorid gefällt wurde. Es wurden 0,1 g farblose Kristalle von 5β-Benzyl-14β-(butoxy)-4,5α-epoxy-3-methoxy-17-methylmorphinan-6-on Hydrochlorid isoliert. Fp. 148-152 °C; MS (Cl): 462 (M⁺+1); IR (KBr): 1729 (C=O) cm⁻¹; ¹H-NMR (DMSO-d₆, δ in ppm): 9,00 (s, breit, HN⁺), 7,29-7,16 (m, 5 arom. H), 6,85 (d, 1 arom. H, J = 8,4 Hz), 6,75 (d, 1 arom. H, J = 8,4 Hz), 3,80 (s, CH₃O), 2,94 (d, CH₃N⁺, J = 4,4 Hz), 1,02 (t, 3 H, C14-O(CH₂)₃CH₃, J = 7,2 Hz).

Eine Lösung von 5β-Benzyl-14β-butoxy-4,5α-epoxy-3-methoxy-17-methylmorphinan-6-on (1,30 g, 2,82 mmol) in 10 ml 48% HBr wurde 20 min rückflußerhitzt. Danach wurde die Lösung abgekühlt, auf 100 ml Eis gegossen, mit konz. NH₄OH alkalisiert und mit Dichlormethan (5 x 50 ml) extrahiert. Die vereinigten organischen Phasen wurden mit gesättigter Natriumchloridlösung (3 x 150 ml) gewaschen, über Natriumsulfat getrocknet und eingedampft. Der Rückstand (0,97 g grauschwarzes Schaumharz) wurde mittels Säulenchromatographie (Kieselgel; CH₂Cl₂ / MeOH / konz. NH₄OH 250 : 6 : 0,5) gereinigt. Ausbeute 0,82 g (65%) dunkelgelbes Öl, von dem 0,06 g in 5 ml Ether gelöst und durch Zugabe von etherischer HCl das Hydrochlorid gefällt wurde. Es wurden 0,06 g beige Kristalle von 5β-Benzyl-14β-butoxy-4,5α-epoxy-3-hydroxy-17-methylmorphinan-6-on Hydrochlorid isoliert. Fp. 200-204 °C; MS (Cl): 448 (M⁺+1); IR (KBr): 1729 (C=O) cm⁻¹; ¹H-NMR (DMSO-d₆, δ in ppm): 9,45 (s, OH), 8,72 (s, breit, HN⁺), 7,25-7,22 (m, 5 arom. H), 6,67-6,63 (s, 2 arom. H, CH-1 und CH-2). 2,93 (d, CH₃N⁺), 1,02 (t, 3 H, C14-O(CH₂)₃CH₃, J = 7,3 Hz).

Eine Lösung von 5β-Benzyl-14β-butoxy-4,5α-epoxy-3-hydroxy-17-methylmorphinan-6-on (0,32 g, 0,72 mmol) und Methyliodid (0,51 g, 3,58 mmol) in 10 ml wasserfreiem Acetonitril wurde unter N₂ bei 40 °C (Badtemperatur) 2 Tage gerührt und dann eingedampft. Der Eindampfrückstand (0,41 g braunes Öl) wurde mittels Säulenchromatographie (Kieselgel; CH₂Cl₂ / MeOH 250 : 6) gereinigt Ausbeute 0,22 g (54%) leicht beige gefärbte Kristalle der Verbindung **45**. Fp. 188-195 °C; MS (Cl): 462 (M⁺); IR (KBr): 1729 (C=O) cm⁻¹; ¹H-NMR (DMSO-d₆, δ in ppm): 9,49 (s, OH), 7,28-7,20 (m, 5 arom. H), 6,69 (d, 1 arom. H, J = 8,2 Hz), 6,62 (d, 1 arom. H, J = 8,2 Hz), 3,61 (s, CH₃N⁺, axial), 3,23 (s, CH₃N⁺, äquatorial), 1,01 (t, 3 H, C14-O(CH₂)₃CH₃, J = 7,0 Hz).

### Beispiel 44:

### Synthese des (17S)-17-Allyl-5β-benzyl-14β-butoxy-4,5α-epoxy-3-hydroxy-17-methyl-6-oxomorphinaniumiodids (Verbindung 46)

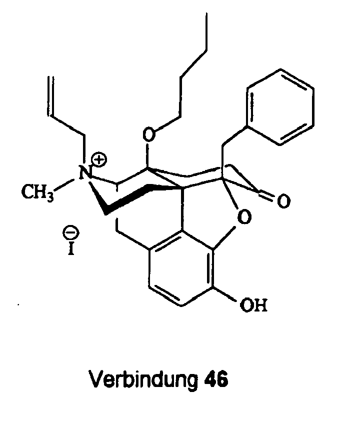

Eine Lösung von 5β-Benzyl-14β-butoxy-4,5α-epoxy-3-hydroxy-17-methylmorphinan-6-on (s. Beispiel 45) (0,31 g, 0,69 mmol) und Allylbromid (0,94 g, 5,59 mmol) in 10 ml wasserfreiem Acetonitril wurde unter N₂ bei 40 °C (Badtemperatur) einen Tag lang gerührt und dann eingedampft. Der Eindampfrückstand (0,45 g braunes Öl) wurde mittels Säulenchromatographie (Kieselgel; CH₂Cl₂ / MeOH 250 : 5) gereinigt. Ausbeute 0,11 g (26%) beige Kristalle der Verbindung **46**. Fp. 200-205 °C; MS (Cl): 488,2 (M⁺); IR (KBr): 1728 (C=O) cm⁻¹; ¹H-NMR (DMSO-d₆, δ in ppm): 9,49 (s, OH), 7,26-7,23 (m, 5 arom. H), 6,67 (s, 2 arom. H, CH-1 und CH-2), 6,22-6,13 (m, 1 olefin. H), 5,80-5,66 (m, 2 olefin. H), 3,50 (s, CH₃N⁺, äquatorial), 1,02 (t, C14-O(CH₂)₃CH₃, J = 7,4 Hz).

### Beispiel 45

### Synthese des 14β-Butoxy-4,5α-epoxy-3-hydroxy-17,17-dimethyl-6-oxomorphinaniumiodids (Verbindung 47)

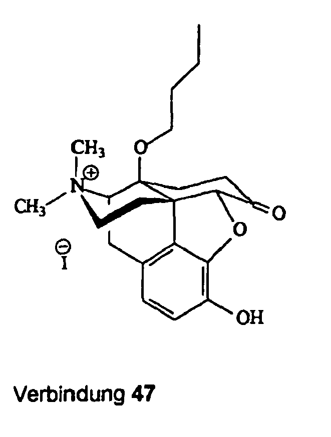

NaH (0,92 g, 38,3 mmol) (erhalten aus 1,53 g 60% NaH-Dispersion in Öl durch Waschen mit Petrolether) wurde einer Suspension von 14-Hydroxycodeinon (lijima et al., J. Med. Chem. 1978, 21, S.398) (4,0 g, 12,8 mmol) in 50 ml wasserfreiem N,N-Dimethylformamid unter N₂ bei 0 °C (Badtemperatur) unter Rühren zugefügt. Nach 20 min wurde in 20 ml DMF gelöstes trans-Crotylbromid (2,24 g, 16,6 mmol) zugetropft und das Gemisch zunächst für 40 min bei 0 °C (Badtemperatur), dann bei Raumtemperatur für 3 h weiter gerührt. Dann wurden Eisstückchen zugesetzt, bis keine Wasserstoffentwicklung mehr festzustellen war, das Gemisch wurde auf 100 ml Wasser gegossen und mit Dichlormethan (1 x 100 ml, 5 x 50 ml) extrahiert, die vereinigten organischen Phasen mit gesättigter Natriumchloridlösung (6 x 250ml) gewaschen und über Natriumsulfat getrocknet. Der Eindampfrückstand (5,2 g gelbbraunes Öl) wurde mittels Säulenchromatographie (Kieselgel; CH₂Cl₂ / MeOH / konz. NH₄OH (250 : 5 : 0,5)) gereinigt. Es resultierte ein gelbliches Öl (2,20 g), das aus Methanol kristallisiert wurde. Ausbeute 1,75 g (36%) gelbliche Kristalle von 14β-{[(E)-(But-2-enyl]oxy}-7,8-didehydro-4,5α-epoxy-3-methoxy-17-methylmorphinan-6-on. Fp. 113-117 °C; MS (Cl): 368,2 (M⁺+1); IR (KBr): 1679 (C=O) cm⁻¹; ¹H-NMR (DMSO-d₆, δ in ppm): 7,13 (d, 1 olefin. H; CH-8, J = 10,0 Hz), 6,73 (d, 1 arom. H, J = 8,0 Hz), 6,64 (d, 1 arom. H, J = 8,0 Hz), 6,14 (d, 1 olefin. H, CH-7, J = 9,8 Hz), 5,69-5,43 (m, 2 olefin. H), 4,78 (s, CH-5), 4,03-3,80 (m, 2H, C14-OCH₂-). 3,72 (s, CH₃O), 2,34 (s, CH₃N), 1,64 (d, OCH₂CHCHCH₃, J = 6,0 Hz).

Ein Gemisch von 14β-([(E)-(But-2-enyl]oxy}-7,8-didehydro-4,5α-epoxy-3-methoxy-17-methylmorphinan-6-on (0,82 g, 2,23 mmol) und 0,82 g Pd/C (10%) in 50 ml Methanol wurde 45 min bei 30 psi und Raumtemperatur hydriert. Der Katalysator wurde abfiltriert, mit 20 ml Methanol gewaschen und das Filtrat eingedampft. Ausbeute 1,02 g hellgelbes Öl, das mittels Säulenchromatographie (Kieselgel; CH₂Cl₂ / MeOH / konz. NH₄OH (250 : 2 : 0,5)) gereinigt wurde. Es resultierte ein gelbliches Öl, das aus Methanol kristallisiert wurde. Ausbeute 0,52 g (63%) farblose Kristalle von 14β-Butoxy-4,5α-epoxy-3-methoxy-17-methylmorphinan-6-on isoliert. Fp. 112-117 °C; MS (Cl): 371,1 (M⁺+1); IR (KBr): 1722 (C=O) cm⁻¹; ¹H-NMR (DMSO-d₆, δ in ppm): 6,74 (d, 1 arom. H, J = 8,0 Hz), 6,65 (d, 1 arom. H, J = 8,2 Hz), 4,75 (s, CH-5), 3,78 (s, CH₃O), 2,29 (s, CH₃N), 0,93 (t, OCH₂CH₂CH₂CH₃, J = 7,2 Hz).

Eine Lösung von 14β-Butoxy-4,5α-epoxy-3-methoxy-17-methylmorphinan-6-on (0,30 g, 0,81 mmol) in 3,2 ml 48% HBr wurde 8 min rücktlußerhitzt. Danach wurde die Lösung abgekühlt, auf 20 ml Eis gegossen, mit konz. NH₄OH alkalisiert und mit Dichlormethan (5 x 30 ml) extrahiert. Die vereinigten organischen Phasen wurden mit gesättigter Natriumchloridlösung (3 x 100 ml) gewaschen, über Natriumsulfat getrocknet und eingedampft. Der Rückstand (0,29 g braunes Öl) wurde mittels Säulenchromatographie (Kieselgel; CH₂Cl₂ / MeOH / konz. NH₄OH 250 : 5 : 0,5) gereinigt. Ausbeute 0,06 g (21 %) dunkelgelbes Öl, welches in 5 ml Ether gelöst wurde und mit etherischer HCl in das Hydrochlorid überführt wurde. Es wurden 0,06 g hellbraune Kristalle von 14ß-Butoxy-4,5α-epoxy-3-hydroxy-17-methylmorphinan-6-on Hydrochlorid isoliert. Fp. 168-172°C; MS (Cl): 358,2 (M⁺+1); IR (KBr): 1725 (C=O) cm⁻¹; ¹H-NMR (DMSO-d₆, δ in ppm): 9,51 (s, OH), 8,75 (s, breit, HN⁺), 6,71 (d, 1 arom. H, J = 8,2 Hz), 6,65 (d, 1 arom. H, J = 8,0 Hz), 4,92 (s, CH-5), 2,82 (d, CH₃N⁺, J = 4,4 Hz), 0,95 (t, O(CH₂)₃CH₃, J = 7,4 Hz).

Eine Lösung von 14β-Butoxy-4,5α-epoxy-3-hydroxy-17-methylmorphinan-6-on (0,53 g, 1,49 mmol) und Methyliodid (1,06 g, 7,44 mmol) in 10 ml wasserfreiem Acetonitril wurde unter N₂ bei 40 °C (Badtemperatur) 2 Tage gerührt und dann eingedampft. Der Eindampfrückstand (0,70 g braunes Öl) wurde mittels Säulenchromatographie (Kieselgel; CH₂Cl₂ / MeOH 250 : 4) gereinigt. Ausbeute 0,31 g (42%) farblose Kristalle der Verbindung 47. Fp. 180-185 °C; MS (Cl): 372,2 (M⁺); IR (KBr): 1726 (C=O) cm⁻¹; ¹H-NMR (DMSO-d₆, δ in ppm): 9,55 (s, OH), 6,68 (s, 2 arom. H), 4,95 (s, CH-5), 3,57 (s,CH₃N⁺,axial), 3,21 (s, CH₃N⁺, äquatorial), 0,94 (t, O(CH₂)₃CH₃, J = 7,3 Hz).

### Beispiel 46

### Synthese des (17R)-17-Cyclopropylmethyl-4,5α-epoxy-3-hydroxy-17-methyl-6-oxo-14β-[(3-phenylpropyl)oxy]morphinaniumiodids (Verbindung 48) und des (17R)-17-Cyclopropylmethyl-4,5α-epoxy-3-methoxy-17-methyl-6-oxo-14β-[(3-phenylpropyl)oxy]morphinaniumiodids (Verbindung 49)

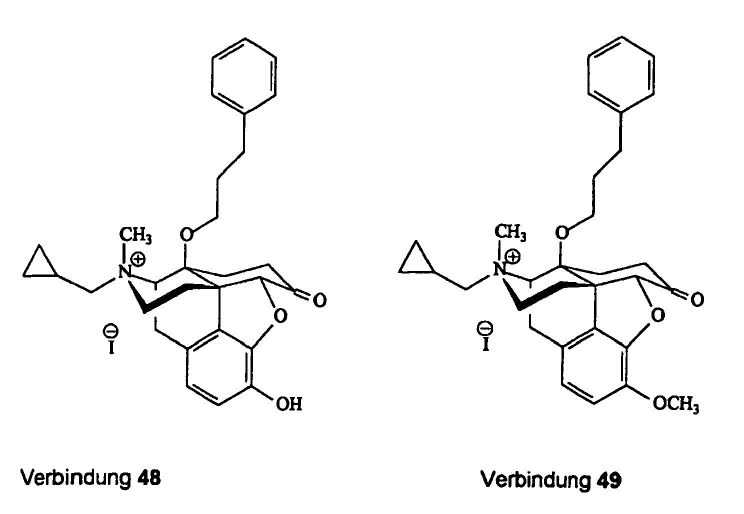

Ein Gemisch aus 17-Cyclopropylmethyl-4,5α-epoxymorphinan-6-spiro-2'-(1,3-dioxolan)-3,14β-diol (Schmidhammer et al., Heterocycles 1998, 49, 1, S. 489-498) (7,92 g, 20,55 mmol), K₂CO₃ (7,67 g, 55,49 mmol), Benzylbromid (4,57 g, 26,72 mmol) und 80 ml wasserfreiem N,N-Dimethylformamid wurde unter N₂ 12 h bei Raumtemperatur gerührt. Das anorganische Material wurde abfiltriert, mit Dichlormethan gewaschen und das Filtrat eingedampft. Der kristalline Eindampfrückstand (10,97 g) wurde aus Methanol umkristallisiert. Ausbeute 8,80 g (90%) farblose Kristalle von 3-Benzyloxy-17-cyclopropylmethyl-4,5α-epoxy-14β-hydroxymorphinan-6-spiro-2'-(1,3-dioxolan). Fp. 130-131 °C; MS (Cl): 476 (M⁺+1); IR (KBr): 3352 (OH) cm⁻¹; ¹H-NMR (CDCl₃, δ in ppm): 7,42-7,27 (m, 5 arom. H), 6,75 (d, 1 arom. H, J = 8,3 Hz), 6,54 (d, 1 arom. H, J = 8,3 Hz), 5,17 (d, 1 H, J = 11,7 Hz, OCH₂Ph), 5,10 (d, 1 H, J = 11,7 Hz, OCH₂Ph), 4,58 (s, CH-5), 4,19-3,73 (m, 4H, OCH₂CH₂O).

NaH (2,24 g, 93,6 mmol) (erhalten aus 3,75 g 60% NaH-Dispersion in Öl durch Waschen mit Petrolether) wurde einer Lösung von 3-Benzyloxy-17-cyclopropylmethyl-4,5α-epoxy-14β-hydroxymorphinan-6-spiro-2'-(1,3-dioxolan) (14,8 g, 31,2 mmol) in 100 ml wasserfreiem N,N-Dimethylformamid unter N₂ bei 0 °C (Badtemperatur) unter Rühren zugefügt. Nach 20 min wurde in 20 ml DMF gelöstes Cinnamylbromid (7,99 g, 40,6 mmol) zugetropft und das Gemisch für 25 min bei 0 °C (Badtemperatur), dann bei Raumtemperatur für 3,5 h gerührt. Dann wurden Eisstückchen zugesetzt, bis keine Wasserstoffentwicklung mehr festzustellen war. Das Gemisch wurde auf 200 ml Wasser gegossen, mit Dichlormethan (4 x 100 ml) extrahiert, die vereinigten organischen Phasen mit gesättigter Natriumchloridlösung (5 x 250ml) gewaschen, über Natriumsulfat getrocknet und eingedampft. Der Eindampfrückstand (18,2 g gelbbraunes Öl) wurde mittels Säulenchromatographie (Kieselgel; CH₂Cl₂ /MeOH / konz. NH₄OH 250 : 3 : 0,5) gereinigt. Es resultierte ein gelbliches Öl (6,1 g, 33%), von dem 0,2 g in 5 ml Ether gelöst wurden und mit etherischer HCl in das Hydrochlorid überführt wurden. Es wurden 0,1 g beige Kristalle von 3-Benzyloxy-17-cyclopropylmethyl-4,5α-epoxy-14β-{[(E)-3-phenylprop-2-enyl]oxy}morphinan-6-spiro-2'-(1,3-dioxolan) Hydrochlorid isoliert. Fp. 133-136 °C; MS (CI): 592 (M⁺+1); ¹H-NMR (DMSO-d₆): δ 8,23 (s, breit, HN⁺), 7,53-7,24 (m, 10 arom. H), 6,93 (d, 1 arom. H, J = 8,4 Hz), 6,70 (d, 1 arom. H, J = 8,4 Hz), 6,70 (m, 2 olefin. H), 5,15 (s, 2H, C3-OCH₂Ph), 4,65 (s, CH-5), 4,33-4,20 (m, 2H, OCH₂CHCHPh), 4,04-3,74 (m, 4H, OCH₂CH₂O).

Ein Gemisch von 3-Benzyloxy-17-cyclopropylmethyl-4,5α-epoxy-14β-{[(E)-3-phenylprop-2-enyl]oxy}morphinan-6-spiro-2'-(1,3-dioxolan) (6,08 g, 10,3 mmol) und 0,60 g Pd/C (10%) in einem Gemisch aus 100 ml Methanol und 50 ml Tetrahydrofuran wurde 2,5 Stunden bei 30 psi und Raumtemperatur hydriert. Der Katalysator wurde abfiltriert, mit je 20 ml Methanol und Tetrahydrofuran gewaschen und das Filtrat eingedampft. Eindampfrückstand 4,02 g hellgelbes Öl, das mittels Säulenchromatographie (Kieselgel; CH₂Cl₂ / MeOH / konz. NH₄OH 250 : 5:0,5) gereinigt wurde. Es resultierte ein gelbliches Öl (2,75g, 53%), von dem 0,07 g in 5 ml Ether gelöst wurden und mit etherischer HCl in das Hydrochlorid übergeführt wurden. Es wurden 0,06 g farblose Kristalle von 17-Cyclopropylmethyl-4,5α-epoxy-3-hydroxy-14β-[(3-phenylpropyl)oxy]morphinan-6-spiro-2'-(1,3-dioxolan) Hydrochlorid isoliert. Fp. 158-162 °C; MS (Cl): 504 (M⁺+1);¹H-NMR (DMSO-d₆, δ in ppm): 9,24 (s, OH), 7,79 (s, breit, HN⁺), 7,35-7,18 (m, 5 arom. H), 6,68 (d, 1 arom. H, J = 8,0 Hz), 6,57 (d, 1 arom. H, J = 8,0 Hz), 4,51 (s, CH-5), 4,09-3,71 (m, 4H, OCH₂CH₂O).

Eine Lösung von 17-Cyclopropylmethyl-4,5α-epoxy-3-hydroxy-14β-[(3-phenylpropyl)oxy]morphinan-6-spira-2'-(1,3-dioxolan) (0.22 g, 0,44 mmol) in 5 ml eines Gemisches aus HCl konz./MeOH (4:9) wurde 1 h lang rückflußerhitzt. Nach dem Abkühlen wurde das entstandene Kristallisat abfiltriert, das Filtrat eingedampft und der Rückstand aus Ether kristallisiert. Ausbeute 0,122 g (61 %) farblose nadelförmige Kristalle von 17-Cyclopropylmethyl-4,5α-epoxy-3-hydroxy-14β-[(3-phenylpropyl)oxy]morphinan-6-on Hydrochlorid. Fp. 200-230 °C; MS (Cl): 460,3 (M⁺+1); IR (KBr): 1725 (C=O) cm⁻¹; ¹H-NMR (DMSO-d₆, δ in ppm): 9,52 (s, OH), 8,20 (s, breit, HN⁺), 7,30-7,18 (m, 5 arom. H), 6,71 (d, 1 arom. H, J = 8,0 Hz), 6,64 (d, 1 arom. H, J = 8,0 Hz), 4,89 (s, CH-5).

Eine Lösung von 17-Cyclopropylmethyl-4,5α-epoxy-3-hydroxy-14β-[(3-phenylpropyl)oxy]morphinan-6-on (0,54 g, 1,17 mmol) und Methyliodid (0,83 g, 5,84 mmol) in 10 ml wasserfreiem Acetonitril wurde unter N₂ bei 40 °C (Badtemperatur) 5 Tage gerührt und dann eingedampft. Der Rückstand (0,72 g braunes Öl) wurde mittels Säulenchromatographie (Kieselgel; CH₂Cl₂ / MeOH 250 : 4) gereinigt. Es wurden zwei verschiedene Produkte isoliert:
Verbindung **48**: Ausbeute 0,21 g (30%) farblose Kristalle. Fp. 175-176 °C; MS (Cl): 474 (M⁺); IR (KBr): 1726 (C=O) cm⁻¹; ¹H-NMR (OMSO-d₆, δ in ppm): 9,53 (s, breit, OH), 7,31-7,17 (m, 5 arom. H), 6,69 (s, 2 arom. H), 4,87 (s, CH-5), 3,53 (s, CH₃N⁺, axial).
Verbindung **49**: Ausbeute 0,03 g (4%) hellorange Kristalle. Fp. 133-136 °C; MS (Cl): 488 (M⁺); IR (KBr): 1725 (C=O) cm⁻¹; ¹H-NMR (DMSO-d₈, δ in ppm): 7,31-7;19 (m, 5 arom. H), 6,89 (d, 1 arom. H, J = 8,4 Hz), 6,82 (d, 1 arom. H, J = 8,4 Hz), 4,94 (s, CH-5), 3,81 (s, C3-OCH₃), 3,55 (s, CH₃N⁺, axial).

### Beispiel 47

### Synthese des (17R)-17-Cyclopropylmethyl-4,5α-epoxy-3-hydroxy-17-Methyl-6-oxo-14β-[(2-Phenylbenzyl)oxy]morphinaniumiodids (Verbindung 50)

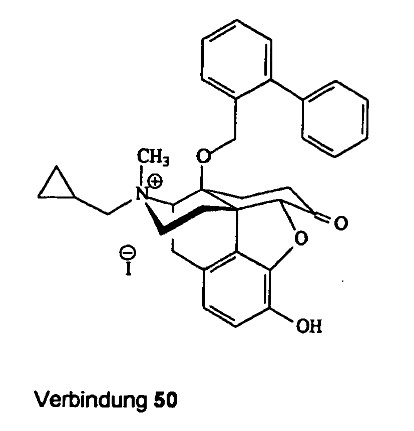

NaH (1,51 g, 62,9 mmol) (erhalten aus 2,51 g 60% NaH-Dispersion in Öl durch Waschen mit Petrolether) wurde einer Lösung von 3-Benzyloxy-17-cyclopropylmethyl-4,5α-epoxy-14β-hydroxymorphinan-6-spiro-2'-(1,3-dioxolan) (s. Beispiel 46) (5,99 g, 12,6 mmol) in 75 ml wasserfreiem N,N-Dimethylformamid unter N₂ bei 0 °C (Badtemperatur) unter Rühren zugefügt. Nach 20 min wurde 2-Phenylbenzylbromid (4,04 g, 16,4 mmol) zugegeben und das Gemisch 20 min bei 0 °C (Badtemperatur), dann bei Raumtemperatur 2 h gerührt. Dann wurden Eisstückchen zugesetzt, bis keine Wasserstoffentwicklung mehr festzustellen war, das Gemisch wurde auf 200 ml Wasser gegossen, mit Dichlormethan (1 x 100 ml, 2 x 75ml) extrahiert, die vereinigten organischen Phasen mit Wasser (5 x 300ml) und gesättigter Natriumchloridlösung (1 x 100ml) gewaschen, über Natriumsulfat getrocknet und eingedampft. Vom Eindampfrückstand (7,90 g braunes Öl) wurden 3.52 g mittels Säulenchromatographie (Kieselgel; CH₂Cl₂ / MeOH / konz. NH₄OH 250 : 2.5 : 0,5) gereinigt Es resultierte ein gelbliches Öl (2,83 g), das in 15 ml Ether gelöst wurde und mit etherischer HCl in das Hydrochlorid überführt wurde. So wurden 2,20 g farblose Kristalle von 3-Benzyloxy-17-cyclopropylmethyl-4,5α-epoxy-14β-[(2-phenylbenzyl)oxy]morphinan-6-spiro-2'-(1,3-dioxolan) Hydrochlorid erhalten. Fp. 142-144 °C; MS (CI): 642 (M⁺+1); ¹H-NMR der gereinigten Base (DMSO-d₆): δ 7,70-7,25 (m, 14 arom. H), 6,76 (d, 1 arom. H, J = 8,4 Hz), 6,53 (d, 1 arom. H, J = 8,4 Hz), 5,10 (s, 2H, C3-OCH₂Ph), 4,63 (d, 1 H, C14-OCH₂, J = 10,6 Hz), 4,46 (s, CH-5), 4,34 (d, 1 H, C14-OCH₂, J =10,6 Hz), 4,10-3,69 (m, 4H, OCH₂CH₂O).

Eine Lösung von 3-Benzyloxy-17-cyclopropylmethyl-4,5α-epoxy-14β-[(2-phenylbenzyl)oxy]morphinan-6-spiro-2'-(1,3-dioxolan) Hydrochlorid (2,10 g, 3,10 mmol) in 40 ml eines Gemisches aus konz. HCl/MeOH (4:9) wurde 1,5 h lang rückflußerhitzt. Nach dem Abkühlen wurde das Gemisch auf 100 ml Eis gegossen, mit konz. NH₄OH alkalisiert und mit Dichlormethan (1 x 100 ml, 4 x 75ml) extrahiert, die vereinigten organischen Phasen mit Wasser (2 x 150ml) und gesättigter Natriumchloridlösung (1 x 100ml) gewaschen, über Natriumsulfat getrocknet und eingedampft. Es wurde ein gelbes Öl (1,90 g) erhalten, das mittels Säulenchromatographie (Kieselgel; CH₂Cl₂/ MeOH / konz. NH₄OH (250 : 3 : 0,5)) gereinigt wurde. Es resultierte ein gelbliches Öl (0,7 g, 42%), von dem 0,16 g in 5 ml Ether gelöst wurden und mit etherischer HCl in das Hydrochlorid überführt wurden. Es wurden 0,12 g farblose Kristalle von 17-Cyclopropylmethyl-4,5α-epoxy-3-hydroxy-14ββ-[(2-phenylbenzyl)oxy]morphinan-6-on Hydrochlorid isoliert. Fp. 181-186 °C; MS (Cl): 508 (M⁺+1); IR (KBr): 1725 (C=O) cm⁻¹; ¹H-NMR (DMSO-d₆, δ in ppm): 9,58 (s, OH), 8,33 (s, breit, HN⁺), 7,95-7,25 (m, 9 arom. H), 6,72 (d, 1 arom. H, J = 8,0 Hz), 6,64 (d, 1 arom. H, J = 8,0 Hz), 5,14 (s, CH-5), 4,84 (d, 1 H, C14-OCH₂-R, J =12,4 Hz), 4,56 (d, 1 H, C14-OCH₂-R, J =12,4 Hz).

Eine Lösung von 17-Cyclopropylmethyl-4,5α-epoxy-3-hydroxy-14β-[(2-phenylbenzyl)oxy]morphinan-6-on (0,39 g, 0,76 mmol) und Methyliodid (0,54 g, 3,79 mmol) in 6 ml wasserfreiem Acetonitril wurde unter N₂ bei 40 °C (Badtemperatur) 2 Tage gerührt und dann eingedampft. Der Rückstand (0,5 g braunes Öl) wurde mittels Säulenchromatographie (Kieselgel; CH₂Cl₂ / MeOH 250 : 4) gereinigt Ausbeute 0,02 g (4%) beige Kristalle der Verbindung **50**. Fp. 219-221 °C; MS (Cl): 522 (M⁺); IR (KBr): 1725 (C=O) cm⁻¹; ¹H-NMR (DMSO-d₆): δ 9,53 (s, OH), 7,68-7,25 (m, 9 arom. H), 6,67 (s, 2 arom. H), 4,73 (d, C14-OCH₂), 4,61 (s, CH-5), 3,26 (s, CH₃N⁺, axial).

### Beispiel 48

### Synthese des (17R)-14β-[(4-Chlorbenzyl)oxy]-17-cyclopropylmethyl-4,5α-epoxy-3-hydroxy-17-methyl-6-oxomorphinaniumiodids (Verbindung 51)

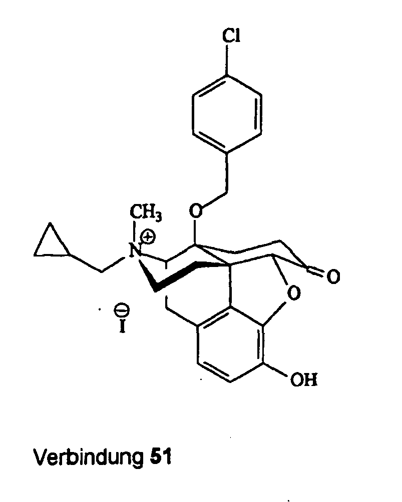

NaH (1,28 g, 53,3 mmol) (erhalten aus 2,13 g 60% NaH-Dispersion in Öl durch Waschen mit Petrolether) wurde einer Lösung von 3-(Benzyloxy)-1 7-(cyclopropylmethyl)-4,5α-epoxy-14β-hydroxymorphinan-6-spiro-2'-(1,3-dioxolan) (s. Beispiel 48) (5,00 g, 10,6 mmol) in 75 ml wasserfreiem N,N-Dimethylformamid unter N₂ bei 0 °C (Badtemperatur) unter Rühren zugefügt. Nach 20 min wurde 4-Chlorbenzylbromid (2,95 g, 14,3 mmol) zugegeben und das Gemisch zunächst 30 min bei 0 °C (Badtemperatur), dann bei Raumtemperatur 1,5 Stunden gerührt. Dann wurden Eisstückchen zugesetzt, bis keine Wasserstoffentwicklung mehr festzustellen war, das Gemisch auf 400 ml Wasser gegossen, mit Dichlormethan (3 x 100 ml) extrahiert, die vereinigten organischen Phasen mit Wasser (5 x 400 ml) und gesättigter Natriumchloridlösung (1 x 100 ml) gewaschen, über Natriumsulfat getrocknet und eingedampft. Der Eindampfrückstand (6,45 g gelbes Öl) wurde aus Methanol kristallisiert. Ausbeute 4,72 g (75%) farblose Kristalle von 3-Benzyloxy-14β-[(4-Chlorbenzyl)oxy]-17-cyclopropylmethyl-4,5α-epoxymorphinan-6-spiro-2'-(1,3-dioxolan). Fp. 122-125 °C; MS (CI): 600 (M⁺+1); ¹H-NMR (DMSO-d₆, δ in ppm): 7,53-7,35 (m, 9 arom. H), 6,78 (d, 1 arom. H, J = 8,4 Hz), 6,57 (d, 1 arom. H, J = 8,4 Hz), 5,11 (d, 2 H, C₃-OCH₂Ph), 4,65 (d, 1 H, C₁₄-OCH₂, J =11,2 Hz), 4,23 (s, CH-5), 4,30 (d, 1 H. C14-OCH₂, J = 11,2 Hz), 4,10-3,70 (m, 4H, OCH₂CH₂O).

Eine Lösung von 3-Benzyloxy-14β-[(4-Chtorbenzyl)oxy]-17-(cyclopropylmethyl)-4.5α-epoxymorphinan-6-spiro-2'-(1,3-dioxolan) (4,60 g, 7,66 mmol) in 100 ml eines Gemisches aus HCl konz./MeOH (4:9) wurde 1,5 h lang rückflußerhitzt. Nach dem Abkühlen wurde das Reaktionsgemisch auf 300 ml Eis gegossen, mit konz. NH₄OH alkalisiert, mit Dichlormethan (3 x 150 ml) extrahiert, die vereinigten organischen Phasen mit Wasser (2 x 150ml) und gesättigter Natriumchloridlösung (1 x 100ml) gewaschen, über Natriumsulfat getrocknet und eingedampft. Der Eindampfrückstand (4,86 g gelbes Öl) wurde mittels Säulenchromatographie (Kieselgel; CH₂Cl₂ / MeOH / konz. NH₄OH 250 : 3 : 0,5) gereinigt. Es resultierte ein gelbliches Öl (1,18 g, 33%), von dem 0,26 g in 5 ml Ether gelöst wurden und mit etherischer HCl in das Hydrochlorid überführt wurden. Es wurden 0,23 g farblose Kristalle von 14β-[(4-Chlorbenzyl)oxy]-17-cyclopropylmethyl-4,5α-epoxy-3-hydroxymorphinan-6-on Hydrochlorid isoliert. Fp. 195-200 °C; MS (Cl): 466,1 (M⁺+1); IR (KBr): 1725 (C=O) cm⁻¹; ¹H-NMR (DMSO-d₆, δ in ppm): 9,59 (s, OH), 8,63 (s, breit, HN⁺), 7,61 (d, 2 arom. H, C14-OCH₂Ph-Cl, J = 8,4 Hz), 7,47 (d, 2 arom. H, C14-OCH₂Ph-Cl, J = 8,4 Hz), 6,73 (d, 1 arom. H, J = 8,2 Hz), 6,68 (d, 1 arom. H, J = 8,0 Hz), 5,08 (s, CH-5), 4,80 (d, 1 H, C14-OCH₂, J = 12 Hz), 4,72 (d, 1H, C14-OCH₂, J =12 Hz).

Eine Lösung von 14β-[(4-Chlorbenzyl)oxy]-17-cyclopropylmethyl-4,5α-epoxy-3-hydroxymorphinan-6-on (0,50 g, 1,07 mmol) und Methyliodid (0,76 g, 5,37 mmol) in 8 ml wasserfreiem Acetonitril wurde unter N₂ bei 40 °C (Badtemperatur) 6 Tage gerührt und dann eingedampft. Der Rückstand (0,56 g braunes Öl) wurde mittels Säulenchromatographie (Kieselgel; CH₂Cl₂ / MeOH 250 : 4) gereinigt. Ausbeute 0,07 g (11%) beige Kristalle der Verbindung **51**. Fp. 214-219 °C; MS (Cl): 480 (M⁺); IR (KBr): 1733 (C=O) cm⁻¹; ¹H-NMR (DMSO-d₆, δ in ppm): 9,57 (s, OH). 7,57 (d, 2 arom.H, C14-OCH₂Ph-Cl, J = 8,6 Hz), 7,48 (d, 2 arom.H, C14-OCH₂Ph-Cl, J = 8,6 Hz), 6,71 (s, 2 arom. H), 5,10 (s, CH-5), 4,86 (d, 1H, C14-OCH₂, J= 11,4 Hz); 4,75-4,66 (m, 3H, C14-OCH₂ und CH-9), 3,44 (s, CH₃N⁺, axial).

### Beispiel 49

### Synthese des 17(R)-4,5α-Epoxy-3-hydroxy-14β-methoxy-17-methyl-6-oxo-17-(2-phenylethyl)morphinaniumiodids (Verbindung 52)

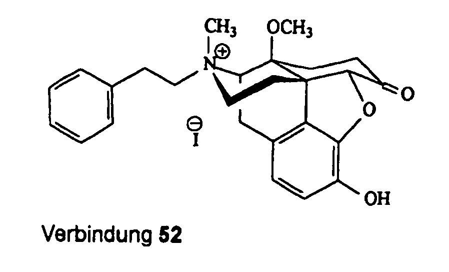

Ein Gemisch aus 4,5α-Epoxy-3,14β-dimethoxymorphinan-6-on (Kobylecki et al., J. Med. Chem., 1982, 25, 2, S.116-120) (1,44 g, 4,57 mmol), Kaliumcarbonat (4,11 g, 29,7 mmol), 2-Phenylethylbromid (1,1 g, 5,94 mmol) und 20 ml N,N-Dimethylformamid wurde 5,5 h bei 80 °C (Badtemperatur) unter N₂ gerührt. Dann wurde 200 ml Wasser zugesetzt und mit Dichlormethan (4 x 50ml) extrahiert. Die vereinigten organischen Phasen wurden mit Wasser (6 x 130ml) gewaschen, über Natriumsulfat getrocknet und eingedampft. Ausbeute 1,95 g gelbliches Öl von 4,5α-Epoxy-3,14β-dimethoxy-17-(2-phenylethyl)morphinan-6-on, welches unverändert für den nächsten Syntheseschritt eingesetzt wurde. Analytische Daten: ¹H-NMR (CDCl₃, δ in ppm): 7,30-7,18 (m, 5 arom. H), 6,69 (d, 1 arom. H, J = 8,0 Hz), 6,60 (d, 1 arom. H, J = 8,0 Hz), 4,64 (s, CH-5), 3,89 (s, C3-OCH₃), 3,18 (s, C14- OCH₃).

Eine Lösung von 4,5α-Epoxy-3,14β-dimethoxy-17-(2-phenylethyl)morphinan-6-on (2,4 g, 5,72 mmol) in 25 ml 48% HBr wurde 13 min rückflußerhitzt. Danach wurde die Lösung abgekühlt, auf 100 ml Eis gegossen, mit konz. NH₄OH alkalisiert und mit Dichlormethan (5 x 50 ml) extrahiert. Die vereinigten organischen Phasen wurden mit gesättigter Natriumchloridlösung (3 x 150 ml) gewaschen, über Natriumsulfat getrocknet und eingedampft. Der Rückstand (2,40 g braunes Öl) wurde mittels Säulenchromatographie (Kieselgel; CH₂Cl₂ / MeOH / konz. NH₄OH 250 : 2 : 0,5) gereinigt. Ausbeute 0,4 g (17%) dunkelgelbes Öl, von dem 0,04 g in 5 ml Ether gelöst wurden und mit etherischer HCl das Hydrochlorid erhalten wurde. Es wurden 0,02 g farblose Kristalle von 4,5α-Epoxy-3-hydroxy-14β-methoxy-17-(2-phenylethyl)morphinan-6-on Hydrochlorid isoliert. Fp. 259-262 °C; MS (Cl): 406 (M⁺+1); IR (KBr): 1731 (C=O) cm⁻¹: ¹H-NMR (DMSO-d₆, δ in ppm): 9,52 (s, OH), 9,28 (s, breit, HN⁺), 7,38-7,25 (m, 5 arom. H), 6,70 (s, 2 arom. H, CH-1 und CH-2), 4,96 (s, CH-5), 3,40 (s, C14-OCH₃).

Eine Lösung von 4,5α-Epoxy-3-hydroxy-14β-methoxy-17-(2-phenylethyl)morphinan-6-on (0,32 g, 0,78 mmol) und Methyliodid (0,56 g, 3,92 mmol) in 6 ml wasserfreiem Acetonitril wurde unter N₂ bei 40 °C (Badtemperatur) 2 Tage gerührt und dann eingedampft. Der Rückstand (0,32 g braunes Öl) wurde mittels Säulenchromatographie (Kieselgel; CH₂Cl₂ / MeOH 250 : 2) gereinigt. Ausbeute 0,07 g (16%) farblose Kristalle der Verbindung **52**. Fp. 180-181 °C; MS (Cl): 420 (M⁺+1); IR (KBr): 1727 (C=O) cm⁻¹; ¹H-NMR (D₂O, δ in ppm): 7,17-7,14 (m, 5 arom. H), 6,56 (s, 2 arom. H), 5,18 (s, CH-5), 3,64 (s, CH₃N⁺, axial), 3,44 (s, OCH₃).

### Beispiel 50

### 4,5α-Epoxy-17-methyl-14β-[(3-phenylpropyl)oxy]morphinan-6-on (Verbindung 53)

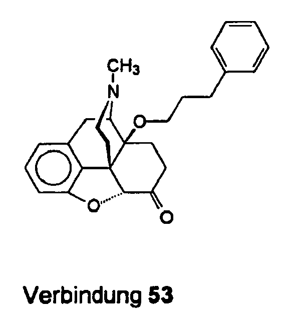

Zu einer Lösung von 4,5α-Epoxy-3-hydroxy-17-methyl-14β-[(3-phenylpropyl)oxy]morphinan-6-on (s. Beispiel 36) (6,38 g, 15,20 mmol) in 40 ml wasserfreiem Dimethylformamid wurde wasserfreies K₂CO₃ (5,67 g, 41,06 mmol) und 5-Chlor-1-phenyl-1H-tetrazol (3,02 g, 16,73 mmol) zugegeben und dieses Gemisch 23 h bei Raumtemperatur gerührt. Nach Zugabe von 300 ml H₂O wurde mit CH₂Cl₂ (3x75 ml) extrahiert. Die vereinigten organischen Phasen wurden mit H₂O (2x200 ml) und gesättigter NaCl-Lösung (3x250 ml) gewaschen, über Na₂SO₄ getrocknet und eingedampft. Der Eindampfrückstand (7,48 g gebliches Öl) wurde säulenchromatographisch gereinigt (Kieselgel; CH₂Cl₂ / MeOH / konz. NH₄OH (250 : 2 : 0,5)). Die geeigneten Fraktionen wurden eingedampft. Es resultierte 6,41 g gelbes Öl (4,5α-Epoxy-17-methyl-14β-[(3-phenylpropyl)oxy)-3-[(1-phenyl-1 H-tetrazol-5-yl)oxy]morphinan-6-on; 75%), das ohne weitere Reinigung für den nächsten Reaktionsschritt verwendet wurde. Für analytische Zwecke wurde eine kleine Menge aus Ether kristallisiert und so 4,5α-Epoxy-17-methyl-14β-[(3-phenylpropyl)oxy]-3-[(1-phenyl-1 H-tetrazol-5-yl)oxy]morphinan-6-on als farblose Kristalle erhalten. Fp. 117-119 °C; MS (Cl): 564 (M⁺+1); IR (KBr): 1723 (C=O) cm⁻¹; ¹H-NMR (DMSO-d₆): δ 7,88-7,837 (m, 2 arom. H), 7,73-7,62 (m, 3 arom. H), 7,30-7,16 (m, 6 arom. H), 6,85 (d, J = 8,4 Hz, 1 arom. H), 4,93 (s, CH-5), 2.29 (s, 3H , N17-CH₃).

Ein Gemisch aus 4,5α-Epoxy-17-methyl-14β-[(3-phenylpropyl)oxy]-3-[(1-phenyl-1 H-tetrazol-5-yl)oxy]morphinan-6-on (6,20 g, 11,00 mmol), 10%-igem Pd/C-Katalysator (2,48 g) und 100 ml Eisessig wurde bei 50 psi und 40 °C 17 h lang hydriert. Nach Abfiltrieren vom Katalysator wurde das Filtrat auf ein Volumen von 30 ml eingeengt, 150 ml Eis/Wasser-Gemisch zugesetzt und nach Alkalisierung mit konz. NH₄OH mit CH₂Cl₂ (1x100 ml, 3x75 ml) extrahiert. Die vereinigten organischen Phasen wurden mit gesättigter NaCl-Lösung (4x200 ml) gewaschen, über Na₂SO₄ getrocknet und eingedampft. Der Eindampfrückstand (4,15 g gelbliches Öl, DC-rein) wurde aus wenig Methanol kristallisiert und so 3,20 g (72%) der Verbindung 53 als farblose Kristalle erhalten. Fp.105-109 °C; MS (Cl): 404 (M⁺+1); IR (KBr): 1721 (C=O) cm⁻¹; ¹H-NMR (DMSO-d₆): δ 7,29-7,16 (m, 5 arom. H), 7,04 (t, J = 8,0 Hz, 1 arom. H), 6,73 (d, J = 8,0 Hz, 1 arom. H), 6,64 (d, J = 8,0 Hz, 1 arom. H), 4,75 (s, 1H, C5-H), 2,28 (s, 3H, N17-CH₃)

### Beispiel 51

### 17-(Cyclopropylmethyl)-4,5α-epoxy-14β-[(3-phenylpropyl)oxy]morphinan-6-on Hydrochlorid (Verbindung 54.HCl)

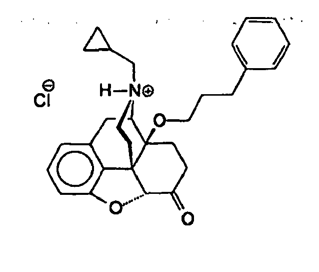

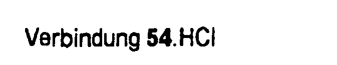

Zu einer Lösung von 53 (3,20 g, 7,93 mmol) in 40 ml ethanolfreiem 1,2-Dichlorethan wurde unter N₂ NaHCO₃ (3,33 g, 39,65 mmol) und 1-Chlorethylchloroformiat (4,32 ml, 39,65 mmol) zugegeben. Dieses Gemisch wurde bei 60 °C (Ölbadtemperatur) unter N₂13,5 h lang gerührt. Dann wurde vom anorganischen Rückstand abfiltriert, der Rückstand mit ethanolfreiem 1,2-Dichlorethan gewaschen und das Filtrat eingedampft. Der Eindampfrückstand (3,7 g gelbbraunes Öl, DC-rein) wurde - ohne weiter gereinigt und charakterisiert zu werden -1 h lang in einem Gemisch aus 15 ml konz. HCl und 35 ml MeOH rückflußerhitzt. Die Reaktionslösung wurde eingedampft und der Eindampfrückstand aus MeOH/Et₂O kristallisiert. Es wurden 2,77 g (82%) 4,5α-Epoxy-14β-[(3-phenylpropyl)oxy]morphinan-6-on Hydrochlorid isoliert. Fp. >225 °C (Zersetzung); MS (Cl): m/z 390 (M⁺+1); IR (KBr): 1729 (C=O) cm⁻¹; ¹H-NMR (DMSO-d₆): δ 9,25 (s, breit, 1 H, ⁺HN), 8,11 (s, breit, 1 H, ⁺HN), 7,34-7,12 (m, 5 arom. H), 7,16 (t, J = 8,0 Hz, 1 arom. H), 6,85 (d, J = 8,0 Hz, 1 arom. H), 6,77 (d, J = 8,0 Hz, 1 arom. H), 4,96 (s, 1H, C5-H), 4,28 (ps-d, C9-H).

Zu einer Lösung von 4,5α-Epoxy-14β-[(3-phenylpropyl)oxy]morphinan-6-on Hydrochlorid (2,36 g, 5,55 mmol) in 20 ml wasserfreiem Dimethylformamid wurde unter N₂ wasserfreies K₂CO₃ (2,13 g, 15,40 mmol) und Cyclopropylmethylbromid (0,64 ml, 6,67 mmol) zugegeben. Dieses Gemisch wurde bei einer Badtemperatur von 80°C unter N₂ gerührt. Da eine DC-Kontrolle nach 7 h zeigte, dass die Reaktion noch nicht vollständig abgelaufen war, wurden nochmals 0,50 ml Cyclopropylmethylbromid (5,24 mmol) zugegeben. Nach einer weiteren Stunde war die Reaktion beendet, und es wurde nach dem Abkühlen vom anorganischen Material abfiltriert, der Rückstand mit CH₂Cl₂ gewaschen und das Filtrat eingedampft. Der Eindampfrückstand wurde in 150 ml CH₂Cl₂ gelöst, mit H₂O (5x150 ml) und mit gesättigter NaCl-Lösung (150 ml) gewaschen, über Na₂SO₄ getrocknet und eingedampft. Es resultierten 2,43 g (99%) 17-(Cyclopropylmethyl)-4,5α-epoxy-14β-[(3-phenylpropyl)oxy]morphinan-6-on (**54**) als gelbliches Öl, das für die nächste Reaktion ohne weitere Reinigung verwendet wurde. 0,30 g des Öls wurden in Ether gelöst und nach Zugabe von etherischer HCl wurden 0,23 g 17-(Cyclopropylmethyl)-4,5α-epoxy-14β-[(3-phenylpropyl)oxy]morphinan-6-on Hydrochlorid (Verbindung **54.**HCl) als farbloses Pulver isoliert. Fp. 145-150 °C; MS (CI): m/z 4.44 (M⁺+1); IR (KBr): 1726 (C=O) cm⁻¹;¹H-NMR (DMSO-d₆): δ 8.26 (s, breit, 1 H), 7.31-7.14 (m, 5 arom. H), 7.18 (t, J = 8,0 Hz, 1 arom. H), 6.85 (d, J = 8,0 Hz, 1 arom. H), 6.78 (d, J = 8,0 Hz, 1 arom. H), 4.96 (s, 1H, C5-H), 4.55 (ps-d, C9-H), 1.09-1.06 (m, 1 H, N17-CH₂CH(CH₂)₂), 0.75-0.69 (m, 2H, N-CH₂CH(CH₂)₂), 0.55-0.42 (m, 2H, N-CH₂CH(CH₂)₂).

### Beispiel 52

### 17-(Cyclopropylmethyl)-4-hydroxy-14β-[(3-phenylpropyl)oxy]morphinan-6-on Hydrochlorid (Verbindung 55·HCl)

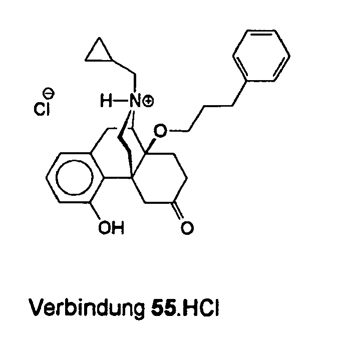

Zu einer rückflusskochenden Lösung von **54** (1,2 g, 2,71 mmol) in 50 ml MeOH wurde unter Rühren NH₄Cl (0,72 g, 13,46 mmol) zugegeben. Zu diesem Gemisch wurden in kleinen Portionen 0,88 g (13,46 mmol) aktivierter Zinkstaub gegeben. Dieses Gemisch wurde 80 min unter Rückfluß gerührt, dann auf Raumtemperatur abgekühlt und vom anorganischen Rückstand abfiltriert, der anorganische Rückstand mit heißem Methanol gewaschen und das Filtrat eingedampft. Der Eindampfrückstand wurde mit 50 ml H₂O versetzt und mit konz. NH₄OH alkalisiert. Dieses Gemisch wurde mit CH₂Cl₂ (3x80 ml) extrahiert, die vereinigten organischen Phasen mit gesättigter NaCI-Lösung (5x100 ml) gewaschen, über Na₂SO₄ getrocknet und eingedampft. Es wurden 1,20 g (99%) 17-(Cyclopropylmethyl)-4-hydroxy-14β-[(3-phenylpropyl)oxy]morphinan-6-on (**55**) als gelbliches Öl erhalten, welches ohne weitere Reinigung für den nächsten Reakionsschritt verwendet wurde. Für analytische Zwecke wurden 0.08 g des Öls in Ether gelöst und nach Zugabe von etherischer HCl wurden 0.059 g 17-(Cyclopropylmethyl)-4-hydroxy-14β-[(3-phenylpropyl)oxy]morphinan-6-on Hydrochlorid (**55**·HCl) als farbloses Pulver isoliert. Fp. >170°C (Zersetzung); MS (Cl): m/z 446 (M⁺+1); IR (KBr): 1709 (C=O) cm⁻¹; ¹H-NMR (DMSO-d₆): δ 9,82 (s, 1H, C4-OH), 8,43 (s, breit, 1H, ⁺N17-H), 7,30-7,20 (m, 5 arom. H, C14-0(CH₂)₃Ph), 7,03 (t, J = 8,0 Hz, 1 arom. H, C2-H), 6,66 (m, 2 arom. H), 4,40 (ps-d, C9-H), 1,09 (m, 1H. N17-CH₂CH(CH₂)₂), 0,70-0,67 (m, 2H, N17-CH₂CH(CH₂)₂), 0,52-0,40 (m, 2H, N17-CH₂CH(CH₂)₂).

### Beispiel 53

### 17-(Cyclopropylmethyl)-4-methoxy-14β-[(3-phenylpropyl)oxy]morphinan-6-on Hydrochlorid (56.HCl)

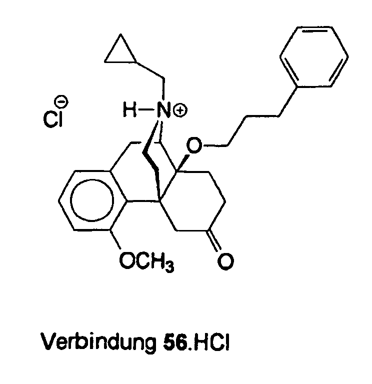

Zu einer Lösung von **55** (0,40 g, 0,90 mmol) in 6 ml wasserfreiem Dimethylformamid wurde unter N₂ wasserfreies K₂CO₃ (0,37 g, 2,69 mmol) und 0,46 g (2,69 mmol) Phenyltrimethylammoniumchlorid zugegeben. Dieses Gemisch wurde bei 80 °C Badtemperatur unter N₂ 3,5 h gerührt. Nach dem Abkühlen wurde vom anorganischen Material abfiltriert, der Rückstand mit CH₂Cl₂ gewaschen und das Filtrat eingedampft. Der Eindampfrückstand wurde in 100 ml CH₂Cl₂ gelöst, mit gesättigter NaCl-Lösung (4x150 ml) gewaschen, mit Na₂SO₄ getrocknet und eingedampft. Der Eindampfrückstand (0,30 g) wurde säulenchromatographisch gereinigt (Kieselgel; CH₂Cl₂/MeOH/konz. NH₄OH (250/4/0,5)) und die geeigneten Fraktionen eingedampft. Der Eindampfrückstand (0.23 g) wurde in Ether gelöst und nach Zugabe von etherischer HCl wurden 0.22 g (48%) der Verbindung **56**.HCl als farbloses Pulver isoliert. Fp. 121-126 °C; MS (Cl): m/z 460 (M⁺+1); IR (KBr): (C=O) 1710 cm⁻¹; ¹H-NMR (DMSO-d₆): δ 8,34 (s, breit, 1H, ⁺N-H), 7,30-7,16 (m, 5 arom. H), 7,22 (t, J = 8,0 Hz, 1 arom. H), 6,88 (d, J = 8,0 Hz, 1 arom. H), 6,82 (d, J = 8,0 Hz, 1 arom. H), 4,42 (ps-d, C9-H), 3,77 (s, 3 H, C4-OCH₃), 1,08 (m, 1H, N-CH₂CH(CH₂)₂), 0.70-0.42 (m, 4H, N-CH₂CH(CH₂)₂).

### Beispiel 54

### 4-(n-Butoxy)-17-(cyclopropylmethyl)-14β-[(3-phenylpropyl)oxy]morphinan-6-on Hydrochlorid (57·HCl)

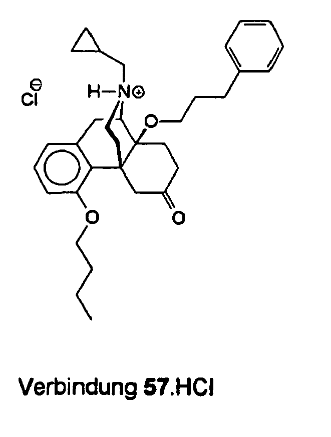

Zu einer Lösung von **55** (0,60 g, 1,34 mmol) in 5 ml wasserfreiem Dimethylformamid wurde unter N₂ wasserfreies K₂CO₃ (0,55 g, 4,01 mmol) und lodbutan (0,31 ml, 2,67 mmol) zugegeben. Dieses Gemisch wurde bei 90 °C Badtemperatur unter N₂7 h gerührt. Daraufhin wurde vom anorganischen Material abfiltriert, der Rückstand mit CH₂Cl₂ gewaschen und das Filtrat eingedampft. Der Eindampfrückstand wurde in 100 ml CH₂Cl₂ gelöst, mit gesättigter NaCl-Lösung (3x150 ml) gewaschen, über Na₂SO₄ getrocknet und eingedampft. Der Eindampfrückstand (0,43 g braunes Öl) wurde säulenchromatographisch gereinigt (Kieselgel; CH₂Cl₂MeOH/konz. NH₄OH (250/4/0,5)) und die geeigneten Fraktionen eingedampft. Der Eindampfrückstand (0,26 g) wurde in Ether gelöst und nach Zugabe von etherischer HCl wurden 0,19 g (26%) **57**.HCl als farbloses Pulver isoliert. Fp. 172-177 °C; MS (Cl): m/z 502 (M⁺+1); IR (KBr): (C=0) 1711 cm⁻¹; ¹H-NMR (DMSO-d₆): δ 8,52 (s, breit, 1H, ⁺N-H), 7,30-7,15 (m, 6 arom. H), 6,86 (d, J = 8,0 Hz, 1 arom. H), 6,80 (d, J = 8,0 Hz, 1 arom. H), 4,42 (ps-d, C9-H), 3,95 (t, 2H, J =6,4 Hz, C4-OCH₂ (CH₂)₂CH₃), 1,13-1,06 (m, 1H, N-CH₂CH(CH₂)₂), 0,95 (t, 3H, J = 7,3 Hz, C4-O(CH₂)₃CH₃), 0,69-0,40 (m, 4H, N-CH₂CH(CH₂)₂).

### Beispiel 55

### Opioid-Rezeptorbindungsstudien

Opioid-Rezeptorbindungsstudien wurden an Rattenhirn-Homogenisaten unter Verwendung von [³H]DAMGO (µ-Rezeptor-Agonist) als Radioligand und unter strikter Einhaltung einer früher publizierten Vorschrift durchgeführt (M. Spetea et al. Neurochemical Research 1998, Vol. 23, S. 1213-1218).

Die Verbindungen **8, 17, 17a, 17b, 38, 40-43, 48, 49** und **54-56** zeigen sehr hohe Affinität zu µ-Opioidrezeptoren, welche hauptsächlich verantwortlich sind für Analgesie (Tabelle 1). Alle haben deutlich höhere Affinität als der µ-opioidrezeptorselektive Antagonist Cyprodime (s. H. Schmidhammer et al. J. Med. Chem. 1995, 38, 3071-3077). Die Verbindungen **8, 17, 17a, 17b, 40, 48** und **54-56** zeigen extrem hohe Affinität zu µ-Opioidrezeptoren (im subnanomolaren Bereich). Morphin und der Opioidantagonist 3-Hydroxycyprodime (s. H. Schmidhammer et al. J. Med. Chem. 1995, 38, 3071-3077) zeigen im Vergleich zu diesen beiden Verbindungen deutlich geringere Affinität zu µ-Opioidrezeptoren, während 14-Methoxymetopon ähnliche Affinität aufweist.

**Tabelle 1. Opioid-Rezeptorbindungsstudien der Verbindungen 8, 17, 17a, 17b, 38, 40 - 43, 48, 49, 54-56, Morphin, 14-Methoxymetopon, Cyprodime und 3-Hydroxycyprodime**

| Verbindung | Kᵢ (nM) (µ-Rezeptoren) |
|---|---|
| **8** | 0, 34 |
| **17** | 0.73 |
| **17a** | 0.62 |
| **17b** | 0.20 |
| **38** | 4,11 |
| **40** | 0,37 |
| **41** | 2, 32 |
| **42** | 5,93 |
| **43** | 1,33 |
| **48** | 0,93 |
| **49** | 1,78 |
| **54** | 0,84 |
| **55** | 0,40 |
| **56** | 0,34 |
| Morphin | 6,5,5 |
| 14-Methoxymetopon | 0,55 |
| Cyprodime | 23,7 |
| 3-Hydroxycyprodime | 6,15 |

### Beispiel 56

### Analgesietests

### a) "Hot-plate Test"

Es wurde ein Test an Mäusen ("hot-ptate Test") wie früher beschrieben durchgeführt (E. L. May et al. J. Med. Chem. 2000; 43, 5030-5036).

Alle getesteten Substanzen zeigen wesentlich höhere analgetische Aktivität als Morphin. Die Verbindungen **6 - 11, 17a, 17b** und **53** zeigen extrem hohe analgetische Aktivität, die signifikant höher ist als die der Referenzsubstanz 14-Methoxymetopon (s. Zs. Fürst et al. Eur. J. Pharmacol. 1993; 236: 209-215). Verbindungen **4** und **32** zeigen im Vergleich zu den Verbindungen **6 - 11** etwas niedrigere analgetische Aktivität, da diese Verbindung in Position 3 eine Etherfunktion (Methoxygruppe) aufweisen, von der man weiß, dass sie die analgetische Aktivität im Vergleich zu den 3-Hydroxyanaloga senkt. Dennoch ist die analgetische Aktivität der Verbindungen **4** und **32** überraschend hoch. Auch die analgetische Aktivität der Verbindung **17** mit einer anderen Etherfunktion (Propargyloxygruppe) in Position 3 ist überraschend hoch.

**Tabelle 2. "Hot-plate Test" der Verbindungen 4, 6 -11, 17, 17a, 17b, 32, 53, Morphin und 14-Methoxymetopon**

| | |
|---|---|
| Verbindung | ED₅₀ (µg/kg, sc^{a}) |
| 4 | 60 |
| **6** | 5,9 |
| **7** | 3,7 |
| **8** | 2,3 |
| **9** | 11,7 |
| 10 | 1,3 |
| **11** | 1,7 |
| 17 | 38 |
| 17a | 2,6 |
| **17b** | 0,10 |
| 32 | 22 |
| 53 | 3,0 |
| Morphin | 850 |
| 14-Methoxymetopon | 30 |

| | |
|---|---|
| ^{a} sc = subkutane Applikation | |

### b) "Mouse Tail Flick Test"

Zur Bestimmung der analgetischen Aktivität von nicht-quarternisierten Verbindungen wurde der "Mouse Tail Flick Test" wie früher beschrieben durchgeführt (E. L. May et al. J. Med. Chem. 2000; 43, 5030-5036).

Alle getesteten Substanzen zeigen wesentlich höhere analgetische Aktivität als Morphin. Die Verbindungen **6 - 8,10, 11, 17a, 17b** und **53** zeigen extrem hohe analgetische Aktivität, die signifikant höher ist als die der Referenzsubstanz 14-Methoxymetopon (s. Zs. Fürst et al. Eur. J. Pharmacol. 1993; 236: 209-215). Verbindung **4** zeigt im Vergleich zu den Verbindungen **6 - 8, 10, 11, 17a, 17b** und **53** etwas niedrigere analgetische Aktivität, da diese Verbindungen in Position 3 eine Methoxygruppe aufweist, von der man weiß, dass sie die analgetische Aktivität im Vergleich zu den 3-Hydroxyanaloga senkt. Dennoch ist die analgetische Aktivität der Verbindung **4** überraschend hoch. Ebenfalls eine Methoxygruppe in Position 3 haben die Verbindungen **17a** und **32**, welche überraschenderweise eine sehr hohe analgetische Aktivität besitzen. Die aktivste Verbindung dieser Serie, Verbindung **17b**, ist mit einer ED₅₀ (µg/kg, sc)von 0,08 aktiver als die bisher als am aktivsten anerkannten analgetisch wirksamen Substanzen Etorphine und Dihydroetorphine (M. D. Aceto et al. Eur. J. Pharmacol 1997; 338, 215-223).

**Tabelle 3. "Mouse Tail Flick Test" der Verbindungen 4, 6 - 8,10,11, 17, 17a, 17b, 32, 53, Morphin und 14-Methoxymetopon**

| Verbindung | ED₅₀ (µg/kg, sc^{a}) |
|---|---|
| **4** | 84 |
| **6** | 5,6 |
| **7** | 8, 2 |
| **8** | 3,2 |
| | |
| **10** | 7,0 |
| **11** | 1,6 |
| **17** | 21 |
| **17a** | 4,4 |
| **17b** | 0,08 |
| **32** | 14 |
| **53** | 0,4 |
| Morphin | 1920 |
| 14-Methoxymetopon | 30 |

| | |
|---|---|
| ^{a} sc = subkutane Applikation | |

### c) "Rat Tail Flick Test"

Zur Bestimmung der analgetischen Aktivität von quartemisierten Verbindungen wurde ein Test an Ratten ("rat tail flick test") verwendet. Dieser Test wurde wie früher beschrieben durchgeführt (Zs. Fürst et al. Eur. J. Pharmacol. 1993; 236: 209-215).

Die Verbindungen **38** und **42** zeigen als quartäre Ammoniumsalze sehr hohe analgetische Wirkung - beide sind wesentlich aktiver als Morphin nach subkutaner Applikation (Tabelle 3). Verbindung **42** ist etwas weniger aktiv als 14-Methoxymetopon nach subkutaner Applikation. Nach intracerebroventrikulärer Applikation ist Verbindung **42** extrem aktiv, was darauf hinweist, dass es nur sehr begrenzt die Blut-Hirn-Schranke überwinden kann und seine Wirksamkeit daher in erster Linie in der Peripherie (außerhalb des Zentralnervensystems) zeigt. Das geht einher mit einer sehr verringerten Nebenwirkungsrate, was zentrale Nebenwirkungen wie z.B. Übelkeit, Erbrechen, Sedation, Benommenheit, Verwirrtheit, Atemdepression und Sucht betrifft.

**Tabelle 4. "Rat tail flick test" der Verbindungen 38, 42, Morphin und 14-Methoxymetopon**

| Verbindung | ED₅₀ (µg/kg, sc^{a}) | ED₅₀ (µg/rat^{b}, icv^{c}) |
|---|---|---|
| 38 | 120 | 12 |
| 42 | 16 | 0,22 |
| Morphin | 1900 | - |
| 14-Methoxymetopon | 7,2 | - |

| | | |
|---|---|---|
| ^{a} sc = subkutane Applikation. ^{b}Das Gewicht der verwendeten Ratten war jeweils 120 g. ^{c} icv = intracerebroventrikuläre Applikation. | | |

### Beispiel 57

### Bestimmung der antagonistischen Aktivität

### Vas Deferens-Präparation der Maus

Dieser Test wurde wie früher beschrieben unter Verwendung von DAMGO (µ-Rezeptor-Agonist) durchgeführt (S. Garadnay et al. Curr. Med. Chem. 2001, 8, 621-626).

Verbindung **48** zeigt überraschenderweise sehr hohe antagonistische Aktivität an µ-Opioidrezeptoren, was für quarternisierte Morphinane ungewöhnlich ist (Tabelle 4).

Normalerweise haben quarternisierte Morphinane eine wesentlich geringere Aktivität im Vergleich zu den nicht-quarternisierten Morphinanen. Es hat wesentlich höhere antagonistische Aktivität als der µ-selektive Opioidrezeptorantagonist Cyprodime. Im Vergleich zu Naloxon hat es etwas geringere Aktivität, während es im Vergleich zu 3-Hydroxycyprodime etwas höhere Aktivität aufweist. Aufgrund seiner Struktur als quartäres Ammoniumsalz kann es nur schwer die Blut-Hirn-Schranke überwinden und entfaltet seine Wirkung überwiegend in der Peripherie. Deswegen kann es z.B. verwendet werden, um opioidbedingter Obstipation oder Ileus entgegenzuwirken bzw. vorzubeugen, ohne dass die Analgesie des verabreichten Opioids aufgehoben wird. Im Vergleich dazu können Naloxone, Cyprodime und 3-Hydroxycyprodime die Blut-Hirn-Schranke relativ leicht passieren und daher auch die analgetische Wirkung von Opioiden aufheben.

**Tabelle 5. Antagonistische Kₑ-Werte der Verbindungen 48, Naloxone, Cyprodime und 3-Hydroxycyprodime in der Vas Deferens-Präparation der Maus**

| Verbindung | Kₑ(νM) (µ-Rezeptoren) |
|---|---|
| **48** | 2,94 |
| Naloxone | 1,4 |
| Cyprodime | 55,4 |
| 3-Hydroxycyprodime | 5,62 |

## Patentansprüche

1. Verbindungen der Formel (I) oder (la), 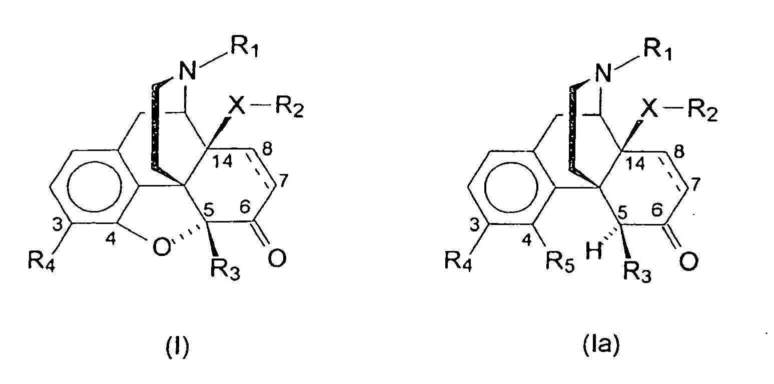 in der die Substituenten die folgende Bedeutung haben:
R₁: C₁-C₆-Alkyl; C₂-C₆-Alkenyl; C₂-C₆-Alkinyl-, C₃-C₁₆-(cyclische gesättigte Gruppe)alkyl, worin Alkyl C₁-C₆ ist; C₄-C₁₆-(cyclische gesättigte Gruppe)alkenyl, worin Alkenyl C₂-C₆ ist; C₄-C₁₆-(cyclische gesättigte Gruppe)alkinyl, worin Alkinyl C₂-C₆ ist; C₇-C₁₆-Arylalkyl, worin Aryl C₆-C₁₀-Aryl ist und Alkyl C₁-C₆-Alkyl ist; C₈-C₁₆-Arylalkenyl, worin Aryl C₆-C₁₀-Aryl und Alkenyl C₂-C₆-Alkenyl ist; C₈-C₁₆-Arylalkinyl, worin Aryl C₆-C₁₀-Aryl ist und Alkinyl C₂-C₆-Alkinyl;
R₂: C₄-C₆-Alkyl; C₂-C₆-Alkenyl, C₂-C₆-Alkinyl; C₃-C₁₆-(cyclische gesättigte Gruppe)alkyl, worin Alkyl C₁-C₆ ist; C₄-C₁₆-(cyclische gesättigte Gruppe)alkenyl, worin Alkenyl C₂-C₆ ist; C₄-C₁₆-(cyclische gesättigte Gruppe)alkinyl, worin Alkinyl C₂-C₆ ist; C₈-C₁₆-Arylalkyl, worin Aryl C₆-C₁₀-Aryl ist und Alkyl C₁-C₆-Alkyl ist; C₈-C₁₆-Arylalkenyl, worin Aryl C₆-C₁₀-Aryl und Alkenyl C₂-C₆-Alkenyl ist; C₈-C₁₆-Arylalkinyl, worin Aryl C₆-C₁₀-Aryl ist und Alkinyl C₂-C₆-Alkinyl; C₃-C₆-Alkenoyl; C₃-C₆-Alkinoyl; C₉-C₁₆-Arylalkenoyl, worin Aryl C₆-C₁₀-Aryl ist und Alkenoyl C₃-C₆-Alkenoyl ist; C₉-C₁₆-Arylalkinoyl, worin Aryl C₆-C₁₀-Aryl ist und Alkinoyl C₃-C₆-Alkinoyl ist;
R₃: Wasserstoff; C₁-C₆-Alkyl; C₂-C₆-Alkenyl-, C₇-C₁₆-Arylalkyl, worin Aryl C₆-C₁₀-Aryl ist und Alkyl C₁-C₆-Alkyi ist; C₈-C₁₆-Arylalkenyl, worin Aryl C₆-C₁₀-Aryl und Alkenyl C₂-C₆-Alkenyl ist; Alkoxyalkyl, worin Alkoxy C₁-C₆-Alkoxy und Alkyl C₁-C₆-Alkyl ist; CO₂(C₁-C₆-Alkyl); CO₂H; CH₂OH.
R₄: Wasserstoff; Hydroxy; C₁-C₆-Alkyloxy; C₂-C₁₀-Alkyloxyalkoxy, worin Alkyloxy C₁-C₄ ist und Alkoxy C₁-C₆-Alkyloxy ist; C₂-C₆-Alkenyloxy; C₂-C₆-Alkinyloxy; C₃-C₁₆-(cyclische gesättigte Gruppe)alkyloxy, worin Alkyl C₁-C₆ ist; C₄-C₁₆-(cyclische gesättigte Gruppe)alkenyloxy, worin Alkenyl C₂-C₆ ist; C₄-C₁₆-(cyclische gesättigte Gruppe)alkinyloxy, worin Alkinyl C₂-C₆ ist; C₇-C₁₆-Arylalkyloxy, worin Aryl C₆-C₁₀-Aryl ist und Alkyl C₁-C₆-Alkyl ist; C₈-C₁₆-Arylalkenyloxy, worin Aryl C₆-C₁₀-Aryl und Alkenyl C₂-C₆-Alkenyl ist; C₈-C₁₆-Arylalkinyloxy, worin Aryl C₆-C₁₀-Aryl ist und Alkinyl C₂-C₆-Alkinyl; C₁-C₆-Alkanoyloxy; C₃-C₆-Alkenoyloxy; C₃-C₆-Alkinoyloxy; C₇-C₁₆-Arylalkanoyloxy, worin Aryl C₆-C₁₀-Aryl ist und Alkanoyloxy C₂-C₆-Alkanoyloxy ist; C₉-C₁₆-Arylalkenoyloxy, worin Aryl C₆-C₁₀-Aryl ist und Alkenoyloxy C₃-C₆-Alkenoyloxy ist; C₉-C₁₆-Arylalkinoyloxy, worin Aryl C₆-C₁₀-Aryl ist und Alkinoyloxy C₃-C₆-Alkinoyloxy ist;
R_{5:} Wasserstoff; Hydroxy; C₁-C₆-Alkyloxy; C₂-C₁₀-Alkyloxyalkoxy, worin Alkyloxy C₁-C₄ ist und Alkoxy C₁-C₆-Alkyloxy ist; C₂-C₅-Alkenyloxy; C₂-C₆-Alkinyloxy; C₃-C₁₆-(cydische gesättigte Gruppe)alkyloxy, worin Alkyl C₁-C₆ ist; C₄-C₁₆-(cyclische gesättigte Gruppe)alkenyloxy, worin Alkenyl C₂-C₆ ist; C₄-C₁₆-(cyclische gesättigte Gruppe)alkinyloxy, worin Alkinyl C₂-C₆ ist; C₇-C₁₆-Arylalkyloxy, worin Aryl C₆-C₁₀-Aryl ist und Alkyl C₁-C₆-Alkyl ist; C₈-C₁₆-Arylalkenyloxy, worin Aryl C₆-C₁₀-Aryl und Alkenyl C₂-C₆-Alkenyl ist; C₈-C₁₆-Arylalkinyloxy, worin Aryl C₆-C₁₀-Aryl ist und Alkinyl C₂-C₆-Alkinyl; C₂-C₆-Alkanoyloxy; C₇-C₁₆-Arylalkanoyloxy, worin Aryl C₆-C₁₀-Aryl ist und Alkanoyloxy C₂-Cs-Alkanoyloxy ist;
X ist Sauerstoff;
wobei zwischen den Kohlenstoffatomen der Nummern 7 und 8 eine Einfach- oder eine Doppelbindung vorliegen kann,
wobei Alkyl, Alkenyl und Alkinyl jeweils verzweigt oder unverzweigt sein können, Aryl unsubstituiert oder mono-, di- oder trisubstituiert sein kann, jeweils unabhängig, mit Hydroxy, Halogen, Nitro, Cyano, Thiocyanato, Trifluormethyl, C₁-C₃-Alkyl, C₁-C₃-Alkoxy, CO₂H, CONH₂, CO₂(C₁-C₃-Alkyl), CONH(C₁-C₃-Alkyl), CON(C₁-C₃-Alkyl)₂, CO(C₁-C₃-Alkyl); amino; (C₁-C₃-Monoalkyl)amino, (C₁-C₃-Dialkyl)amino, C₅-C₆-Cycloalkylamino; (C₁-C₃-Alkanoyl)amido, SH, SO₃H, SO₃(C₁-C₃-Alkyl), SO₂(C₁-C₃-Alkyl), SO(C₁-C₃-Alkyl), C₁-C₃-Alkylthio oder C₁-C₃-Alkanoylthio,
wobei -(cyclische gesättigte Gruppe) entweder bevorzugt C₃-C₁₀-Cycloalkyl ist oder eine heterocyclische Gruppe mit 2 bis 9 Kohlenstoffatomen, enthaltend weiter ein oder mehrere Heteroatome,
**mit der Ausnahme von Verbindungen** worin R₁ Methyl ist, R₂ C₄-C₆-Alkyl ist, R₃ Wasserstoff oder Methyl ist, R₄ Hydroxy oder Methoxy ist und R₅ Hydroxy, Methoxy oder ein an das Kohlenstoffatom in 5-Stellung gebundenes Sauerstoffatom ist, wenn X Sauerstoff ist;
**mit der weiteren Ausnahme von Verbindungen** worin R₁ Cyclopropylmethyl und XR₂ Benzyloxy ist, wenn R₄ Wasserstoff oder Benzyloxy ist und R₅ ein an das Kohlenstoffatom in 5-Stellung gebundenes Sauerstoffatom ist;
**mit der weiteren Ausnahme von Verbindungen** worin R₁ Cyclopropylmethyl und XR₂ Benzyloxy ist, wenn R₄ Wasserstoff, Hydroxy oder Benzyloxy ist und R₅ Hydroxy oder Methoxy ist.

2. Verbindungen der Formel (IA) oder (IAa), 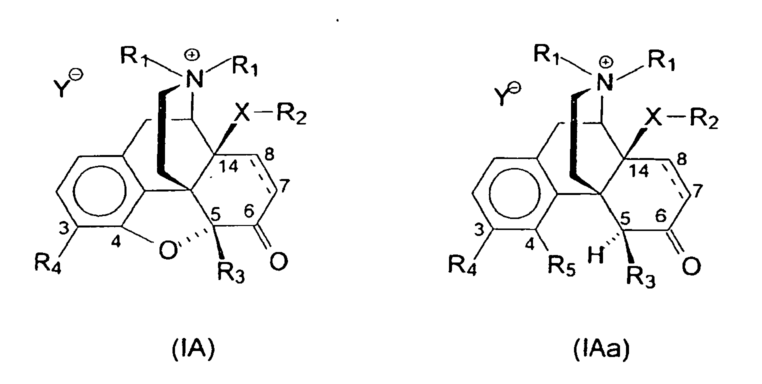 worin die Substituenten die folgende Bedeutung haben:
R₁: C₁-C₆-Alkyl; C₂-C₆-Alkenyl; C₂-C₆-Alkinyl; C₃-C₁₆-(cyclische gesättigte Gruppe)alkyl, worin Alkyl C₁-C₆ ist; C₄-C₁₆-(cyclische gesättigte Gruppe)alkenyl, worin Alkenyl C₂-C₆ ist; C₄-C₁₆-(cyclische gesättigte Gruppe)alkinyl, worin Alkinyl C₂-C₆ ist; C₇-C₁₆-Arylalkyl, worin Aryl C₆-C₁₀-Aryl ist und Alkyl C₁-C₆-Alkyl ist; C₈-C₁₆-Arylalkenyl, worin Aryl C₆-C₁₀-Aryl und Alkenyl C₂-C₆-Alkenyl ist; C₈-C₁₆-Arylalkinyl, worin Aryl C₆-C₁₀-Aryl ist und Alkinyl C₂-C₆-Alkinyl;
wobei die beiden Substituenten R₁ gleich oder verschieden sein können;
R₂: C₁-C₆-Alkyl; C₂-C₆-Alkenyl; C₂-C₆-Alkinyl; C₃-C₁₆-(cyclische gesättigte Gruppe)alkyl, worin Alkyl C₁-C₆ ist; C₄-C₁₆-(cyclische gesättigte Gruppe)alkenyl, worin Alkenyl C₂-C₆ ist; C₄-C₁₆-(cyclische gesättigte Gruppe)alkinyl, worin Alkinyl C₂-C₆ ist; C₈-C₁₆-Arylalkyl, worin Aryl C₆-C₁₀-Aryl ist und Alkyl C₁-C₆-Alkyl ist; C₈-C₁₆-Arylalkenyl, worin Aryl C₆-C₁₀-Aryl und Alkenyl C₂-C₆-Alkenyl ist; C₈-C₁₆-Arylalkinyl, worin Aryl C₆-C₁₀-Aryl ist und Alkinyl C₂-C₆-Alkinyl; C₃-C₆-Alkenoyl; C₃-C₆-Alkinoyl; C₉-C₁₆-Arylalkenoyl, worin Aryl C₆-C₁₀-Aryl ist und Alkenoyl C₃-C₆-Alkenoyl ist; C₉-C₁₆-Arylalkinoyl, worin Aryl C₆-C₁₀-Aryl ist und Alkinoyl C₃-C₆-Alkinoyl ist;
R₃: Wasserstoff; C₁-C₆-Alkyl; C₂-C₆-Alkenyl; C₇-C₁₆-Arylalkyl, worin Aryl C₆-C₁₀-Aryl ist und Alkyl C₁-C₆-Alkyl ist; C₈-C₁₆-Arylalkenyl, worin Aryl C₆-C₁₀-Aryl und Alkenyl C₂-C₆-Alkenyl ist; Alkoxyalkyl, worin Alkoxy C₁-C₆-Alkoxy und Alkyl C₁-C₆-Alkyl ist; CO₂(C₁-C₆-Alkyl); CO₂H; CH₂OH.
R₄: Wasserstoff; Hydroxy; C₁-C₆-Alkyloxy; C₂-C₁₀-Alkyloxyalkoxy, worin Alkyloxy C₁-C₄ ist und Alkoxy C₁-C₆-Alkyloxy ist; C₂-C₆-Alkenyloxy; C₂-C₆-Alkinyloxy; C₃-C₁₆-(cyclische gesättigte Gruppe)alkyloxy, worin Alkyl C₁-C₆ ist; C₄-C₁₆-(cyclische gesättigte Gruppe)alkenyloxy, worin Alkenyl C₂-C₆ ist; C₄-C₁₆-(cyclische gesättigte Gruppe)alkinyloxy, worin Alkinyl C₂-C₆ ist; C₇-C₁₆-Arylalkyloxy, worin Aryl C₆-C₁₀-Aryl ist und Alkyl C₁-C₆-Alkyl ist; C₈-C₁₆-Arylalkenyloxy, worin Aryl C₆-C₁₀-Aryl und Alkenyl C₂-C₆-Alkenyl ist; C₈-C₁₆-Arylalkinyloxy, worin Aryl C₆-C₁₀-Aryl ist und Alkinyl C₂-C₆-Alkinyl; C₂-C₆-Alkanoyloxy; C₃-C₆-Alkenoyloxy; C₃-C₆-Alkinoyloxy; C₈-C₁₆-Arylalkanoyloxy, worin Aryl C₆-C₁₀-Aryl ist und Alkanoyloxy C₂-C₆-Alkanoyloxy ist; C₉-C₁₆-Arylalkenoyloxy, worin Aryl C₆-C₁₀-Aryl ist und Alkenoyloxy C₃-C₆-Alkenoyloxy ist; C₉-C₁₆-Arylalkinoyloxy, worin Aryl C₆-C₁₀-Aryl ist und Alkinoyloxy C₃-C₆-Alkinoyloxy ist;
R_{5:}Wasserstoff; Hydroxy; C₁-C₆-Alkyloxy; C₂-C₁₀-Alkyloxyalkoxy, worin Alkyloxy C₁-C₄ ist und Alkoxy C₁-C₆-Alkyloxy ist; C₂-C₆-Alkenyloxy; C₂-C₆-Alkinyloxy; C₃-C₁₆-(cyclische gesättigte Gruppe)alkyloxy, worin Alkyl C₁-C₆ ist; C₄-C₁₆-(cyclische gesättigte Gruppe)alkenyloxy, worin Alkenyl C₂-C₆ ist; C₄-C₁₆-(cyclische gesättigte Gruppe)alkinyloxy, worin Alkinyl C₂-C₆ ist; C₇-C₁₆-Arylalkyloxy, worin Aryl C₆-C₁₀-Aryl ist und AlkylC₁-C₆-Alkyl ist; C₈-C₁₆-Arylalkenyloxy, worin Aryl C₆-C₁₀-Aryl und Alkenyl C₂-C₆-Alkenyl ist; C₈-C₁₆-Arylalkinyloxy, worin Aryl C₆-C₁₀-Aryl ist und Alkinyl C₂-C₆-Alkinyl; C₂-C₆-Alkanoyloxy; C₇-C₁₆-Arylalkanoyloxy, worin Aryl C₆-C₁₀-Aryl ist und Alkanoyloxy C₂-C₆-Alkanoyloxy ist;
X ist Sauerstoff;
Y⁻ ist I⁻, Br⁻, CI⁻, OH- oder ein anderes pharmakologisch akzeptierbares Gegenion;
wobei zwischen den Kohlenstoffatomen der Nummern 7 und 8 eine Einfach- oder eine Doppelbindung vorliegen kann,
wobei Alkyl, Alkenyl und Alkinyl jeweils verzweigt oder unverzweigt sein können, Aryl unsubstituiert oder mono-, di- oder trisubstituiert sein kann, jeweils unabhängig, mit Hydroxy, Halogen, Nitro, Cyano, Thiocyanato, Trifluormethyl, C₁-C₃-Alkyl, C₁-C₃-Alkoxy, CO₂H, CONH₂, CO₂(C₁-C₃-Alkyl), CONH(C₁-C₃-Alkyl), CON(C₁-C₃-Alkyl)₂, CO(C₁-C₃-Alkyl); amino; (C₁-C₃-Monoalkyl)amino, (C₁-C₃-Dialkyl)amino, C₅-C₆-Cycloalkylamino; (C₁-C₃-Alkanoyl)amido, SH, SO₃H, SO₃(C₁-C₃-Alkyl), SO₂(C₁-C₃-Alkyl), SO(C₁-C₃-Alkyl), C₁-C₃-Alkylthio oder C₁-C₃-Alkanoylthio,
wobei -(cyclische gesättigte Gruppe) entweder bevorzugt C₃-C₁₀-Cycloalkyl ist oder eine heterocyclische Gruppe mit 2 bis 9 Kohlenstoffatomen, enthaltend weiter ein oder mehrere Heteroatome.

3. Verbindungen der Formeln (I) und (IA) der Ansprüche 1 und 2, in denen X Sauerstoff ist; R₁ C₁-C₆-Alkyl, C₂-C₆-Alkenyl, C₄-C₁₆-Cycloalkylalkyl ist, worin Cycloalkyl C₃-C₁₀ ist und Alkyl C₁-C₆ ist, C₇-C₁₆-Arylalkyl ist, worin Aryl C₆-C₁₀ -Aryl ist und Alkyl C₁-C₆-Alkyl ist; R₂ C₇-C₁₆-Arylalkyl, worin Aryl C₆-C₁₀-Aryl ist und Alkyl C₁-C₆-Alkyl ist; C₈-C₁₆-Arylalkenyl, worin Aryl C₆-C₁₀-Aryl und Alkenyl C₂-C₆-Alkenyl ist; R₃ Wasserstoff oder Methyl ist: R₄ Hydroxy, Methoxy oder Acetoxy ist.

4. Verbindungen der Formel (IA) des Anspruchs 2, in denen X Sauerstoff ist; R₁ C₁-C₆₋Alkyl, C₂-C₆-Alkenyl, C₄-C₁₆-Cycloalkylalkyl ist, worin Cycloalkyl C₃-C₁₀ ist und Alkyl C₁-C₆ ist, C₇-C₁₆-Arylalkyl ist, worin Aryl C₆-C₁₀-Aryl ist und Alkyl C₁-C₆-Alkyl ist; R₂ C₁-C₆-Alkyl oder C₂-C₆-Alkenyl ist, R₃ Wasserstoff oder Methyl ist; R₄ Hydroxy, Methoxy oder Acetoxy ist.

5. Verbindungen der Ansprüche 1 und 2, ausgewählt unter:
17-Allyl-4,5α-epoxy-3-methoxy-14β-(3-phenylpropyloxy)morphinan-6-on, 17-Allyl-4,5α-epoxy-3-hydroxy-14β-(3-phenylpropyloxy)morphinan-6-on, 17-Allyl-4,5α-epoxy-3-methoxy-5β-methyl-14β-(3-phenylpropyloxy)morphinan-6-on, 17-Allyl-4,5α-epoxy-3 hydroxy-5β-methyl-14β-(3-phenylpropyloxy)morphinan-6-on, 17-Cyclobutylmethyl-4,5α-epoxy-3-methoxy-14β-(3-phenylpropyloxy)morphinan-6-on, 17-Cyclobutylmethyl-4,5α-epoxy-3-hydroxy-14β-(3-phenylpropyloxy)morphinan-6-on, 17-Cyclobutylmethyl-4,5α-epoxy-3-methoxy-5β-methyl-14β-(3-phenylpropyloxy)morphinan-6-on, 17-Cyclobutylmethyl-4,5α-epoxy-3-hydroxy-5β-methyl-14β-(3-phenylpropyloxy)morphinan-6-on, 17-Cyclopropylmethyl-4,5α-epoxy-3-methoxy-14β-(3-phenylpropyloxy)morphinan-6-on, 17-Cyclopropylmethyl-4,5α-epoxy-3-hydroxy-14β-(3-phenylpropyloxy)morphinan-6-on, 17-Cyclopropylmethyl-4,5α-epoxy-3-methoxy-5β-methyl-14β-(3-phenylpropyloxy)morphinan-6-on, 17-Cyclopropylmethyl-4,5α-epoxy-3-hydroxy-5β-methyl-14β-(3-phenylpropyloxy)morphinan-6-on, 4,5α-Epoxy-3-methoxy-5β,17-dimethyl-14β-[(3-phenylpropyl)oxy]morphinan-6-on, 4,5α-Epoxy-3-hydroxy-5β,17-dimethyl-14β-[(3-phenylpropyl)oxy]morphinan-6-on, 17-Propyl-4,5α-epoxy-3-methoxy-14β-(3-phenylpropyloxy)morphinan-6-on, 17-Propyl-4,5α-epoxy-3-hydroxy-14β-(3-phenylpropytoxy)morphinan-6-on, 17-Propyl -4,5α-epoxy-3-methoxy-5β-methyl-14β-(3-phenylpropyloxy)morphinan-6-on, 17-Propyl-4,5α-epoxy-3-hydroxy-5β-methyl-14β-(3-phenylpropytoxy)morphinan-6-on, 17-Tetrahydrofurfuryl-4,5α-epoxy-3-methoxy-14β-(3-phenylpropyloxy)morphinan-6-on, 17-Tetrahydrofurfuryl-4,5α-epoxy-3-hydroxy-14β-(3-phenylpropyloxy)morphinan-6-on, 17-Tetrahydrofurfuryl-4,5α-epoxy-3-methoxy-5β-methyl-14β-(3-phenylpropyloxy)morphinan-6-on, 17-Tetrahydrofurfuryl-4,5α-epoxy-3-hydroxy-5β-methyl-14β-(3-phenylpropyloxy)morphinan-6-on, 17-(2-Phenylethyl)-4,5α-epoxy-3-methoxy-14β-(3-phenylpropyloxy)morphinan-6-on, 17-(2-Phenylethyl)-4,5α-epoxy-3-hydroxy-14β-(3-phenylpropyloxy)morphinan-6-on, 17-(2-Phenylethyl)-4,5α-epoxy-3-methoxy-5β-methyl-14β-(3-phenylpropyloxy)morphinan-6-on, 17-(2-Phenylethyl)-4,5α-epoxy-3-hydroxy-5β-methyl-14β-(3-phenylpropyloxy)morphinan-6-on, 17-Ethyl-4,5α-epoxy-3-methoxy-14β-(3-phenylpropyloxy)morphinan-6-on, 17-Ethyl-4,5α-epoxy-3-hydroxy-14β-(3-phenylpropyloxy)morphinan-6-on, 17-Ethyl-4,5α-epoxy-3-methoxy-5β-methyl-14β-(3-phenylpropyloxy)morphinan-6-on, 17-Ethyl-4,5α-epoxy-3-hydroxy-5β-methyl-14β-(3-phenylpropyloxy)morphinan-6-on, 17-Cyclopropylmethyl-4,5α-epoxy-3-hydroxy-14β-[(2-methylbenzyl)oxy]-morphinan-6-on, 14β-[(2-Chlorbenzyl)oxy]-17-(cyclopropylmethyl)-4,5α-epoxy-3-hydroxymorphinan-6-on, 14β-Benzyloxy-17-cyclopropylmethy)-4,5α-epoxy-3-hydroxymorphinan-6-on, 14β-Butoxy-17-cyclopropylmethyl-4,5α-epoxy-3-hydroxymorphinan-6-on, 17-Cyclopropylmethyl-4,5α-epoxy-3-hydroxy-14β-[(3-methylbutyl)oxy]morphinan-6-on, 4,5α-Epoxy-5β,17-dimethyl-14β-[(3-phenylpropyl)oxy]-3-[(prop-2-inyl)oxy]morphinan-6-on, 14β-[(3-Chlorbenzy 4,5α-epoxy-17-methyl-3-[(prop-2-inyl)oxy]morphinan-6-on, 4,5α-Epoxy-17-ethyl-3-methoxy-14β-[(3-phenylpropyl)oxy]morphinan-6-on, 4,5α-Epoxy-17-ethyl-3-hydroxy-14β-[(3-phenylpropyl)oxy]morphinan-6-on, 4,5α-Epoxy-3-hydroxy-14β-[(3-methylbutyl)oxy]-17-propylmorphinan-6-on, 5β-Benzyl-14-methoxycodeinon (= 5-Benzyl-7,8-didehydro-4,5α-epoxy-3,14β-dimethoxy-17-methyl-morphinan-6-on), 5β-Benzyl-4,5α-epoxy-3,14β-dimethoxy-17-methylmorphinan-6-on, 5β-Benzyl-4,5α-epoxy-3-hydroxy-14β-methoxy-17-methylmorphinan-6-on, 4-Hydroxy-3-methoxy-17-methyl-14-[(3-phenylpropyl)oxy]-morphinan-6-on, 3,4-Dimethoxy-17-methyl-14-[(3-phenylpropyl)oxy]morphinan-6-on, 14β-Benzyloxy-4-hydroxy-3-methoxy-17-methylmorphinan-6-on, 14β-Benzyloxy-3,4-dimethoxy-17-methylmorphinan-6-on, 4-Hydroxy-3-methoxy-17-methyl-14β-[(2-naphthylmethyl)oxy]morphinan-6-on, 3,4-Dimethoxy-17-methyl-14β-[(2-naphtylmethyl)oxy]morphinan-6-on, 4-Hydroxy-3-methoxy-5β,17-dimethyl-14β-[(3-phenylpropyl)oxylmorphinan-6-on, 3,4-Dimethoxy-5β,17-dimethyl-14β-[(3-phenylpropyl)oxy]morphinan-6-on, 14β-Ethoxy-4-hydroxy-3-methoxy-5β,17-dimethylmorphinan-6-on, 14β-Ethoxy-3,4-dimethoxy-5β,17-dimethylmorphinan-6-on, 14β-Benzyloxy-3,4-dimethoxy-5β,17-dimethylmorphinan-6-on, 4,5α-Epoxy-3-hydroxy-17,17-dimethyl-6-oxo-14β-[(3-phenylpropyl]oxy]morphinaniumiodid, (17S)-4,5α-Epoxy-17-ethyl-3-hydroxy-17-methyl-6-oxo-14β-[(3-phenylpropyl]oxy]morphinaniumiodid, (17R)-4,5α-Epoxy-3-hydroxy-17-methyl-6-oxo-14β-[(3-phenylpropyl)oxy]-17-[(2(R,S)-tetrahydrofurfuran-2-yl)methyl]morphinaniumiodid, (17R)-17-Allyl-4,5α-epoxy-14β-ethoxy-3-hydroxy-17-methyl-6-oxomorphinaniumiodid, (17R)-17-Allyl-4,5α-epoxy 3-hydroxy-14β-methoxy-17-methyl-6-oxomorphinaniumiodid, (175)-17-Allyl-4,5α-epoxy-3-hydroxy-14β-methoxy-17-methyl-6-oxomorphinaniumiodid, 4,5α-Epoxy-3-hydroxy-14β-methoxy-17,17-dimethyl-6-oxo-morphinaniumiodid, 5β-Benzyl-14β-(butyloxy)-4,5α-epoxy-3-hydroxy-17,17-dimethyl-6-Oxomorphinaniumiodid, (17S)-17-Allyl-5β-benzyl-14β-butoxy-4,5α-epoxy-3-hydroxy-17-methyl-6-oxomorphinaniumiodid, 14β-Butoxy-4,5α-epoxy-3-hydroxy-17,17-dimethyl-6-oxomorphinaniumiodid, (17R)-17-Cyclopropylmethyl-4,5α-epoxy-3-hydroxy-17-methyl-6-oxo-14β-[(3-phenylpropyl)oxy]morphinaniumiodid, (17R)-17-Cyclopropylmethyl-4,5α-epoxy-3-methoxy-17-methyl-6-oxo-14β-[(3-phenylpropyl)oxy]morphinaniumiodid, (17R)-17-Cyclopropylmethyl-4,5α-epoxy-3-hydroxy-17-methyl-6-oxo-14β-[(2-phenylbenzyl)oxy]morphinaniumiodid, (17R)-14β-[(4-Chlorbenzyl)oxy]-17-cyclopropylmethyl-4,5α-epoxy-3-hydroxy-17-methyl-6-oxomorphinaniumiodid, 17(R)-4,5α-Epoxy-3-hydroxy-14β-methoxy-17-methyl-6-oxo-17-(2-phenylethyl)morphinaniumiodid, 4,5α-Epoxy-3-methoxy-17-methyl-14β-[(3-phenylpropyl)oxy]morphinan-6-on,
4,5α-Epoxy-3-methoxy-14β-[(3-phenylpropyl)oxy]morphinan-6-on,
4,5α-Epoxy-3-hydroxy-17-methyl-14β-[(3-phenylpropyl)oxy]morphinan-6-on,
4,5α-Epoxy-17-methyl-14β-[(3-phenylpropyl)oxy]morphinan-6-on,
17-(Cyclopropylmethyl)-4,5α-epoxy-14β-[(3-phenylpropyl)oxy]morphinan-6-on,
4,5α-Epoxy-14β-[(3-phenylpropyl)oxy]morphinan-6-on,
17-(Cyclopropylmethyl)-4-hydroxy-14β-[(3-phenylpropyl)oxy]morphinan-6-on,
17-(Cyclopropylmethyl)-4-methoxy-14β-[(3-phenylpropyl)oxy]morphinan-6-on,
4-(n-Butyloxy)-17-(cyclopropylmethyl)-14β-[(3-phenylpropyl)oxy]morphinan-6-on,
oder irgendein pharmazeutisch akzeptables Salz oder leicht zugängliches Derivat davon.

6. Zusammensetzung, umfassend eine Verbindung der Ansprüche 1 bis 5 und/oder ein pharmazeutisch akzeptables Säureadditionssalz davon, zusammen mit einem pharmazeutisch akzeptablen Trägerstoff.

7. Verbindung nach irgendeinem der Ansprüche 1 bis 6 als Medikament.

8. Verwendung einer Verbindung der Ansprüche 1 bis 5 für die Herstellung eines Medikaments zur Behandlung von Schmerzen, einschließlich chronischer und akuter Schmerzen, Operationsschmerzen, rheumatischen Erkrankungen (z. B. Arthritis), Ileus, Obstipation, Übergewicht, Sucht, einschließlich Opioid-, Kokain- und Alkoholsucht, sowie zur Herstellung eines Narkotikums.

9. Verbindungen nach Anspruch 1 oder 2, wobei R₅ OH oder Alkoxy ist.

10. Verbindungen nach Anspruch 1, 2 oder 9, wobei R₃ Wasserstoff, Alkyl oder Aralkyl ist, bevorzugt Wasserstoff oder Alkyl.

11. Verbindungen nach Anspruch 1, 2, 9 oder 10, wobei R₄ OH, Alkoxy oder Alkenyloxy oder Alkinyloxy ist.

12. Verbindungen nach Anspruch 1, 2, 9, 10 oder 11, wobei zwischen den Kohlenstoffatomen der Nummern 7 und 8 eine Einfachbindung vorliegt.

13. Verbindungen nach Anspruch 1, 2, 9, 10, 11 oder 12, wobei R₂, Alkyl oder Aralkyl ist, bevorzugt Aralkyl.

14. Verbindungen nach Anspruch 1, 2, 9, 10, 11, 12 oder 13, wobei R₁ Alkyl, (cyclische gesättigte Gruppe)alkyl, Aralkyl oder Alkenyl ist.

15. Verbindungen nach Anspruch 1 oder 2, wobei R₁ C₁-C₆-Alkyl; C₂-C₆-Alkenyl, C₂-C₆-Alkinyl; C₃-C₁₆-(cyclische gesättigte Gruppe)alkyl, worin Alkyl C₁-C₆ ist; C₄-C₁₆-(cyclische gesättigte Gruppe)alkenyl, worin Alkenyl C₂-C₆ ist; C₄-C₁₆-(cyclische gesättigte Gruppe)alkinyl, worin Alkinyl C₂-C₆ ist; C₇-C₁₆-Arylalkyl, worin Aryl C₆-C₁₀-Aryl ist und Alkyl C₁-C₆-Alkyl ist; C₈-C₁₆-Arylalkenyl, worin Aryl C₆-C₁₀-Aryl und Alkenyl C₂-C₆-Alkenyl ist; C₈-C₁₆-Arylalkinyl, worin Aryl C₆-C₁₀-Aryl ist und Alkinyl C₂-C₆-Alkinyl ist.

## Claims

1. Compounds of the formula (I) or (1a), 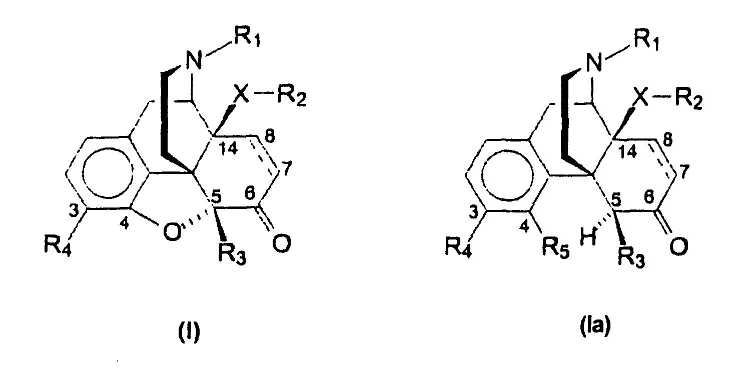 in which the substituents have the following significance:
R₁: C₁-C₆-alkyl; C₂-C₆-alkenyl; C₂-C₆-alkinyl; C₃-C₁₆-(cyclical saturated group)alkyl, where alkyl is C₁-C₆; C₄-C₁₆-(cyclical saturated group)alkenyl, where alkenyl is C₂-C₆; C₄-C₁₆-(cyclical saturated group)alkinyl, where alkinyl is C₂-C₆; C₇-C₁₆-arylalkyl, where aryl is C₆-C₁₀-aryl and alkyl is C₁-C₆-alkyl; C₈-C₁₆-arylalkenyl, where aryl is C₆-C₁₀-aryl and alkenyl is C₂-C₆-alkenyl; C₈-C₁₆-arylalkinyl, where aryl is C₆-C₁₀-aryl and alkinyl is C₂-C₆-alkinyl;
R₂: C₄-C₆-alkyl; C₂-C₆-alkenyl; C₂-C₆-alkinyl; C₃-C₁₆-(cyclical saturated group)alkyl, where alkyl is C₁-C₆; C₄-C₁₆-(cyclical saturated group)alkenyl, where alkenyl is C₂-C₆; C₄-C₁₆-(cyclical saturated group)alkinyl, where alkinyl is C₂-C₆; C₈-C₁₆-arylalkyl, where aryl is C₆-C₁₀-aryl and alkyl is C₁-C₆-alkyl; C₈-Ci₆-arylalkenyl, where aryl is C₆-C₁₀-aryl and alkenyl is C₂-C₆-alkenyl; C₈-C₁₆-arylalkinyl, where aryl is C₆-C₁₀-aryl and alkinyl is C₂-C₆-aklinyl; C₃-C₆-alkenoyl; C₃-C₆-alkinoyl; C₉-C₁₆-arylalkenoyl, where aryl is C₆-C₁₀-aryl and alkenoyl is C₃-C₆-alkenoyl; C₉-C₁₆-arylalkinoyl, where aryl is C₆-C₁₀-aryl and alkinoyl is C₃-C₆-alkinoyl;
R₃: hydrogen; C₁-C₆-alkyl; C₂-C₆-alkenyl; C₇-C₁₆-arylalkyl, where aryl is C₆-C₁₀-aryl and alkyl is C₁-C₆-alkyl; C₈-C₁₆-arylalkenyl, where aryl is C₆-C₁₀-aryl and alkenyl is C₂-C₆-alkenyl; alkoxyalkyl, where alkoxy is C₁-C₆-alkoxy and alkyl is C₁-C₆-alkyl; CO₂(C₁-C₆-alkyl); CO₂H; CH₂OH.
R₄: hydrogen; hydroxy; C₁-C₆-alkyloxy; C₂-C₁₀-alkyloxyalkoxy, where alkyloxy is C₁-C₄ and alkoxy is C₁-C₆-alkyloxy; C₂-C₆-alkenyloxy; C₂-C₆-alkinyloxy; C₃-C₁₆-(cyclical saturated group)alkyloxy, where alkyl is C₁-C₆; C₄-C₁₆-(cyclical saturated group)alkenyloxy, where alkenyl is C₂-C₆; C₄-C₁₆-(cyclical saturated group)alkinyloxy where alkinyl is C₂-C₆; C₇-C₁₆-arylalkyloxy, where aryl is C₆-C₁₀-aryl and alkyl is C₁-C₆-alkyl; C₈-C₁₆-arylalkenyloxy, where aryl is C₆-C₁₀-aryl and alkenyl is C₂-C₆-alkenyl; C₈-C₁₆-arylalkinyloxy, where aryl is C₆-C₁₀-aryl and alkinyl is C₂-C₆-alkinyl; C₁-C₆-alkanoyloxy; C₃-C₆-alkenoyloxy; C₃-C₆-alkinoyloxy; C₇-C₁₆-arylalkanoyloxy, where aryl is C₆-C₁₀-aryl and alkanoyloxy is C₂-C₆-alkanoyloxy; C₉-C₁₆-arylaikenoyloxy, where aryl is C₉-C₁₀-aryl and alkenoyloxy is C₃-C₆-alkenoyloxy; C₉-C₁₆-arylalkinoyloxy, where aryl is C₆-C₁₀-aryl and alkinoyloxy is C₃-C₆-alkinoyloxy;
R₅: hydrogen; hydroxy; C₁-C₆-alkyloxy; C₂-C₁₀-alkyloxyalkoxy, where alkyloxy is C₁-C₄ and alkoxy is C₁-C₆-alkyloxy; C₂-C₆-alkenyloxy; C₂-C₆-alkinyloxy; C₃-C₁₆-(cyclical saturated group)alkyloxy, where alkyl is C₁-C₆; C₄-C₁₆-(cyclical saturated group)alkenyloxy, where alkenyl is C₂-C₆; C₄-C₁₆-(cyclical saturated group)alkinyloxy, where alkinyl is C₂-C₆; C₇-C₁₆-arylalkyloxy, where aryl is C₆-C₁₀-aryl and alkyl is C₁-C₆-alkyl; C₈-C₁₆-arylalkenyloxy, where aryl is C₆-C₁₀-aryl and alkenyl is C₂-C₆-alkenyl; C₈-C₁₆-arylalkinyloxy, where aryl is C₆-C₁₀-aryl and alkinyl is C₂-C₆-alkinyl; C₂C₆-alkanoyloxy; C₇-C₁₆-arylalkanoyloxy, where aryl is C₆-C₁₀-aryl and alkanoyloxy is C₂-C₆-alkanoyloxy;
X is oxygen;
wherein a single or double bond can be present between the carbon atoms of numbers 7 and 8,
wherein alkyl, alkenyl and alkinyl can each be branched or unbranched, aryl can be unsubstituted or mono-, di- or trisubstituted, independently in each case, with hydroxy, halogen, nitro, cyano, thiocyanato, trifluoromethyl, C₁-C₃-alkyl, C₁-C₃-alkoxy, CO₂H, CONH₂, CO₂(C₁-C₃-alkyl), CONH(C₁-C₃-alkyl), CON(C₁-C₃-alkyl)₂, CO(C₁-C₃-alkyl); amino; (C₁-C₃-monoalkyl)amino, (C₁-C₃-dialkyl)amino; C₅-C₆-cycloalkylamino; (C₁-C₃-alkanoyl)amido, SH, SO₃H, SO₃(C₁-C₃-alkyl), SO₂(C₁-C₃-alkyl), SO(C₁-C₃-alkyl), C₁-C₃-alkylthio or C₁-C₃-alkanoylthio,
wherein -(cyclical saturated group) is either preferably C₃-C₁₀-cycloalkyl or a heterocyclical group with 2 to 9 carbon atoms, containing further one or more heteroatoms,
**with the exception of compounds** where R₁ is methyl, R₂ is C₄-C₆-alkyl, R₃ is hydrogen or methyl, R₄ is hydroxy or methoxy and R₅ is hydroxy, methoxy or an oxygen atom bound to the carbon atom in the 5^{th} position, when X is oxygen;
**with the further exception of compounds** where R₁ is cyclopropylmethyl and XR₂ is benzyloxy, when R₄ is oxygen or benzyloxy and R₅ is an oxygen atom bound to the carbon atom in the 5^{th} position;
**with the further exception of compounds** where R₁ is cyclopropylmethyl and XR₂ is benzyloxy, when R₄ is oxygen, hydroxy or benzyloxy and R₅ is hydroxy or methoxy.

2. Compounds of the formula (IA) or (IAa), 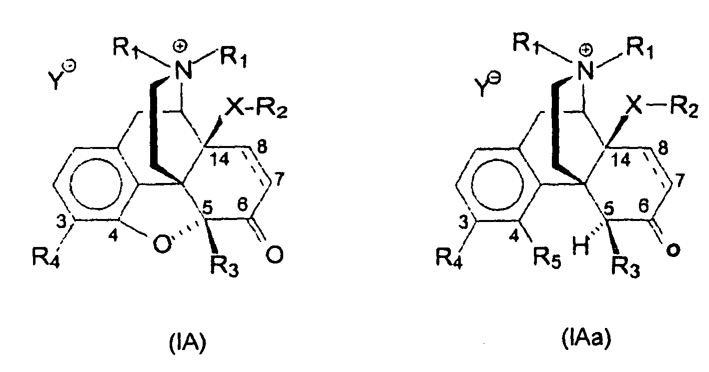 where the substituents have the following significance:
R₁: C₁-C₆-alkyl; C₂-C₆-alkenyl; C₂-C₆-alkinyl; C₃-C₁₆-(cyclical saturated group)alkyl, where alkyl is C₁- C₆; C₄-C₁₆-(cyclical saturated group)alkenyl, where alkenyl is C₂-C₆; C₄-C₁₆-(cyclical saturated group)alkinyl, where alkinyl is C₂-C₆; C₇-C₁₆-arylalkyl, where aryl is C₆-C₁₀-aryl and alkyl is C₁-C₆-alkyl; C₈-C₁₆-arylalkenyl, where aryl is C₆-C₁₀-aryl and alkenyl is C₂-C₆-alkenyl; C₈-C₁₆-arylalkinyl, where aryl is C₆-C₁₀-aryl and alkinyl is C₂-C₆-alkinyl;
wherein the two substituents R₁ can be the same or different;
R₂: C₁-C₆-alkyl; C₂-C₆-alkenyl; C₂-C₆-alkinyl; C₃-C₁₆-(cyclical saturated group)alkyl, where alkyl is C₁-C₆; C₄-C₁₆-(cyclical saturated group)alkenyl, where alkenyl is C₂-C₆; C₄-C₁₆-(cyclical saturated group)alkinyl, where alkinyl is C₂-C₆; C₈-C₁₆-arylalkyl, where aryl is C₆-C₁₀-aryl and alkyl is C₁-C₆-alkyl; C₈-C₁₆-arylalkenyl, where aryl is C₆-C₁₀-aryl and alkenyl is C₂-C₆-alkenyl; C₈-C₁₆-arylalkinyl, where aryl is C₆-C₁₀-aryl and alkinyl is C₂-C₆-alkinyl; C₃-C₆-alkenoyl; C₃-C₆-alkinoyl; C₉-C₁₆-arylalkenoyl, where aryl is C₆-C₁₀-aryl and alkenoyl is C₃-C₆-alkenoyl; C₉-C₁₆-arylalkinoyl, where aryl is C₆-C₁₀-aryl and alkinoyl is C₃-C₆-alkinoyl;
R₃: hydrogen; C₁-C₆-alkyl; C₂-C₆-alkenyl; C₇-C₁₆-arylalkyl, where aryl is C₆-C₁₀-aryl and alkyl is C₁-C₆-alkyl; C₈-C₁₆-arylalkenyl, where aryl is C₆-C₁₀-aryl and alkenyl is C₂-C₆-alkenyl; alkoxyalkyl, where alkoxy is C₁-C₆-alkoxy and alkyl is C₁-C₆-alkyl; CO₂(C₁-C₆-alkyl); CO₂H; CH₂OH.
R₄: hydrogen; hydroxy; C₁-C₆-alkyloxy; C₂-C₁₀-alkyloxyalkoxy, where alkyloxy is C₁-C₄ and alkoxy is C₁-C₆-alkyloxy; C₂-C₆-alkenyloxy; C₂-C₆-alkinyloxy; C₃-C₁₆-(cyclical saturated group)alkyloxy, where alkyl is C₁-C₆; C₄-C₁₆-(cyclical saturated group)alkenyloxy, where alkenyl is C₂-C₆; C₄-C₁₆-(cyclical saturated group)alkinyloxy where alkinyl is C₂-C₆; C₇-C₁₆-arylalkyloxy, where aryl is C₆-C₁₀-aryl and alkyl is C₁-C₆-alkyl; C₈-C₁₆-arylalkenyloxy, where aryl is C₆-C₁₀-aryl and alkenyl is C₂-C₆-alkenyl; C₈-C₁₆-arylalkinyloxy, where aryl is C₆-C₁₀-aryl and alkinyl is C₂-C₆-alkinyl; C₂-C₆-alkanoyloxy; C₃-C₆-alkenoyloxy; C₃-C₆-alkinoyloxy; C₈-C₁₆-arylalkanoyloxy, where aryl is C₆-C₁₀-aryl and alkanoyloxy is C₂-C₆-alkanoyloxy; C₉-C₁₆-arylalkenoyloxy, where aryl is C₆-C₁₀-aryl and alkenoyloxy is C₃-C₆-alkenoyloxy; C₉-C₁₆-arylalkinoyloxy, where aryl is C₆-C₁₀-aryl and alkinoyloxy is C₃-C₆-alkinoyloxy;
R₅: hydrogen; hydroxy; C₁-C₆-alkyloxy; C₂-C₁₀-alkyloxyalkoxy, where alkyloxy is C₁-C₄ and alkoxy is C₁-C₆-alkyloxy; C₂-C₆-alkenyloxy; C₂-C₆-alkinyloxy; C₃-C₁₆-(cyclical saturated group)alkyloxy, where alkyl is C₁-C₆; C₄-C₁₆-(cyclical saturated group)alkenyloxy, where alkenyl is C₂-C₆; C₄-C₁₆-(cyclical saturated group)alkinyloxy, where alkinyl is C₂-C₆; C₇-C₁₆-arylalkyloxy, where aryl is C₆-C₁₀-aryl and alkyl is C₁-C₆-alkyl; C₈-C₁₆-arylalkenyloxy, where aryl is C₆-C₁₀-aryl and alkenyl is C₂-C₆-alkenyl; C₈-C₁₆-arylalkinyloxy, where aryl is C₆-C₁₀-aryl and alkinyl is C₂-C₆-alkinyl; C₂-C₆-alkanoyloxy; C₇-C₁₆-arylalkanoyloxy, where aryl is C₆-C₁₀-aryl and alkanoyloxy is C₂-C₆-alkanoyloxy;
X is oxygen;
Y⁻ is I⁻, Br⁻, Cl⁻, OH- or another pharmacologically acceptable counterion;
wherein a single or double bond can be present between the carbon atoms of numbers 7 and 8,
wherein alkyl, alkenyl and alkinyl can each be branched or unbranched, aryl can be unsubstituted or mono-, di- or trisubstituted, independently in each case, with hydroxy, halogen, nitro, cyano, thiocyanato, trifluoromethyl, C₁-C₃-alkyl, C₁-C₃-alkoxy, CO₂H, CONH₂, CO₂(C₁-C₃-alkyl), CONH(C₁-C₃-alkyl), CON(C₁-C₃-alkyl)₂, CO(C₁-C₃-alkyl); amino; (C₁-C₃-monoalkyl)amino, (C₁-C₃-dialkyl)amino; C₅-C₆-cycloalkylamino; (C₁-C₃-alkanoyl)amido, SH, SO₃H, SO₃(C₁-C₃-alkyl), SO₂(C₁-C₃-alkyl), SO(C₁-C₃-alkyl), C₁-C₃-alkylthio or C₁C₃-alkanoylthio,
wherein -(cyclical saturated group) is either preferably C₃-C₁₀-cycloalkyl or a heterocyclical group with 2 to 9 carbon atoms, containing furthermore one or more heteroatoms.

3. Compounds of the formulae (I) or (IA) of Claims 1 and 2, in which X is oxygen; R₁ is C₁-C₆-alkyl; C₂-C₆-alkenyl; C₄-C₁₆-cycloalkylalkyl, where cycloalkyl is C₃-C₁₀ and alkyl is C₁-C₆; C₇-C₁₆-arylalkyl, where aryl is C₆-C₁₀-aryl and alkyl is C₁-C₆-alkyl; R₂ is C₇-C₁₆-arylalkyl, where aryl is C₆-C₁₀-aryl and alkyl is C₁-C₆-alkyl; C₈-C₁₆-arylalkenyl, where aryl is C₆-C₁₀-aryl and alkenyl is C₂-C₆-alkenyl; R₃ is hydrogen or methyl; R₄ is hydroxy, methoxy or acetoxy.

4. Compounds of the formula (IA) of Claim 2, in which X is oxygen; R₁ is C₁-C₆-alkyl; C₂-C₆-alkenyl; C₄-C₁₆-cycloalkylalkyl, where cycloalkyl is C₃-C₁₀ and alkyl is C₁-C₆, C₇-C₁₆-arylalkyl, where aryl is C₆-C₁₀-aryl and alkyl is C₁-C₆-alkyl; R₂ is C₁-C₆-alkyl or C₂-C₆-alkenyl, R₃ is hydrogen or methyl; R₄ is hydroxy, methoxy or acetoxy.

5. Compounds of Claims 1 and 2, selected from:
17-allyl-4,5a-epoxy-3-methoxy-14f3-(3-phenylpropyloxy)morphinan-6-one, 17-allyl-4,5α-epoxy-3-hydroxy-14β-(3-phenylpropyloxy)morphinan-6-one, 17-allyl-4,5α-epoxy-3-methoxy-5β-methyl-14β-(3-phenylpropyloxy)morphinan-6-one, 17-allyl-4,5α-epoxy-3-hydroxy-5β-methyl-14β-(3-phenylpropyloxy)morphinan-6-one, 17-cyclobutylmethyl-4,5α-epoxy-3-methoxy-14β-(3-phenylpropyloxy)morphinan-6-one, 17-cyclobutylmethyl-4,5α-epoxy-3-hydroxy-14β-(3-phenylpropyloxy)morphinan-6-one, 17-cydobutylmethyl-4,5α-epoxy-3-methoxy-5β-methyl-14β-(3-phenylpropyloxy)morphinan-6-one, 17-cyclobutylmethyl-4,5α-epoxy-3-hydroxy-5β-methyl-14β-(3-phenylpropyloxy)morphinan-6-one, 17-cyclopropylmethyl-4,5α-epoxy-3-methoxy-14β-(3-phenylpropyloxy)morphinan-6-one, 17-cyclopropylmethyl-4,5α-epoxy-3-hydroxy-14β-(3-phenylpropyloxy)morphinan-6-one, 17-cyclopropylmethyl-4,5α-epoxy-3-methoxy-5β-methyl-14β-(3-phenylpropyloxy)morphinan-6-one, 17-cyclopropylmethyl-4,5α-epoxy-3-hydroxy-5β-methyl-14β-(3-phenylpropyloxy)morphinan-6-one, 4,5α-epoxy-3-methoxy-5β,17-dimethyl-14β-[(3-phenylpropyl)oxy]morphinan-6-one, 4,5α-epoxy-3-hydroxy-5β,17-dimethyl-14β-[(3-phenylpropyl)oxy]morphinan-6-one, 17-propyl-4,5α-epoxy-3-methoxy-14β-(3-phenylpropyloxy)morphinan-6-one, 17-propyl-4,5α-epoxy-3-hydroxy-14β-(3-phenylpropyloxy)morphinan-6-one, 17-propyl-4,5α-epoxy-3-methoxy-5β-methyl-14β-(3-phenylpropyloxy)morphinan-6-one, 17-propyl-4,5α-epoxy-3-hydroxy-5β-methyl-14β-(3-phenylpropyloxy)morphinan-6-one, 17-tetrahydrofurfuryl-4,5α-epoxy-3-methoxy-14β-(3-phenylpropyloxy)morphinan-6-one, 17-tetrahydrofuriuryl-4,5α-epoxy-3-hydroxy-14β-(3-phenylpropyloxy)morphinan-6-one, 17-tetrahydrofurfuryl-4,5α-epoxy-3-methoxy-5β-methyl-14β-(3-phenylpropyloxy)morphinan-6-one, 17-tetrahydrofurfuryl-4,5α-epoxy-3-hydroxy-5β-methyl-14β-(3-phenylpropyloxy)morphinan-6-one, 17-(2-phenylethyl)-4,5α-epoxy-3-methoxy-14β-{3-phenylpropyloxy)morphinan-6-one, 17-(2-phenylethyl)-4,5α-epoxy-3-hydroxy-14β-(3-phenylpropyloxy)morphinan-6-one, 17-(2-phenylethyl)-4,5α-epoxy-3-methoxy-5β-methyl-14β-(3-phenylpropyloxy)morphinan-6-one, 17-(2-phenylethyl)-4,5α-epoxy-3-hydroxy-5β-methyl-14β-(3-phenylpropyloxy)morphinan-6-one, 17-ethyl-4,5α-epoxy-3-methoxy-14β-(3-phenylpropyloxy)morphinan-6-one, 17-ethyl-4,5α-epoxy-3-hydroxy-14β-(3-phenylpropyloxy)morphinan-6-one, 17-ethyl-4,5α-epoxy-3-methoxy-5β-methyl-14β-(3-phenylpropyloxy)morphinan-6-one, 17-ethyl-4,5α-epoxy-3-hydroxy-5β-methyl-14β-(3-phenylpropyloxy)morphinan-6-one, 17-cyclopropylmethyl-4,5α-epoxy-3-hydroxy-14β-[(2-methylbenzyl)oxy]morphinan-6-one, 14β-[(2-chlorobenzyl)oxy]-17-(cyclopropylmethyl)-4,5α-epoxy-3-hydroxymorphinan-6-one, 14β-benzyloxy-17-cyclopropylmethyl-4,5α-epoxy-3-hydroxymorphinan-6-one, 14β-butoxy-17-cyclopropylmethyl-4,5α-epoxy-3-hydroxymorphinan-6-one, 17-cyclopropylmethyl-4,5α-epoxy-3-hydroxy-14β-[(3-methylbutyl)oxy]morphinan-6-one, 4,5a-epoxy-5β,17-dimethyl-14β-[(3-phenylpropyl)oxy]-3-[(prop-2-inyl)oxy]morphinan-6-one, 14β-[(3-chlorobenzyl)oxy]-4,5α-epoxy-17-methyl-3-[(prop-2-inyl)oxy]morphinan-6-one, 4,5α-epoxy-17-ethyl-3-methoxy-14β-[(3-phenylpropyl)oxy]morphinan-6-one, 4,5α-epoxy-17-ethyl-3-hydroxy-14β-[(3-phenylpropyl)oxy]morphinan-6-one, 4,5α-epoxy-3-hydroxy-14β-[(3-methylbutyl)oxy]-17-propylmorphinan-6-one, 5β-benzyl-14-methoxycodeinone (= 5-benzyl-7,8-didehydro-4,5α-epoxy-3,14β-dimethoxy-17-methyl-morphinan-6-one), 5β-benzyl-4,5α-epoxy-3,14β-dimethoxy-17-methylmorphinan-6-one, 5β-benzyl-4,5α-epoxy-3-hydroxy-14β-methoxy-17-methylmorphinan-6-one, 4-hydroxy-3-methoxy-17-methyl-14-[(3-phenylpropyl)oxy]-morphinan-6-one, 3,4-dimethoxy-17-methyl-14-[(3-phenylpropyl)oxy]-morphinan-6-one, 14β-benzyloxy-4-hydroxy-3-methoxy-17-methylmorphinan-6-one, 14β-benzyloxy-3,4-dimethoxy-17-methylmorphinan-6-one, 4-hydroxy-3-methoxy-17-methyl-14β-[(2-naphthylmethyl)oxy]morphinan-6-one, 3,4-dimethoxy-17-methyl-14β-[(2-naphthylmethyl)oxy]morphinan-6-one, 4-hydroxy-3-methoxy-5β, 17-dimethyl-14β-[(3-phenylpropyl)oxy]-morphinan-6-one, 3,4-dimethoxy-5β,17-dimethyl-14β-[(3-phenylpropyl)oxy]-morphinan-6-one, 14β-ethoxy-4-hydroxy-3-methoxy-5β, 17-dimethylmorphinan-6-one, 14β-ethoxy-3,4-dimethoxy-5β,17-dimethylmorphinan-6-one, 14β-benzyloxy-3,4-dimethoxy-5β,17-dimethylmorphinan-6-one, 4,5α-epoxy-3-hydroxy-17,17-dimethyl-6-oxo-14β-[(3-phenylpropyl)oxy]morphinanium-iodide, (17S)-4,5α-epoxy-17-ethyl-3-hydroxy-17-methyl-6-oxo-14β-[(3-phenylpropyl)oxy]morphinanium-iodide, (17R)-4,5α-epoxy-3-hydroxy-17-methyl-6-oxo-14β-[(3-phenylpropyl)oxy]-17-[(2(R,S)-tetrahydrofurfuran-2-yl)methyl]morphinanium-iodide, (17R)-17-allyl-4,5α-epoxy-14β-ethoxy-3-hydroxy-17-methyl-6-oxomorphinanium-iodide, (17R)-17-allyl-4,5α-epoxy 3-hydroxy-14β-methoxy-17-methyl-6-oxomorphinanium-iodide, (17S)-17-allyl-4,5α-epoxy-3-hydroxy-14β-methoxy-17-methyl-6-oxomorphinanium-iodide, 4,5α-epoxy-3-hydroxy-14β-methoxy-17,17-dimethyl-6-oxo-morphinanium-iodide, 5β-benzyl-14β-(butyloxy)-4,5α-epoxy-3-hydroxy-17,17-dimethyl-6-oxomorphinanium-iodide, (17S)-17-allyl-5β-benzyl-14β-butoxy-4,5α-epoxy-3-hydroxy-17-methyl-6-oxomorphinanium-iodide, 14β-butoxy-4,5α-epoxy-3-hydroxy-17,17-dimethyl-6-oxomorphinanium-iodide, (17R)-17-cyclopropylmethyl-4,5α-epoxy-3-hydroxy-17-methyl-6-oxo-14β-[(3-phenylpropyl)oxy]morphinanium-iodide, (17R)-17-cyclopropylmethyl-4,5α-epoxy-3-methoxy-17-methyl-6-oxo-14β-[(3-phenylpropyl)oxy]morphinanium-iodide, (17R)-17-cyclopropylmethyl-4,5α-epoxy-3-hydroxy-17-methyl-6-oxo-14β-[(2-phenylbenzyl)oxy]morphinanium-iodide, (17R)-14β-[(4-chlorobenzyl)oxy]-17-cyclopropylmethyl-4,5α-epoxy-3-hydroxy-17-methyl-6-oxomorphinanium-iodide, 17(R)-4,5α-epoxy-3-hydroxy-14β-methoxy-17-methyl-6-oxo-17-(2-phenylethyl)morphinanium-iodide, 4,5α-expoxy-3-methoxy-17-methyl-14β-[(3-phenylpropyl)oxy]morphinan-6-one, 4,5α-expoxy-3-methoxy-14β-[(3-phenylpropyl)oxy]morphinan-6-one, 4,5α-expoxy-3-hydroxy-17-methyl-14β-[(3-phenylpropyl)oxy]morphinan-6-one, 4,5α-expoxy-17-methyl-14β-[(3-phenylpropyl)oxy]morphinan-6-one, 17-(cyclopropylmethyl)-4,5α-epoxy-14β-[(3-phenylpropyl)oxy]morphinan-6-one, 4,5α-epoxy-14β-[(3-phenylpropyl)oxy]morphinan-6-one, 17-(cyclopropylmethyl)-4-hydroxy-14β-[(3-phenylpropyl)oxy]morphinan-6-one, 17-(cyclopropylmethyl)-4-methoxy-14β-[(3-phenylpropyl)oxy]morphinan-6-one, 4-(n-butyloxy)-17-{cyclopropylmethyl)-14β-[(3-phenylpropyl)oxy]morphinan-6-one,
or any pharmaceutically acceptable salt or easily accessible derivative of it.

6. Composition, comprising a compound of Claims 1 to 5 and/or a pharmaceutically acceptable acid addition salt of it, together with a pharmaceutically acceptable carrier substance.

7. Compound according to any of Claims 1 to 6 as medicament.

8. Use of a compound of Claims 1 to 5 for the manufacture of a medicament for the treatment of pain, including chronic and acute pain, post-operative pain, rheumatic diseases (e.g. arthritis), ileus, obstipation, overweight, addiction, including opioid, cocaine and alcohol addiction as well as for the manufacture of a narcotic.

9. Compounds according to Claim 1 or 2, wherein R₅ is OH or alkoxy.

10. Compounds according to Claim 1, 2 or 9, wherein R₃ is hydrogen, alkyl or aralkyl, preferably hydrogen or alkyl.

11. Compounds according to Claim 1, 2, 9 or 10 , wherein R₄ is OH, alkoxy or alkenyloxy or alkinyloxy.

12. Compounds according to Claim 1, 2, 9, 10 or 11, wherein a single bond is present between the carbon atoms of the numbers 7 and 8.

13. Compounds according to Claim 1, 2, 9, 10, 11 or 12, wherein R₂ is alkyl or aralkyl, preferably aralkyl.

14. Compounds according to Claim 1, 2, 9,10,11,12 or 13, wherein R₁ is alkyl, (cyclical saturated group)alkyl, aralkyl or alkenyl.

15. Compounds according to Claim 1 or 2, wherein R₁ is C₁-C₆-alkyl; C₂-C₆-alkenyl; C₂-C₆-alkinyl; C₃-C₁₆-(cyclical saturated group)alkyl, where alkyl is C₁-C₆; C₄-C₁₆-(cyclical saturated group)alkenyl, where alkenyl is C₂-C₆; C₄-C₁₆-(cyclical saturated group)alkinyl, where alkinyl is C₂-C₆; C₇-C₁₆-arylalkyl, where aryl is C₆-C₁₀-aryl and alkyl is C₁-C₆-alkyl; C₈-C₁₆-arylalkenyl, where aryl is C₆-C₁₀-aryl and alkenyl is C₂-C₆-alkenyl; C₈-C₁₆-arylalkinyl, where aryl is C₆-C₁₀-aryl and alkinyl is C₂-C₆-alkinyl.

## Revendications

1. Composés de formules (I) ou (Ia) 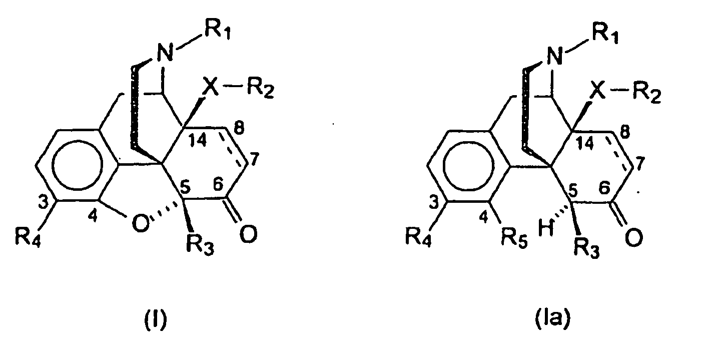 dans lesquelles les substituants ont la signification suivante :
R₁ : alkyle en C₁-C₆ ; alcényle en C₂-C₆ ; alcinyle en C₂-C₆ ; (groupe cyclique saturé)-alkyle en C₃-C₁₆, où l'alkyle est en C₁-C₆ ; (groupe cyclique saturé)alcényle en C₄-C₁₆, où l'alcényle est en C₂-C₆ ; (groupe cyclique saturé)alcinyle en C₄-C₁₆, où l'alcinyle est en C₂-C₆ ; arylalkyle en C₇-C₁₆, où l'aryle est un aryle en C₆-C₁₀ et l'alkyle est un alkyle en C₁-C₆ ; arylalcényle en C₈-C₁₆, où l'aryle est un aryle en C₆-C₁₀ et l'alcényle est un alcényle en C₂-C₆ ; arylalcinyle en C₈-C₁₆, où l'aryle est un aryle en C₆-C₁₀ et l'alcinyle est un alcinyle en C₂-C₆ ;
R₂ : alkyle en C₄-C₆ ; alcényle en C₂-C₆ ; alcinyle en C₂-C₆ ; (groupe cyclique saturé)-alkyle en C₃-C₁₆, où l'alkyle est en C₁-C₆ ; (groupe cyclique saturé)alcényle en C₄-C₁₆, où l'alcényle est en C₂-C₆ ; (groupe cyclique saturé) alcinyle en C₄-C₁₆, où l'alcinyle est en C₂-C₆; arylalkyle en C₈-C₁₆, où l'aryle est un aryle en C₆-C₁₀ et l'alkyle est un alkyle en C₁-C₆; arylalcényle en C₈-C₁₆, où l'aryle est un aryle en C₆-C₁₀ et l'alcényle est un alcényle en C₂-C₆; arylalcinyle en C₈-C₁₆, où l'aryle est un aryle en C₆-C₁₀ et l'alcinyle est un alcinyle en C₂-C₆; alcénoyle en C₃-C₆; alcinoyle en C₃-C₆; arylalcénoyle en C₉-C₁₆, où l'aryle est un aryle en C₆-C₁₀ et l'alcénoyle est un alcénoyle en C₃-C₆ ; arylalcinoyle en C₉-C₁₆, où l'aryle est un aryle en C₆-C₁₀ et l'alcinoyle est un alcinoyle en C₃-C₆ ;
R₃ : hydrogène ; alkyle en C₁-C₆ ; alcényle en C₂-C₆ ; arylalkyle en C₇-C₁₆, où l'aryle est un aryle en C₆-C₁₀ et l'alkyle est un alkyle en C₁-C₆ ; arylalcényle en C₈-C₁₆, où l'aryle est un aryle en C₆-C₁₀ et l'alcényle est un alcényle en C₂-C₆ ; alcoxyalkyle, où l'alcoxy est un alcoxy en C₁-C₆ et l' alkyle est un alkyle en C₁-C₆ ; CO₂(alkyle en C₁-C₆) ; CO₂H ; CH₂OH.
R₄ : hydrogène ; hydroxy ; alkyloxy en C₁-C₆ ; alkyloxyalcoxy en C₂-C₁₀, où l'alkyloxy est en C₁-C₄ et l'alcoxy est un alkyloxy en C₁-C₆ ; alcényloxy en C₂-C₆ ; alcinyloxy en C₂-C₆ ; (groupe cyclique saturé) alkyloxy en C₃-C₁₆, où l'alkyle est en C₁-C₆ ; (groupe cyclique saturé)-alcényloxy en C₄-C₁₆, où l'alcényle est en C₂-C₆ ; (groupe cyclique saturé)alcinyloxy en C₄-C₁₆, où l'alcinyle est en C₂-C₆ ; arylalkyloxy en C₇-C₁₆, où l'aryle est un aryle en C₆-C₁₀ et l'alkyle est un alkyle en C₁-C₆ ; arylalcényloxy en C₈-C₁₆, où l'aryle est un aryle en C₆-C₁₀ et l'alcényle est un alcényle en C₂-C₆ ; arylalcinyloxy en C₈-C₁₆, où l'aryle est un aryle en C₆-C₁₀ et l'alcinyle est un alcinyle en C₂-C₆ ; alcanoyloxy en C₁-C₆ ; alcénoyloxy en C₃-C₆ ; alcinoyloxy en C₃-C₆ ; arylalcanoyloxy en C₇-C₁₆, où l'aryle est un aryle en C₆-C₁₀ et l'alcanoyloxy est un alcanoyloxy en C₂-C₆ ; arylalcènoyloxy en C₉-C₁₆, où l'aryle est un aryle en C₆-C₁₀ et l' alcènoyloxy est un alcènoyloxy en C₃-C₆ ; arylalcinoyloxy en C₉-C₁₆, où l'aryle est un aryle en C₆-C₁₀ et l'alcinoyloxy est un alcinoyloxy en C₃-C₆ ;
R₅ : hydrogène ; hydroxy ; alkyloxy en C₁-C₆ ; alkyloxyalcoxy en C₂-C₁₀, où l'alkyloxy est en C₁-C₄ et l'alcoxy est un alkyloxy en C₁-C₆ ; alcényloxy en C₂-C₆ ; alcinyloxy en C₂-C₆ ; (groupe cyclique saturé) alkyloxy en C₃-C₁₆, où l'alkyle est en C₁-C₆ ; (groupe cyclique saturé)-alcényloxy en C₄-C₁₆, où l'alcényle est en C₂-C₆ ; (groupe cyclique saturé)alcinyloxy en C₄-C₁₆, où l'alcinyle est en C₂-C₆ ; arylalkyloxy en C₇-C₁₆, où l'aryle est un aryle en C₆-C₁₀ et l'alkyle est un alkyle en C₁-C₆ ; arylalcényloxy en C₈-C₁₆, où l'aryle est un aryle en C₆-C₁₀ et l'alcényle est un alcényle en C₂-C₆ ; arylalcinyloxy en C₈-C₁₆, où l'aryle est un aryle en C₆-C₁₀ et l'alcinyle est un alcinyle en C₂-C₆ ; alcanoyloxy en C₂-C₆ ; arylalcanoyloxy en C₇-C₁₆, où l'aryle est un aryle en C₆-C₁₀ et l'alcanoyloxy est un alcanoyloxy en C₂-C₆ ;
X est oxygène ;
où, entre les atomes de carbone des numéros 7 et 8, il peut y avoir une simple ou une double liaison,
où les groupes alkyle, alcényle et alcinyle peuvent être respectivement ramifiés ou non ramifiés, aryle peut être non substitué ou mono-, di- ou trisubstitué, respectivement indépendamment, avec des groupes hydroxy, halogène, nitro, cyano, thiocyanato, trifluorométhyle, alkyle en C₁-C₃, alcoxy en C₁-C₃, CO₂H, CONH₂, CO₂(alkyle en C₁-C₃), CONH(alkyle en C₁-C₃), CON(alkyle en C₁-C₃)₂ ; CO(alkyle en C₁-C₃) ; amino ; (monoalkyle en C₁-C₃)amino, (dialkyle en C₁-C₃)amino, cyclo-alkylamino en C₅-C₆ ; (alcanoyle en C₁-C₃)amido, SH, SO₃H, SO₃(alkyle en C₁-C₃), SO₂(alkyle en C₁-C₃), SO(alkyle en C₁-C₃), alkylthio en C₁-C₃ ou alcanoylthio en C₁-C₃,
où -(groupe saturé cyclique) est soit de préférence un cycloalkyle en C₃-C₁₀, soit un groupe hétérocyclique avec de 2 à 9 atomes de carbone, contenant également un ou plusieurs hétéro-atomes,
à l'exception des composés dans lesquels R₁ est méthyle, R₂ est alkyle en C₄-C₆, R₃ est hydrogène ou méthyle, R₄ est hydroxy ou méthoxy et R₅ est hydroxy, méthoxy ou un atome d'oxygène lié en position 5 à l'atome de carbone, quand X est oxygène ;
à l'exception encore des composés dans lesquels R₁ est cyclopropylméthyle et XR₂ est benzyloxy, quand R₄ est hydrogène ou benzyloxy et R₅ est un atome d'oxygène lié en position 5 à l'atome de carbone ;
à l'exception encore des composés dans lesquels R₁ est cyclopropylméthyle et XR₂ est benzyloxy, quand R₄ est hydrogène, hydroxy ou benzyloxy et R₅ est hydroxy ou méthoxy.

2. Composés de formules (IA) ou (IAa) 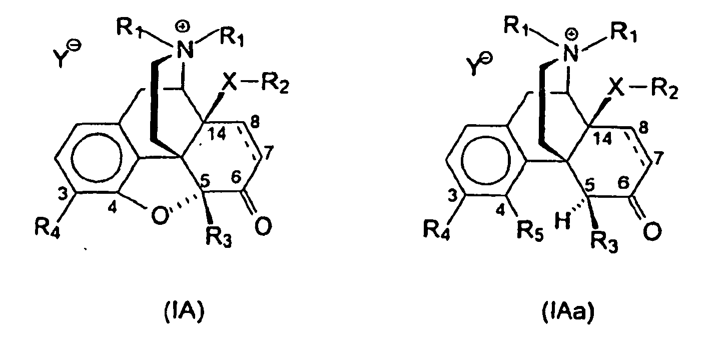 dans lesquelles les substituants ont la signification suivante :
R₁ : alkyle en C₁-C₆ ; alcényle en C₂-C₆ ; alcinyle en C₂-C₆ ; (groupe cyclique saturé)alkyle en C₃-C₁₆, où l'alkyle est en C₁-C₆ ; (groupe cyclique saturé)alcényle en C₄-C₁₆, où l'alcényle est en C₂-C₆ ; (groupe cyclique saturé) alcinyle en C₄-C₁₆, où l'alcinyle est en C₂-C₆ ; arylalkyle en C₇-C₁₆, où l'aryle est un aryle en C₆-C₁₀ et l'alkyle est un alkyle en C₁-C₆ ; arylalcényle en C₈-C₁₆, où l'aryle est un aryle en C₆-C₁₀ et l'alcényle est un alcényle en C₂-C₆ ; arylalcinyle en C₈-C₁₆, où l'aryle est un aryle en C₆-C₁₀ et l'alcinyle est un alcinyle en C₂-C₆ ;
où les deux substituants R₁ peuvent être identiques ou différents ;
R₂ : alkyle en C₄-C₆ ; alcényle en C₂-C₆ ; alcinyle en C₂-C₆ ; (groupe cyclique saturé)alkyle en C₃-C₁₆, où l'alkyle est en C₁-C₆ ; (groupe cyclique saturé) alcényle en C₄-C₁₆, où l'alcényle est en C₂-C₆ ; (groupe cyclique saturé)alcinyle en C₄-C₁₆, où l' alcinyle est en C₂-C₆ ; arylalkyle en C₈-C₁₆, où l'aryle est un aryle en C₆-C₁₀ et l'alkyle est un alkyle en C₁-C₆ ; arylalcényle en C₈-C₁₆, où l'aryle est un aryle en C₆-C₁₀ et l'alcényle est un alcényle en C₂-C₆ ; arylalcinyle en C₈-C₁₆, où l'aryle est un aryle en C₆-C₁₀ et l'alcinyle est un alcinyle en C₂-C₆ ; alcénoyle en C₃-C₆ ; alcinoyle en C₃-C₆ ; arylalcénoyle en C₉-C₁₆, où l'aryle est un aryle en C₆-C₁₀ et l'alcénoyle est un alcénoyle en C₃-C₆ ; arylalcinoyle en C₉-C₁₆, où l'aryle est un aryle en C₆-C₁₀ et l'alcinoyle est un alcinoyle en C₃-C₆ ;
R₃ : hydrogène ; alkyle en C₁-C₆ ; alcényle en C₂-C₆ ; arylalkyle en C₇-C₁₆, où l'aryle est un aryle en C₆-C₁₀ et l'alkyle est un alkyle en C₁-C₆ ; arylalcényle en C₈-C₁₆, où l'aryle est un aryle en C₆-C₁₀ et l'alcényle est un alcényle en C₂-C₆ ; alcoxyalkyle, où l'alcoxy est un alcoxy en C₁-C₆ et l'alkyle est un alkyle en C₁-C₆ ; CO₂(alkyle en C₁-C₆) ; CO₂H ; CH₂OH.
R₄ : hydrogène ; hydroxy ; alkyloxy en C₁-C₆ ; alkyloxyalcoxy en C₂-C₁₀, où l'alkyloxy est en C₁-C₄ et l'alcoxy est un alkyloxy en C₁-C₆ ; alcényloxy en C₂-C₆ ; alcinyloxy en C₂-C₆ ; (groupe cyclique saturé)alkyloxy en C₃-C₁₆, où l'alkyle est en C₁-C₆; (groupe cyclique saturé)alcényloxy en C₄-C₁₆, où l'alcényle est en C₂-C₆ ; (groupe cyclique saturé)alcinyloxy en C₄-C₁₆, où l'alcinyle est en C₂-C₆ ; arylalkyloxy en C₇-C₁₆, où l'aryle est un aryle en C₆-C₁₀ et l'alkyle est un alkyle en C₁-C₆ ; arylalcényloxy en C₈-C₁₆, où l'aryle est un aryle en C₆-C₁₀ et l'alcényle est un alcényle en C₂-C₆ ; arylalcinyloxy en C₈-C₁₆, où l'aryle est un aryle en C₆-C₁₀ et l'alcinyle est un alcinyle en C₂-C₆ ; alcanoyloxy en C₂-C₆ ; alcénoyloxy en C₃-C₆ ; alcinoyloxy en C₃-C₆ ; arylalcanoyloxy en C₈-C₁₆, où l'aryle est un aryle en C₆-C₁₀ et l'alcanoyloxy est un alcanoyloxy en C₂-C₆ ; arylalcènoyloxy en C₉-C₁₆, où l'aryle est un aryle en C₆-C₁₀ et l'alcènoyloxy est un alcènoyloxy en C₃-C₆ ; arylalcinoyloxy en C₉-C₁₆, où l'aryle est un aryle en C₆-C₁₀ et l'alcinoyloxy est un alcinoyloxy en C₃-C₆ ;
R₅ : hydrogène ; hydroxy ; alkyloxy en C₁-C₆ ; alkyloxyalcoxy en C₂-C₁₀, où l'alkyloxy est en C₁-C₄ et l'alcoxy est un alkyloxy en C₁-C₆ ; alcényloxy en C₂-C₆ ; alcinyloxy en C₂-C₆ ; (groupe cyclique saturé)alkyloxy en C₃-C₁₆, où l'alkyle est en C₁-C₆ ; (groupe cyclique saturé)alcényloxy en C₄-C₁₆, où l'alcényle est en C₂-C₆ ; (groupe cyclique saturé)alcinyloxy en C₄-C₁₆, où l'alcinyle est en C₂-C₆ ; arylalkyloxy en C₇-C₁₆, où l'aryle est un aryle en C₆-C₁₀ et l'alkyle est un alkyle en C₁-C₆ ; arylalcényloxy en C₈-C₁₆, où l'aryle est un aryle en C₆-C₁₀ et l'alcényle est un alcényle en C₂-C₆ ; arylalcinyloxy en C₈-C₁₆, où l'aryle est un aryle en C₆-C₁₀ et l'alcinyle est un alcinyle en C₂-C₆ ; alcanoyloxy en C₂-C₆ ; arylalcanoyloxy en C₇-C₁₆, où l'aryle est un aryle en C₆-C₁₀ et l'alcanoyloxy est un alcanoyloxy en C₂-C₆ ;
X est oxygène ;
Y⁻ est I⁻, Br⁻, Cl⁻, OH⁻ ou un autre contre-ion pharmacologiquement acceptable ;
où, entre les atomes de carbone des numéros 7 et 8, il peut y avoir une simple ou une double liaison,
où les groupes alkyle, alcényle et alcinyle peuvent être respectivement ramifiés ou non ramifiés, aryle peut être non substitué ou mono-, di- ou trisubstitué, respectivement indépendamment, avec des groupes hydroxy, halogène, nitro, cyano, thiocyanato, trifluorométhyle, alkyle en C₁-C₃, alcoxy en C₁-C₃, CO₂H, CONH₂, CO₂(alkyle en C₁-C₃), CONH (alkyle en C₁-C₃), CON (alkyle en C₁-C₃)₂ ; CO (alkyle en C₁-C₃); amino ; (monoalkyle en C₁-C₃)amino, (dialkyle en C₁-C₃)amino, cyclo-alkylamino en C₅-C₆ ; (alcanoyle en C₁-C₃)amido, SH, SO₃H, SO₃(alkyle en C₁-C₃), SO₂(alkyle en C₁-C₃), SO (alkyle en C₁-C₃), alkylthio en C₁-C₃ ou alcanoylthio en C₁-C₃,
où -(groupe saturé cyclique) est soit de préférence un cycloalkyle en C₃-C₁₀, soit un groupe hétérocyclique avec de 2 à 9 atomes de carbone, contenant également un ou plusieurs hétéro-atomes.

3. Composés de formules (I) et (IA) des revendications 1 et 2, dans lesquelles X est oxygène ; R₁ est alkyle en C₁-C₆, alcényle en C₂-C₆, cycloalkylalkyle en C₄-C₁₆, où le cycloalkyle est en C₃-C₁₀ et l'alkyle est en C₁-C₆, arylalkyle en C₇-C₁₆, où l'aryle est un aryle en C₆-C₁₀ et l'alkyle est un alkyle en C₁-C₆ ; R₂ est arylalkyle en C₇-C₁₆, où l'aryle est un aryle en C₆-C₁₀ et l'alkyle est un alkyle en C₁-C₆ ; arylalcényle en C₈-C₁₆, où l'aryle est un aryle en C₆-C₁₀ et l'alcényle est un alcényle en C₂-C₆ ; R₃ est hydrogène ou méthyle ; R₄ est hydroxy, méthoxy ou acétoxy.

4. Composés de formule (IA) de la revendication 2, dans lesquelles X est oxygène ; R₁ est alkyle en C₁-C₆, alcényle en C₂-C₆, cycloalkylalkyle en C₄-C₁₆, où le cycloalkyle est en C₃-C₁₀ et l'alkyle est en C₁-C₆, arylalkyle en C₇-C₁₆, où l'aryle est un aryle en C₆-C₁₀ et l'alkyle est un alkyle en C₁-C₆ ; R₂ est alkyle en C₁-C₆ ou alcényle en C₂-C₆ ; R₃ est hydrogène ou méthyle ; R₄ est hydroxy, méthoxy ou acétoxy.

5. Composés des revendications 1 et 2, choisis parmi :
17-allyl-4,5α-époxy-3-méthoxy-14β-(3-phénylpropyloxy)morphinan-6-one, 17-allyl-4,5α-époxy-3-hydroxy-14β-(3-phénylpropyloxy)morphinan-6-one, 17-allyl-4,5α-époxy-3-méthoxy-5β-méthyl-14β-(3-phénylpropyloxy)-morphinan-6-one, 17-allyl-4, 5α-époxy-3-hydroxy-5β-méthyl-14β-(3-phénylpropyloxy)morphinan-6-one, 17-cyclobutylméthyl-4,5α-époxy-3-méthoxy-14β-(3-phénylpropyloxy)-morphinan-6-one, 17-cyclobutylméthyl-4,5a-époxy-3-hydroxy-14β-(3-phénylpropyloxy)morphinan-6-one, 17-cyclobutylméthyl-4,5α-époxy-3-méthoxy-5β-méthyl-14β-(3-phénylpropyloxy)morphinan-6-one, 17-cyclobutylméthyl-4,5α-époxy-3-hydroxy-5β-méthyl-14β-(3-phénylpropyloxy)morphinan-6-one, 17-cyclopropylméthyl-4,5α-époxy-3-méthoxy-14β-(3-phénylpropyloxy)morphinan-6-one, 17-cyclopropylméthyl-4,5α-époxy-3-hydroxy-14β-(3-phénylpropyloxy)morphinan-6-one, 17-cyclopropylméthyl-4,5α-époxy-3-méthoxy-5β-méthyl-14β-(3-phénylpropyloxy)morphinan-6-one,17-cyclopropylméthyl-4,5α-époxy-3-hydroxy-5β-méthyl-14β-(3-phénylpropyloxy)-morphinan-6-one,4,5α-époxy-3-méthoxy-5β,17-diméthyl-14β-[(3-phénylpropyl)oxy]morphinan-6-one, 4,5α-époxy-3-hydroxy-5β, 17-diméthyl-14β-[(3-phénylpropyl)oxy]-morphinan-6-one, 17-propyl-4,5α-époxy-3-méthoxy-14β-(3-phénylpropyloxy) -morphinan-6-one, 17-propyl-4,5α-époxy-3-hydroxy-14β-(3-phénylpropyloxy)morphinan-6-one, 17-propyl-4,5α-époxy-3-méthoxy-5β-méthyl-14β-(3-phénylpropyloxy)-morphinan-6-one, 17-propyl-4,5α-époxy-3-hydroxy-5β-méthyl-14β-(3-phénylpropyloxy)morphinan-6-one, 17-tétrahydrofurfuryl-4,5α-époxy-3-méthoxy-14β-(3-phénylpropyloxy)morphinan-6-one, 17-tétrahydrofurfuryl-4,5α-époxy-3-hydroxy-14β-(3-phénylpropyloxy)morphinan-6-one, 17-tétrahydrofurfuryl-4,5α-époxy-3-méthoxy-5β-méthyl-14β-(3-phénylpropyloxy)morphinan-6-one,17-tétrahydrofurfuryl-4,5α-époxy-3-hydroxy-5β-méthyl-14β-(3-phénylpropyloxy)morphinan-6-one,17-(2-phényléthyl)-4,5α-époxy-3-méthoxy-14β-(3-phénylpropyloxy)-morphinan-6-one, 17-(2-phényléthyl)-4,5α-époxy-3-hydroxy-14β-(3-phénylpropyloxy)morphinan-6-one, 17-(2-phényléthyl)-4,5α-époxy-3-méthoxy-5β-méthyl-14β-(3-phénylpropyloxy)morphinan-6-one, 17-(2-phényléthyl)-4,5α-époxy-3-hydroxy-5β-méthyl-14β-(3-phénylpropyloxy)morphinan-6-one, 17-éthyl-4,5α-époxy-3-méthoxy-14β-(3-phénylpropyloxy)morphinan-6-one, 17-éthyl-4,5α-époxy-3-hydroxy-14β-(3-phénylpropyloxy)morphinan-6-one, 17-éthyl-4,5α-époxy-3-méthoxy-5β-méthyl-14β-(3-phénylpropyloxy)-morphinan-6-one, 17-éthyl-4, 5α-époxy-3-hydroxy-5β-méthyl-14β-(3-phénylpropyloxy)morphinan-6-one, 17-cyclopropylméthyl-4,5α-époxy-3-hydroxy-14β-[(2-méthylbenzyl)oxy]morphinan-6-one, 14β-[(2-chlorobenzyl)oxy]-17-(cyclopropylméthyl)-4,5α-époxy-3-hydroxymorphinan-6-one, 14β-benzyloxy-17-cyclopropylméthyl-4,5α-époxy-3-hydroxymorphinan-6-one, 14β-butoxy-17-cyclopropylméthyl-4,5α-époxy-3-hydroxymorphinan-6-one, 17-cyclopropylméthyl-4,5α-époxy-3-hydroxy-14β-[(3-méthylbutyl)oxy]-morphinan-6-one, 4,5α-époxy-5β, 17-diméthyl-14β-[(3-phénylpropyl)oxy]-3-[(prop-2-inyl)oxy]-morphinan-6-one, 14β-[(3-chlorobenzyl)oxy]-4,5α-époxy-17-méthyl-3-[(prop-2-inyl) oxy] - morphinan-6-one, 4,5α-époxy-17-éthyl-3-méthoxy-14β-[(3-phénylpropyl)oxy]-morphinan-6-one, 4,5α-époxy-17-éthyl-3-hydroxy-14β-[(3-phénylpropyl)oxy] - morphinan-6-one, 4,5α-époxy-3-hydroxy-14β-[(3-méthylbutyl)oxy]-17-propylmorphinan-6-one, 5β-benzyl-14-méthoxycodéinone (= 5-benzyl-7,8-didéshydro-4,5α-époxy-3, 14β-diméthoxy-17-méthylmorphinan-6-one), 5β-benzyl-4,5α-époxy-3, 14β-diméthoxy-17-méthylmorphinan-6-one, 5β-benzyl-4,5α-époxy-3-hydroxy-14β-méthoxy-17-méthylmorphinan-6-one, 4-hydroxy-3-méthoxy-17-méthyl-14-[(3-phénylpropyl)oxy]morphinan-6-one, 3,4-diméthoxy-17-méthyl-14-[(3-phénylpropyl)oxy]-morphinan-6-one, 14β-benzyloxy-4-hydroxy-3-méthoxy-17-méthylmorphinan-6-one, 14β-benzyloxy-3,4-diméthoxy-17-méthylmorphinan-6-one, 4-hydroxy-3-méthoxy-17-méthyl-14β-[(2-naphtylméthyl)oxy]morphinan-6-one, 3,4-diméthoxy-17-méthyl-14β-[(2-naphtylméthyl)-oxy]morphinan-6-one, 4-hydroxy-3-méthoxy-5β, 17-diméthyl-14β-[(3-phénylpropyl)oxy]morphinan-6-one, 3,4-diméthoxy-5β, 17-diméthyl-14β-[(3-phénylpropyl)oxy]morphinan-6-one, 14β-éthoxy-4-hydroxy-3-méthoxy-5β, 17-diméthylmorphinan-6-one, 14β-éthoxy-3,4-diméthoxy-5β, 17-diméthylmorphinan-6-one, 14β-benzyloxy-3,4-diméthoxy-5β, 17-diméthylmorphinan-6-one, iodure de 4,5α-époxy-3-hydroxy-17, 17-diméthyl-6-oxo-14β-[(3-phénylpropyl)oxy]-morphinanium, iodure de (17S)-4,5α-époxy-17-éthyl-3-hydroxy-17-méthyl-6-oxo-14β-[(3-phénylpropyl) -oxy]-morphinanium, iodure de (17R)-4,5α-époxy-3-hydroxy-17-méthyl-6-oxo-14β-[(3-phénylpropyl)-oxy]-17-[(2(R,S)-tétrahydrofurfuran-2-yl)méthyl]morphinanium, iodure de (17R)-17-allyl-4,5α-époxy-19β-éthoxy-3-hydroxy-17-méthyl-6-oxomorphinanium, iodure de (17R)-17-allyl-4,5a-époxy-3-hydroxy-14β-méthoxy-17-méthyl-6-oxomorphinanium, iodure de (17S)-17-allyl-4,5α-époxy-3-hydroxy-14β-méthoxy-17-méthyl-6-oxomorphinanium, iodure de 4,5α-époxy-3-hydroxy-14β-méthoxy-17, 17-diméthyl-6-oxomorphinanium, iodure de 5β-benzyl-14β-(butyloxy)-4,5α-époxy-3-hydroxy-17, 17-diméthyl-6-oxomorphinanium, iodure de (17S)-17-allyl-5β-benzyl-14β-butoxy-4,5α-époxy-3-hydroxy-17-méthyl-6-oxomorphinanium, iodure de 14β-butoxy-4,5α-époxy-3-hydroxy-17,17-diméthyl-6-oxo-morphinanium, iodure de (17R)-17-cyclopropylméthyl-4,5α-époxy-3-hydroxy-17-méthyl-6-oxo-14β-[(3-phénylpropyl)oxy]morphinanium, iodure de (17R)-17-cyclopropylméthyl-4,5α-époxy-3-méthoxy-17-méthyl-6-oxo-14β-[(3-phénylpropyl)-oxy]morphinanium, iodure de (17R)-17-cyclopropylméthyl-4,5α-époxy-3-hydroxy-17-méthyl-6-oxo-14β-[(2-phénylbenzyl)oxy]morphinanium, iodure de (17R)-14β-[(4-chlorobenzyl)oxy]-17-cyclopropylméthyl-4,5α-époxy-3-hydroxy-17-méthyl-6-oxomorphinanium, iodure de (17R)-4,5α-époxy-3-hydroxy-14β-méthoxy-17-méthyl-6-oxo-17-(2-phényléthyl)morphinanium, 4,5α-époxy-3-méthoxy-17-méthyl-14β-[(3-phénylpropyl)oxy]morphinan-6-one, 4,5α-époxy-3-méthoxy-14β-[(3-phénylpropyl)oxy]morphinan-6-one, 4,5α-époxy-3-hydroxy-17-méthyl-14β-[(3-phénylpropyl)oxy]morphinan-6-one, 4,5α-époxy-17-méthyl-14β-[(3-phénylpropyl)oxy]morphinan-6-one, 17-(cyclopropylméthyl)-4,5α-époxy-14β-[(3-phénylpropyl)oxy]morphinan-6-one, 4,5α-époxy-14β-[(3-phénylpropyl)oxy]morphinan-6-one, 17-(cyclopropylméthyl)-4-hydroxy-14β-[(3-phénylpropyl)oxy]morphinan-6-one, 17-(cyclopropylméthyl)-4-méthoxy-14β-[(3-phénylpropyl)-oxy]-morphinan-6-one, 4-(n-butyloxy)-17-(cyclopropylméthyl)-14β-[(3-phénylpropyl)oxy]morphinan-6-one,
ou n'importe quel sel pharmaceutiquement accetable ou dérivé facilement accessible de celui-ci.

6. Composition, comprenant un composé des revendications 1 à 5 et/ou un sel d'addition acide pharmaceutiquement acceptable de celui-ci, conjointement avec un véhicule pharmaceutiquement acceptable.

7. Composé selon l'une quelconque des revendications 1 à 6 en tant que médicament.

8. Utilisation d'un composé des revendications 1 à 5 pour la fabrication d'un médicament destiné au traitement des douleurs, y compris des douleurs chroniques et aigues, des douleurs post-opératoires, des maladies rhumatoïdes (par exemple arthrite), de l'iléus, de la constipation, du surpoids, de la dépendance, y compris de la dépendance aux opiacés, à la cocaïne et à l'alcool, ainsi que pour la fabrication d'un narcotique.

9. Composés selon la revendication 1 ou 2, dans lesquels R₅ est OH ou alcoxy.

10. Composés selon la revendication 1, 2 ou 9, dans lesquels R₃ est hydrogène, alkyle ou aralkyle, de préférence hydrogène ou alkyle.

11. Composés selon la revendication 1, 2, 9 ou 10, dans lesquels R₄ est OH, alcoxy ou alcényloxy ou alcinyloxy.

12. Composés selon la revendication 1, 2, 9, 10 ou 11, dans lesquels il y a une simple liaison entre les atomes de carbone de numéros 7 et 8.

13. Composés selon la revendication 1, 2, 9, 10, 11 ou 12, dans lesquels R₂ est alkyle ou aralkyle, de préférence aralkyle.

14. Composés selon la revendication 1, 2, 9, 10, 11, 12 ou 13, dans lesquels R₁ est alkyle, (groupe saturé cyclique)alkyle, aralkyle ou alcényle.

15. Composés selon la revendication 1 ou 2, dans lesquels R₁ est alkyle en C₁-C₆ ; alcényle en C₂-C₆ ; alcinyle en C₂-C₆ ; (groupe cyclique saturé)-alkyle en C₃-C₁₆, où l'alkyle est en C₁-C₆ ; (groupe cyclique saturé)alcényle en C₉-C₁₆, où l' alcényle est en C₂-C₆ ; (groupe cyclique saturé) - alcinyle en C₉-C₁₆, où l'alcinyle est en C₂-C₆; arylalkyle en C₇-C₁₆, où l'aryle est un aryle en C₆-C₁₀ et l'alkyle est un alkyle en C₁-C₆ ; arylalcényle en C₈-C₁₆, où l'aryle est un aryle en C₆-C₁₀ et l'alcényle est un alcényle en C₂-C₆ ; arylalcinyle en C₈-C₁₆, où l'aryle est un aryle en C₆-C₁₀ et l'alcinyle est un alcinyle en C₂-C₆.
